(19) Europäisches Patentamt
European Patent Office
Office européen des brevets

(11) EP 1 773 823 B1

(12) FASCICULE DE BREVET EUROPEEN

(45) Date de publication et mention de la délivrance du brevet:
**21.08.2013 Bulletin 2013/34**

(21) Numéro de dépôt: **05790796.6**

(22) Date de dépôt: **20.07.2005**

(51) Int Cl.:
***C07D 413/06*** *(2006.01)* ***C07D 413/14*** *(2006.01)*
***C07D 265/10*** *(2006.01)* ***A61K 31/537*** *(2006.01)*

(86) Numéro de dépôt international:
**PCT/FR2005/001852**

(87) Numéro de publication internationale:
**WO 2006/021654 (02.03.2006 Gazette 2006/09)**

# (54) DERIVES DE 4-ARYLMORPHOLIN-3-ONE, LEUR PREPARATION ET LEUR APPLICATION EN THERAPEUTIQUE

4-ARYLMORPHOLIN-3-ONDERIVATE, HERSTELLUNG UND THERAPEUTISCHE VERWENDUNG DAFÜR

4-ARYLMORPHOLIN-3-ONE DERIVATIVES, PREPARATION AND THERAPEUTIC USE THEREOF

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **23.07.2004 FR 0408222**

(43) Date de publication de la demande:
**18.04.2007 Bulletin 2007/16**

(73) Titulaire: **SANOFI**
**75008 Paris (FR)**

(72) Inventeurs:
- **EMONDS-ALT, Xavier**
**F-34980 Combaillaux (FR)**
- **PROIETTO, Vincenzo**
**F-34680 Saint-Georges-d'Orques (FR)**

(74) Mandataire: **Kugel, Dominique**
**Sanofi**
**Département Brevets**
**54, rue La Boétie**
**75008 Paris (FR)**

(56) Documents cités:
**WO-A-00/58292 WO-A-98/04561**
**WO-A-02/094821 US-A- 5 641 777**
**US-A- 6 028 082 US-A- 6 159 967**
**US-B1- 6 288 059**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 773 823 B1

Printed by Jouve, 75001 PARIS (FR)

## Description

La présente invention a pour objet de nouveaux dérivés de 4-arylmorpholin-3-one, leur préparation et leur application en thérapeutique.

Les composés selon la présente invention présentent une très forte affinité pour les récepteurs $NK_2$ humains de la neurokinine A.

La neurokinine A (NKA) appartient à un groupe de neuropeptides appelés tachykinines ou neurokinines qui inclut la substance P (SP) et la neurokinine B (NKB). Les effets biologiques de la neurokinine A ainsi que des autres tachykinines sont médiés par des récepteurs spécifiques de la famille des récepteurs à sept domaines transmembranaires couplés aux G protéines qui sont nommés $NK_1$, $NK_2$ et $NK_3$. Le récepteur $NK_2$ aux tachykinines fixe la neurokinine A tandis que les récepteurs $NK_1$ et $NK_3$ fixent respectivement la substance P et la neurokinin B (Pennefather J.N. et al., Life Sci., 2004, 74, 1445-1463). Les récepteurs $NK_2$ aux tachykinines sont très largement exprimés dans le système nerveux périphérique, où ils médient la large variété d'effets produits par la neurokinine A, spécialement et de manière non limitative dans le système respiratoire (bronchoconstriction, toux, inflammation, hyperactivité bronchique,..) (Joos G.F., Handb. Exp. Pharm., 2004, 164, 491-510 ; Advenier C. et al., Eur. Respir. J., 1997, 10, 1892-1906), gastrointestinal (inflammation, infection, motricité, douleur, ...) (Holzer P, Handb Exp. Pharm., 2004, 164, 511-558) et urinaire (hyperactivité vésicale, inflammation, infection, ...) (Kiss S. et al., Neurosci. Lett., 2001, 313, 57-60 ; Wamer F.J. et al., Eur. J. Pharmacol., 2002, 438, 171-177). Le récepteur $NK_2$ aux tachykinines est également exprimé dans le cerveau (Hagan R.M. et al., Regul. Pept., 1993, 46, 9-19 ; Steinberg R. et al, Eur. J. Neurosci., 1998, 10, 2337-2345 ; Bensaid M. et al., Neurosci. Lett., 2001, 303, 25-28 ; Saffroy M. et al., J. Neurochem., 2001, 79, 985-996 ; Saffroy M. et al., Neuroscience, 2003, 116, 761-773) et la moelle épinière (Yashpal K. et al., Brain Res., 1990, 506, 59-266) où il médie les effets centraux de la neurokinine A.

A titre d'exemples, de nombreuses données expérimentales montrent que le blocage du récepteur $NK_2$ aux tachykinines par un antagoniste peut être un traitement de la dépression majeure (Emonds-Alt, Handb Exp. Pharm., 2004, 219-244) ainsi que des troubles fonctionels et douloureux gastrointestinaux tels que le syndrome du colon irritable (IBS) (Emonds-Alt, Handb Exp. Pharm., 2004, 219-244 ; Lecci A. et al., Br. J. Pharmacol., 2004, 141, 1249-1263).

De nombreux brevets ou demandes de brevet décrivent des composés actifs sur les récepteurs des tachykinines. Ainsi la demande internationale WO 96/23787 concerne les composés de formule :

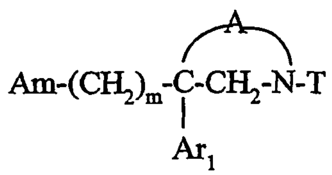

(A)

dans laquelle notamment :

- A peut représenter le radical bivalent -O-CH2-CO- ;
- Am, m, $Ar_1$ et T ont différentes valeurs.

Les composés (A) présentent une affinité pour les récepteurs $NK_1$, $NK_2$ ou $NK_3$ des tachykinines en général.

La demande de brevet EP-A-0 776 893 concerne les composés de formule :

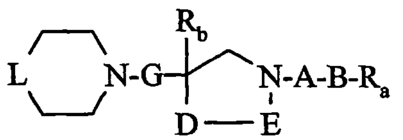

(B)

dans laquelle notamment :

- D-E peut représenter un radical bivalent $-O-CH_2-CH_2-$ ;
- L, G, E, A, B, $R_a$ et $R_b$ ont différentes valeurs.

Les composés (B) sont des antagonistes à la fois des récepteurs $NK_1$ et des récepteurs $NK_2$ des tachykinines.

La demande WO 00/34274 concerne des dérivés de cyclohexylpipéridine qui sont des antagonistes à la fois des récepteurs $NK_1$ de la substance P et des récepteurs $NK_2$ de la neurokinine A.

La demande WO 02/094821 concerne des dérivés de morpholine qui sont des antagonistes à la fois des récepteurs $NK_2$ humains et des récepteurs $NK_3$ humains des tachykinines.

On a maintenant trouvé de nouveaux composés qui présentent une très forte affinité pour les récepteurs $NK_2$ humains de la neurokinine A et qui sont des antagonistes desdits récepteurs.

De plus, les composés selon la présente invention présentent une bonne biodisponibilité lorsqu'ils sont administrés par la voie orale et passent la barrière hématoencéphalique.

La présente invention a pour objet des composés répondant à la formule (I) :

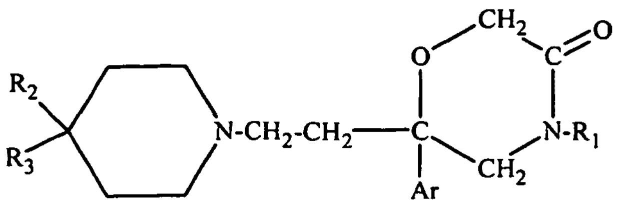

(I)

dans laquelle :

- Ar représente un phényle disubstitué par un atome d'halogène ;
- $R_1$ représente un phényle non substitué ou substitué une ou deux fois par un atome d'halogène ;
- $R_2$ représente :
  - un pyridyle ;
  - un phényle non substitué ou substitué une ou deux fois par un ou deux substituants choisis indépendamment parmi un atome d'halogène, un $(C_1\text{-}C_4)$alkyle, un $(C_1\text{-}C_4)$alcoxy, un groupe trifluorométhyle, un groupe trifluorométhoxy ;
  - $R_2$ peut de plus représenter un radical hétérocyclique choisi parmi l'azétidine, la pyrrolidine, la pipéridine, la morpholine, la thiomorpholine ou la perhydroazétidine lorsque $R_3$ représente un groupe $-CONR_{11}R_{12}$ ;
- $R_3$ représente un groupe choisi parmi :

  (5) $-(CH_2)_q-OH$ dans lequel q est 0 ;
  (10)$-(CH_2)_q-NR_7COR_8$ dans lequel q est 0 ;
  (11)$-(CH_2)_q-NR_7COOR_9$ dans lequel q est 0 ;
  (18)$-CONR_nR_{12}$;

  à l'état de base ou de sel d'addition à un acide, ainsi qu'à l'état d'hydrate ou de solvat.
- $R_7$ représente un atome d'hydrogène ou un $(C_1\text{-}C_4)$alkyle ;
- $R_8$ représente un atome d'hydrogène ; un $(C_1\text{-}C_4)$alkyle ; un vinyle ; un phényle ; un benzyle ; un pyridyle ; un $(C_3\text{-}C_7)$cycloalkyle non substitué ou substitué par un ou plusieurs méthyles ; un furyle ; un thiényle ; un pyrrolyle ; un imidazolyle ;
- ou $R_7$ et $R_8$ ensemble représentent un groupe $-(CH_2)_p-$ ;
- p est 3 ou 4 ;
- $R_9$ représente un $(C_1\text{-}C_4)$alkyle ou un phényle ;
- ou $R_7$ et $R_9$ ensemble représentent un groupe $-(CH_2)_n-$ ;
- n est 2 ou 3 ;
- $R_{11}$ et $R_{12}$ représentent chacun indépendamment un hydrogène ou un $(C_1\text{-}C_4)$alkyle ; $R_{12}$ peut de plus représenter un $(C_3\text{-}C_7)$cycloalkyle, un $(C_3\text{-}C_7)$cycloalkylméthyle, un hydroxy, un $(C_1\text{-}C_4)$alcoxy, un benzyle ou un phényle ; ou $R_1$ et $R_{12}$ ensemble avec l'atome d'azote auquel ils sont liés constituent un hétérocycle choisi parmi l'azétidine, la pyrrolidine, la pipéridine, la morpholine, la thiomorpholine ou la perhydroazépine.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables mais les sels d'autres acides utiles pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.
Par atome d'halogène on entend un atome de brome, de chlore, de fluor ou d'iode.
Par ($C_1$-$C_4$)alkyle, on entend un radical alkyle linéaire ou ramifié de un à quatre atomes de carbone, tel que le radical méthyle, éthyle, propyle, isopropyle, butyle, isobutyle, *sec*-butyle, *tert*-butyle.
Par ($C_1$-$C_4$)alcoxy on entend un radical alcoxy linéaire ou ramifié de un à quatre atomes de carbone, tel que le radical méthoxy, éthoxy, propoxy, isopropoxy, butoxy, isobutoxy, *sec*-butoxy, *tert*-butoxy.
Par ($C_3$-$C_7$)cycloalkyle on entend un groupe alkyle cyclique de 3 à 7 atomes de carbone, tel que le groupe cyclopropyle, cyclobutyle, cyclopentyle, cyclohexyle ou cycloheptyle.
Parmi les composés de formule (I), objets de l'invention, on peut citer les composés de formule (I) pour lesquels :

- Ar représente un 3,4-dichlorophényle ou un 3,4-difluorophényle ;
- $R_1$ représente un phényle, un 4-chlorophényle, un 4-fluorophényle, un 3,4-difluorophényle ;
- $R_2$ représente :
  - un pyridin-2-yle ;
  - un phényle, un 4-chlorophényle, un 3-fluorophényle, un 4-fluorophényle, un 3,4-difluorophényle, un 3-méthylphényle, un 3,4-diméthylphényle, un 4-méthoxyphényle, un 3-(trifluorométhyi)phényle, un 4-(trifluorométhyl)phényle, un 4-(trifluorométhoxy)phényle ;
  - $R_2$ peut de plus représenter un pipéridin-1-yle lorsque $R_3$ représente un groupe-$CONH_2$ ou un groupe -$CON(CH_3)_2$ ;
- $R_3$ représente un groupe choisi parmi :
  - un hydroxy ;
  - -$NHCOCH_3$ ;

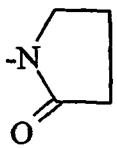 ; 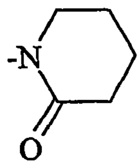 ;

  - 

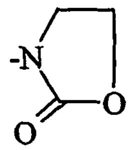 ; 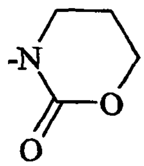 ;

  - -$CONH_2$ ;

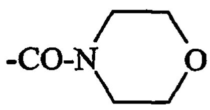 ;

-$CON(CH_3)_2$ ;

à l'état de base ou de sel d'addition à un acide, ainsi qu'à l'état d'hydrate ou de solvat.
Parmi les composés de formule (I), objets de l'invention, on peut notamment citer les composés suivants :

- 6-(3,4-dichlorophényl)-6-[2-[4-hydroxy-4-[3-(trifluorométhyl)phényl]pipéridin-1-yl]éthyl]-4-phénylmorpholin-3-one, isomère dextrogyre ;
- N-[1-[2-[2-(3,4-dichlorophényl)-5-oxo-4-phénylmorpholin-2-yl]éthyl]-4-phénylpipéridin-4-yl]acétamide, isomère

dextrogyre ;

- N-[1-[2-[2-(3,4-dichlorophényl)-5-oxo-4-phénylmorpholin-2-yl]éthyl]-4-(3-fluorophényl)pipéridin-4-yl]acétamide, isomère dextrogyre ;
- N-[1-[2-[2-(3,4-dichlorophényl)-5-oxo-4-phénylmorpholin-2-yl]éthyl]-4-(3,4-difiuorophényl)pipéridin-4-yl]acétamide, isomère dextrogyre ;
- N-[1-[2-[2-(3,4-dichlorophényl)-5-oxo-4-phénylmorpholin-2-yl]éthyl]-4-(4-méthylphényl)pipéridin-4-yl]acétamide, isomère dextrogyre ;
- N-[1-[2-[2-(3,4-dichlorophényl)-5-oxo-4-phénylmorpholin-2-yl]éthyl]-4-(4-méthoxyphényl)pipéridin-4-yl]acétamide, isomère dextrogyre ;
- N-[1-[2-[2-(3,4-dichlorophényl)-5-oxo-4-phénylmorpholin-2-yl]éthyl]-4-[4-(trifluorométhoxy)phényl]pipéridin-4-yl] acétamide, isomère dextrogyre ;
- 6-(3,4-dichlorophényl)-6-[2-[4-(3-fluorophényl)-4-(2-oxopyrrolidin-1-yl)pipéridin-1-yl]éthyl]-4-phénylmorpholin-3-one, isomère dextrogyre ;
- 6-(3,4-dichlorophényl)-6-[2-[4-(3,4-difluorophényl)-4-(2-oxopyrrolidin-1-yl)pipéridin-1-yl]éthyl]-4-phénylmorpholin-3-one, isomère dextrogyre ;
- 6-(3,4-dichlorophényl)-6-[2-[4-(4-méthylphényl)-4-(2-oxopyrrolidin-1-yl)pipéridin-1-yl]éthyl]-4-phénylmorpholin-3-one, isomère dextrogyre ;
- 1'-[2-[2-(3,4-dichlorophényl)-5-oxo-4-phénylmorpholin-2-yl]éthyl]-4'-(4-méthylphényl)-1-4'-bipipéridin-2-one, isomère dextrogyre ;
- 6-(3,4-dichlorophényl)-6-[2-[4-(3-méthylphényl)-4-(2-oxopyrrolidin-1-yl)pipéridin-1-yl]éthyl]-4-phénylmorpholin-3-one, isomère dextrogyre ;
- 6-(3,4-dichlorophényl)-6-[2-[4-(3-fluorophényl)-4-(2-oxo-1,3-oxazolidin-3-yl)pipéridin-1-yl]éthyl]-4-phénylmorpholin-3-one, isomère dextrogyre ;
- 6-(3,4-dichlorophényl)-6-[2-[4-(4-fluorophényl)-4-(2-oxo-1,3-oxazolidin-3-yl)pipéridin-1-yl]éthyl]-4-phénylmorpholin-3-one, isomère dextrogyre ;
- 6-(3,4-dichlorophényl)-6-[2-[4-(3,4-diméthylphényl)-4-(2-oxo-1,3-oxazolidin-3-yl)pipéridin-1-yl]éthyl]-4-phénylmorpholin-3-one, isomère dextrogyre ;
- 3-[1-[2-[2-(3,4-dichlorophényl)-5-oxo-4-phénylmorpholin-2-yl]éthyl]-4-phénylpipéridin-4-yl]-1,3-oxazinan-2-one, isomère dextrogyre ;
- 3-[1-[2-[2-(3,4-dichlorophényl)-5-oxo-4-phénylmorpholin-2-yl]éthyl]-4-(4-fluorophényl)pipéridin-4-yl]-1,3-oxazinan-2-one, isomère dextrogyre ;
- 3-[1-[2-[2-(3,4-dichlorophényl)-5-oxo-4-phénylmorpholin-2-yl]éthyl]-4-(3,4-difluorophényl)pipéridin-4-yl]-1,3-oxazinan-2-one, isomère dextrogyre ;
- 6-(3,4-dichlorophényl)-6-[2-[4-(morpholin-4-ylcarbonyl)-4-phénylpipéridin-1-yl]éthyl]-4-phénylmorpholin-3-one, isomère dextrogyre ;
- 1'-[2-[2-(3,4-dichlorophényl)-5-oxo-4-phénylmorpholin-2-yl]éthyl]-1,4'-bipipéridine-4'-carboxamide, isomère dextrogyre ;
- 6-(3,4-dichlorophényl)-6-[2-[4-(3,4-difluorophényl)-4-(2-oxo-1,3-oxazolidin-3-yl)pipéridin-1-yl]éthyl]-4-phénylmorpholin-3-one, isomère dextrogyre ;
- N-[1-[2-[2-(3,4-difluorophényl)-5-oxo-4-phénylmorpholin-2-yl]éthyl]-4-phénylpipéridin-4-yl]acétamide, isomère dextrogyre ;
- N-[1-[2-[2-(3,4-difluorophényl)-5-oxo-4-phénylmorpholin-2-yl]éthyl]-4-[4-(triffuorométhyl)phényl]pipéridin-4-yl]acétamide, isomère dextrogyre ;
- 1'-[2-[2-(3,4-difluorophényl)-5-oxo-4-phénylmorpholin-2-yl]éthyl]-1,4'-bipipéridine-4'-carboxamide, isomère dextrogyre ;
- N-[1-[2-[2-(3,4-dichlorophényl)-5-oxo-4-phénylmorpholin-2-yl]éthyl]-4-[3-(trifluorométhyl)phényl]pipéridin-4-yl]acétamide, isomère dextrogyre ;
- 6-(3,4-dichlorophényl)-6-[2-(4-hydroxy-4-pyridin-2-ylpipéridin-1-yl)éthyl]-4-phénylmorpholin-3-one, isomère dextrogyre ;
- N-[1-[2-[4-(4-chlorophényl)-2-(3,4-dichlorophényl)-5-oxo-morpholin-2-yl]éthyl]-4-phénylpipéridin-4-yl]acétamide ;
- 4-(4-chlorophényl)-6-[2-[4-(4-chlorophényl)-4-(2-oxo-1,3-oxazolidin-3-yl)pipéridin-1-yl]éthyl]-6-(3,4-dichlorophényl)morpholin-3-one ;
- 1'-[2-[4-(4-chlorophényl)-2-(3,4-dichlorophényl)-5-oxo-morpholin-2-yl]éthyl]-N,N-diméthyl-1,4'-bipipéridine-4'-carboxamide ;
- N-[1-[2-[2-(3,4-dichlorophényl)-4-(4-fluorophényl)-5-oxo-morpholin-2-yl]éthyl]4-phénylpipéridin-4-yl]acétamide ;
- N-[1-[2-[2-(3,4-dichlorophényl)-4-(4-fluorophényl)-5-oxo-morpholin-2-yl]éthyl]-4-(3,4-difluorophényl)pipéridin-4-yl] acétamide ;
- 1'-[2-[2-(3,4-dichlorophényl)-4-(4-fluorophényl)-5-oxo-morpholin-2-yl]éthyl]-N,N-diméthyl-1,4'-bipipéridine-4'-

carboxamide ;

- N-[1-[2-[2-(3,4-dichlorophényl)-4-(3,4-difluorophényl)-5-oxo-morpholin-2-yl]éthyl]-4-phénylpipéridin-4-yl] acétamide ;

à l'état de base ou de sel d'addition à un acide, ainsi qu'à l'état d'hydrate ou de solvat.

Le composé suivant :

- N-[1-[2-[2-(3,4-dichlorophényl)-5-oxo-4-phénylmorpholin-2-yl]éthyl]-4-(3-fluorophényl)pipéridin-4-yl]acétamide, isomère dextrogyre ;

à l'état de base ou de sel d'addition à un acide, ainsi qu'à l'état d'hydrate ou de solvat est préféré.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans "Protective Group in Organic Synthesis", Green et al., 2nd Edition (John Wiley & Sons, Inc., New York), 1991.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans "Advanced in Organic Chemistry", J. March, 3rd Edition, Wiley Interscience, 1985, p. 310-316.

Conformément à l'invention, on peut préparer les composés de formule (I) selon un procédé qui est caractérisé en ce que :

on fait réagir un composé de formule :

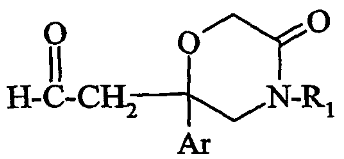

(II)

dans laquelle Ar et $R_1$ sont tels que définis pour un composé de formule (I), avec un composé de formule :

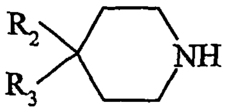

(III)

dans laquelle $R_2$ et $R_3$ sont tels que définis pour un composé de formule (I), en présence d'un acide, dans un solvant, puis on réduit le sel d'iminium formé intermédiairement au moyen d'un agent réducteur.

Eventuellement, on transforme le composé de formule (I) en l'un de ses sels d'addition à un acide.

La réaction s'effectue en présence d'un acide tel que l'acide acétique, dans un solvant tel que le méthanol ou le dichlorométhane, à une température comprise entre la température ambiante et la température de reflux du solvant, et forme *in-situ* une imine intermédiaire qui est réduite chimiquement en utilisant, par exemple, le cyanoborohydrure de sodium ou le triacétoxyborohydrure de sodium ou catalytiquement en utilisant l'hydrogène et un catalyseur tel que le palladium sur charbon ou le nickel de Raney®.

Selon une variante du procédé :

on fait réagir un composé de formule :

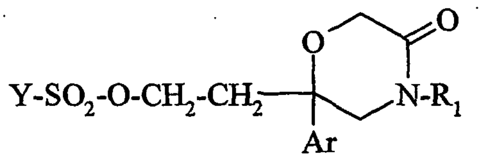

(IV)

dans laquelle Ar et $R_1$ sont tels que définis pour un composé de formule (I), et Y représente un groupe méthyle, phényle, tolyle ou trifluorométhyle, avec un composé de formule:

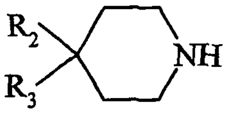

(III)

dans laquelle $R_2$ et $R_3$ sont tels que définis pour un composé de formule (I).

Eventuellement, on transforme le composé de formule (I) en l'un de ses sels d'addition à un acide.

La réaction s'effectue dans un solvant tel que le N,N-diméthylformamide, l'acétonitrile, le dichlorométhane, le toluène ou le propan-2-ol et en présence ou en l'absence d'une base. Lorsqu'on utilise une base, celle-ci est choisie parmi les bases organiques telles que la triéthylamine, la N,N-diisopropyléthylamine ou la N-méthylmorpholine ou parmi les carbonates ou bicarbonates de métal alcalin tels que le carbonate de potassium, le carbonate de sodium ou le bicarbonate de sodium. En l'absence de base, la réaction s'effectue en utilisant un excès du composé de formule (III) et en présence d'un iodure de métal alcalin tel que l'iodure de potassium ou l'iodure de sodium. La réaction s'effectue à une température comprise entre la température ambiante et 100°C.

Le composé de formule (I) ainsi obtenu peut être ultérieurement séparé du milieu réactionnel et purifié selon les méthodes classiques, par exemple, par cristallisation ou chromatographie.

Les composés de formule (II) se préparent par oxydation des composés de formule :

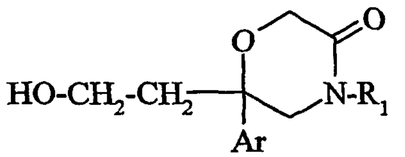

(V)

dans laquelle Ar et $R_1$ sont tels que définis pour un composé de formule (I).

La réaction d'oxydation s'effectue en utilisant par exemple le chlorure d'oxalyle, le diméthylsulfoxyde et la triéthylamine dans un solvant tel que le dichlorométhane et à une température comprise entre -78°C et la température ambiante.

Les composés de formule (V) se préparent par déprotection des composés de formule :

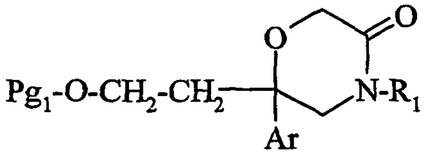

(VI)

dans laquelle Ar et $R_1$ sont tels que définis pour un composé de formule (I) et $Pg_1$ représente un groupe O-protecteur classique tel que, par exemple le tétrahydropyran-2-yle, le benzoyle ou un $(C_1-C_4)$alkylcarbonyle.

La déprotection s'effectue selon les méthodes classiques bien connues de l'homme de l'art. Par exemple, lorsque $Pg_1$ représente un groupe tétrahydropyran-2-yle, la déprotection s'effectue par hydrolyse acide en utilisant l'acide chlorhydrique dans un solvant tel que l'éther, le méthanol ou le mélange de ces solvants, ou en utilisant le *p*-toluènesulfonate de pyridinium dans un solvant tel que le méthanol ou encore, en utilisant une résine Amberlyst® dans un solvant tel que le méthanol. La réaction s'effectue à une température comprise entre la température ambiante et la température de reflux du solvant. Lorsque $Pg_1$ représente un groupe benzoyle ou un groupe $(C_1-C_4)$alkylcarbonyle, la déprotection s'effectue par hydrolyse en milieu alcalin en utilisant par exemple un hydroxyde de métal alcalin tel que l'hydroxyde de sodium, l'hydroxyde de potassium ou l'hydroxyde de lithium, dans un solvant tel que l'eau, le méthanol,

l'éthanol, le dioxane ou un mélange de ces solvants, à une température comprise entre 0°C et la température de reflux du solvant.

Les composés de formule (VI) se préparent par cyclisation des composés de formule :

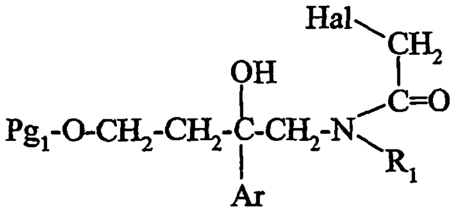
(VII)

dans laquelle Ar et $R_1$ sont tels que définis pour un composé de formule (I), $Pg_1$ est tel que défini pour un composé de formule (VI) et Hal représente un atome d'halogène, de préférence le chlore ou le brome. La réaction de cyclisation s'effectue en présence d'une base telle que le *tert*-butylate de potassium dans un solvant tel que le tétrahydrofurane et à une température comprise entre -60°C et la température ambiante.

Les composés de formule (VII) se préparent par réaction d'un composé de formule :

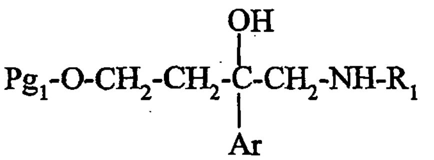
(VIII)

dans laquelle Ar et $R_1$ sont tels que définis pour un composé de formule (I), et $Pg_1$ est tel que défini pour un composé de formule (VI), avec un composé de formule Hal'-CO-$CH_2$-Hal dans laquelle Hal et Hal' représente un atome d'halogène, de préférence le chlore ou le brome, en présence d'une base telle que la triéthylamine, la N-méthylmorpholine ou la pyridine. La réaction s'effectue dans un solvant tel que le dichlorométhane, le 1,2-dichloroéthane, le tétrahydrofurane, le dioxane ou le N,N-diméthylformamide, à une température comprise entre -70°C et la température ambiante.

Les composés de formule (VIII) se préparent par réaction des composés de formule :

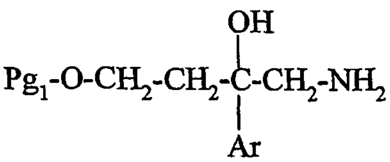
(IX)

dans laquelle Ar est tel que défini pour un composé de formule (I) et $Pg_1$ est tel que défini pour un composé de formule (VI), avec un composé de formule :

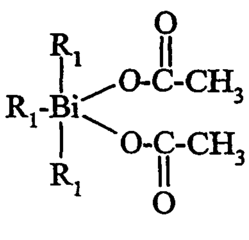
(X)

dans laquelle $R_1$ est tel que défini pour un composé de formule (I), en présence de dipivaloate de cuivre (II) selon les méthodes d'arylation d'amine décrites dans Tetrahedron, 1997, 53 (12), 4137-4144 ; Tetrahedron Letters, 1996, 37 (19), 3295-3298 ; Tetrahedron, 1998, 54, 4313-4318.

Les composés de formule (IX) sont connus et se préparent selon les méthodes décrites dans WO 96/23787 ou dans WO 00/58292.

Les composés de formule (X) se préparent à partir de triarylbismuthane de formule :

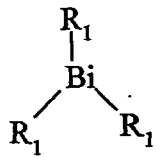

(XI)

dans laquelle $R_1$ est tel que défini pour un composé de formule (I) selon des méthodes connues telles que celles décrites dans Synthetic Communications, 1996, 26 (24), 4569-4575 ou dans la littérature précédemment citée pour la réaction d'arylation.

Les composés de formule (XI) sont commerciaux ou se préparent selon les méthodes décrites dans Synthetic Communications, 1996, 26 (24), 4569-4575.

Les composés de formule (III) sont connus ou se préparent selon des méthodes connues telles que celles décritent dans EP-0 428 434, EP-0 512 901, EP-0 515 240, WO 96/23787, WO 02/094821 et WO 03/104225.

Les composés de formule (III) sont généralement préparés sous forme protégée sur l'atome d'azote de la pipéridine ; après une étape de déprotection, on obtient les composés de formule (III) attendus.

De façon particulière on prépare les composés de formule (III) dans laquelle $R_3$ représente un groupe $-(CH_2)_q-NR_7COR_8$ dans lequel $R_7$ et $R_8$ ensemble représentent un groupe $-(CH_2)_p-$ selon le SCHEMA I ci-après, dans lequel $Pg_2$ représente un groupe N-protecteur tel qu'un benzyle, un benzyloxycarbonyle ou un *tert*-butyloxycarbonyle, Hal représente un atome d'halogène, de préférence le chlore et $R_2$, q et p étant tels que définis pour un composé de formule (I).

SCHEMA I

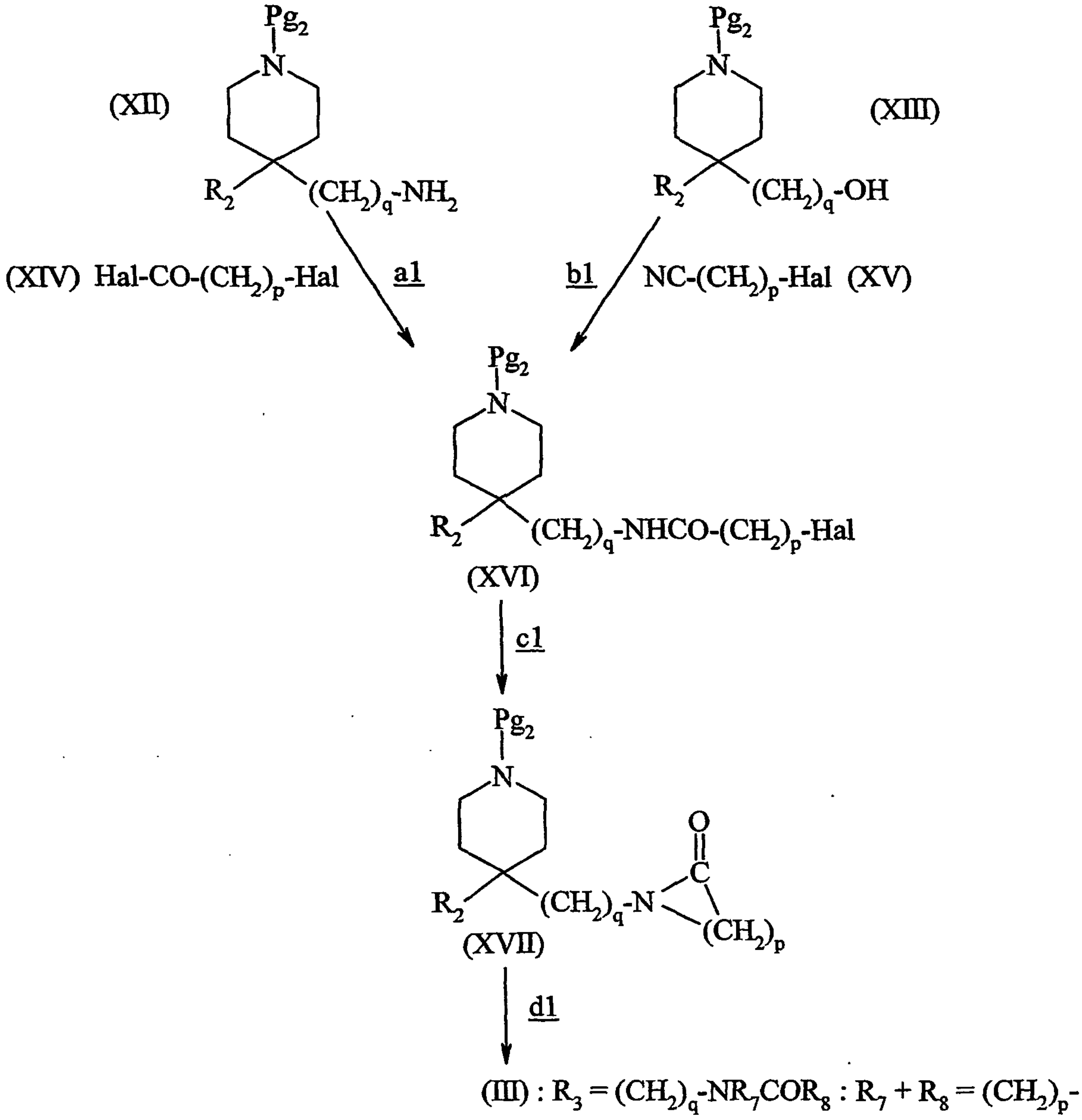


A l'étape a1 du SCHEMA I on fait réagir un composé de formule (XII) avec un composé de formule (XIV) en présence d'une base telle que la triéthylamine, dans un solvant tel que l'acétonitrile ou le dichlorométhane et à une température comprise entre 0°C et la température ambiante pour obtenir un composé de formule (XVI).

On peut également obtenir un composé de formule (XVI) par réaction, à l'étape b1, d'un composé de formule (XIII) avec un composé de formule (XV), en présence d'un acide fort tel que l'acide sulfurique, sans solvant et à une température comprise entre 0 et 5 °C.

La cyclisation, à l'étape c1, du composé de formule (XVI) pour donner le composé de formule (XVII), s'effectue en présence d'une base telle que l'hydrure de sodium, dans un solvant tel que le N,N-diméthylformamide et à une température comprise entre la température ambiante et 60°C.

Le composé de formule (XVII) est déprotégé à l'étape d1 selon les méthodes connues pour donner les composés de formule (III) attendus.

On prépare les composés de formule (III) dans laquelle $R_3$ représente un groupe - $(CH_2)_q$-$NR_7COOR_9$ dans lequel $R_7$ et $R_9$ ensemble représentent un groupe -$(CH_2)_n$-selon le SCHEMA II ci-après, dans lequel $Pg_2$ représente un groupe N-protecteur tel que défini précédemment, Hal représente un atome d'halogène de préférence le chlore et $R_2$, q et n étant tels que définis pour un composé de formule (I).

SCHEMA II

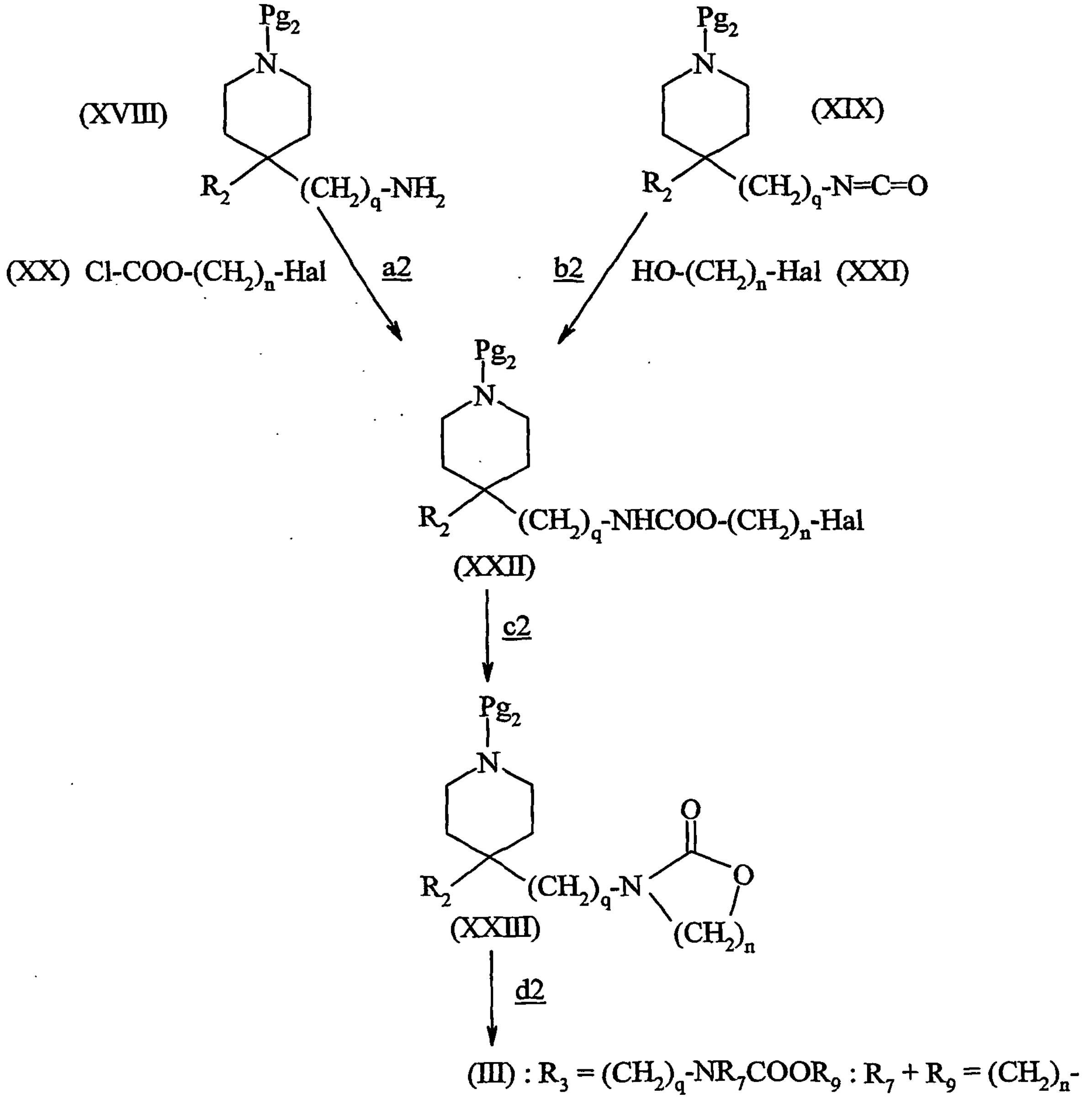


A l'étape a2 du SCHEMA II, on fait réagir un composé de formule (XVIII) avec un composé de formule (XX), en présence d'une base telle que la triéthylamine, dans un solvant tel que le dichlorométhane ou le 1,2-dichloroéthane et à une température comprise entre 0°C et la température ambiante, pour obtenir un composé de formule (XXII).

On peut également obtenir un composé de formule (XXII) par réaction, à l'étape b2, d'un composé de formule (XIX) avec un composé de formule (XXI), dans un solvant tel que le 1,2-dichloroéthane et à la température de reflux du solvant.

Le composé de formule (XIX) s'obtient par action de l'azidure de sodium sur le chlorure d'acide correspondant selon la méthode décrite dans Organic Synthèses, 51, 48-52.

La cyclisation, à l'étape c2, du composé de formule (XXII) pour donner le composé de formule (XXIII) s'effectue en présence d'une base telle que l'hydrure de sodium, dans un solvant tel que le N,N-diméthylformamide et à une température comprise entre la température ambiante et 60°C.

Le composé de formule (XXIII) est déprotégé à l'étape d2 selon les méthodes classiques pour donner le composé de formule (III) attendu.

On prépare les composés de formule (III) dans laquelle $R_3$ représente un groupe $-(CH_2)_q-NR_7CONR_{11}R_{12}$ dans lequel $R_7$ et $R_{12}$ ensemble représentent un groupe $-(CH_2)_m-$ selon le SCHEMA III ci-après dans lequel $Pg_2$ représente un groupe N-protecteur tel que défini précédemment, Hal représente un atome d'halogène, de préférence le chlore et $R_2$, q et m étant tels que définis pour un composé de formule (I).

## SCHEMA III

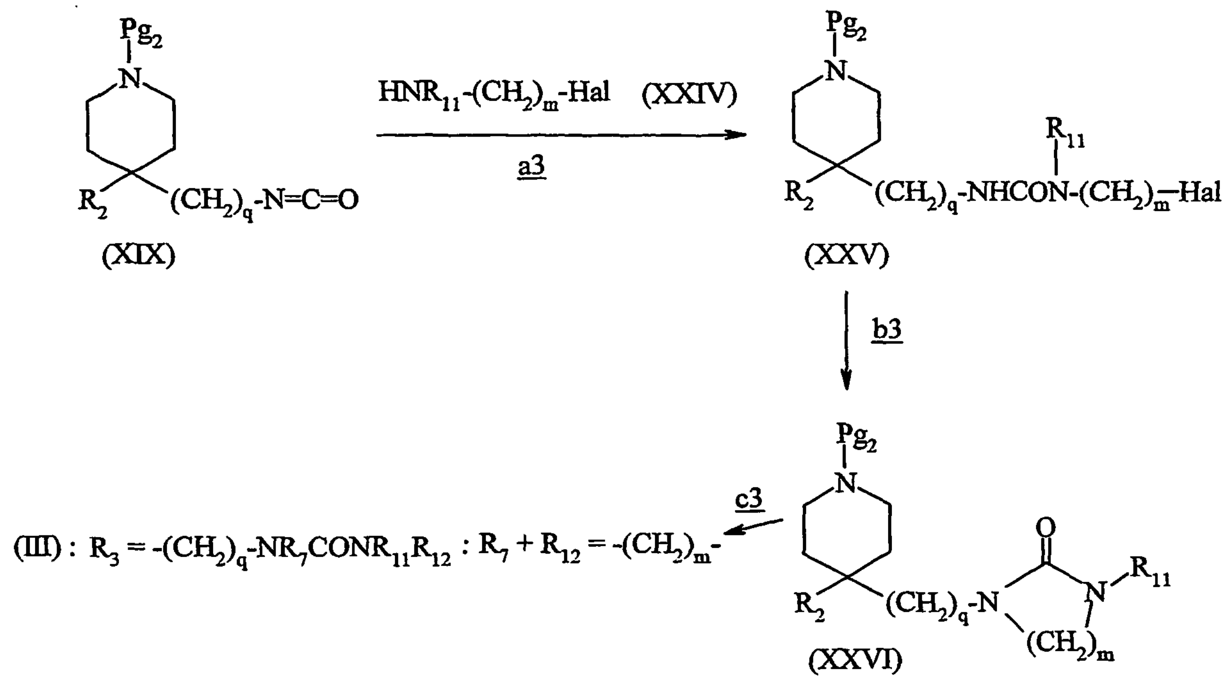


A l'étape a3 du SCHEMA III, on fait réagir un composé de formule (XIX) avec un composé de formule (XXIV) dans un solvant tel que le 1,2-dichloroéthane et à une température comprise entre la température ambiante et la température de reflux du solvant.

La cyclisation, à l'étape b3, du composé de formule (XXV) pour donner le composé de formule (XXVI) s'effectue en présence d'une base telle que l'hydrure de sodium, dans un solvant tel que le N,N-diméthylformamide et à une température comprise entre la température ambiante et 60°C.

Le composé de formule (XXVI) est déprotégé à l'étape c3 selon les méthodes classiques pour donner le composé de formule (III) attendu.

Les composés de formule (IV) se préparent par réaction d'un composé de formule (V) avec un composé de formule :

$Y\text{-}SO_2\text{-}Cl$ (XXVII)

dans laquelle Y représente un groupe méthyle, phényle, tolyle ou trifluorométhyle, en présence d'une base telle que la triéthylamine, la N,N-diisopropyléthylamine ou la pyridine, dans un solvant tel que le dichlorométhane ou le toluène, et à une température comprise entre -20°C et la température de reflux du solvant.

L'invention, selon un autre de ses aspects, a également pour objet les composés de formule (II), (IV), (V), (VI), (VII), (VITI) et certains composés de formule (III). Ces composés sont utiles comme intermédiaire de synthèse des composés de formule (I). Ainsi, l'invention a pour objet des composés de formule :

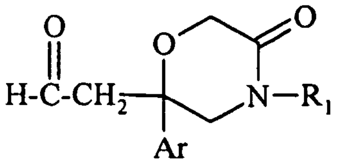
(II)

dans laquelle:

- Ar représente un phényle disubstitué par un atome d'halogène ;
- $R_1$ représente un phényle non substitué ou substitué une ou deux fois par un atome d'halogène.

L'invention a aussi pour objet des composés de formule :

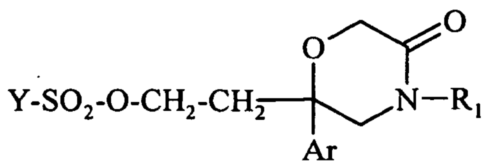

(IV)

dans laquelle :

- Y représente un groupe méthyle, phényle, tolyle, trifluorométhyle ;
- Ar représente un phényle disubstitué par un atome d'halogène ;
- $R_1$ représente un phényle non substitué ou substitué une ou deux fois par un atome d'halogène.

L'invention a aussi pour objet des composés de formule :

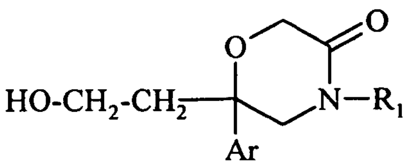

(V)

dans laquelle :

- Ar représente un phényle disubstitué par un atome d'halogène ;
- $R_1$ représente un phényle non substitué ou substitué une ou deux fois par un atome d'halogène.

L'invention a aussi pour objet des composés de formule :

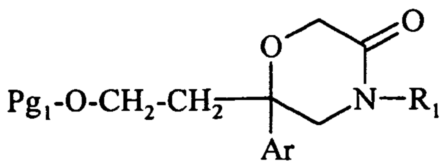

(VI)

dans laquelle :

- $Pg_1$ représente un radical tétrahydropyran-2-yle, benzoyle ou ($C_1$-$C_4$)alkylcarbonyle ;
- Ar représente un phényle disubstitué par un atome d'halogène ;
- $R_1$ représente un phényle non substitué ou substitué une ou deux fois par un atome d'halogène.

L'invention a aussi pour objet des composés de formule :

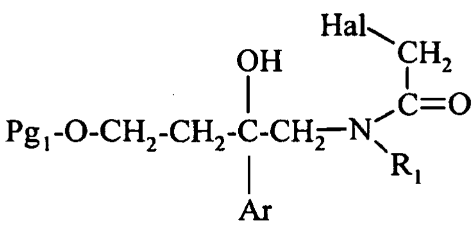

(VII)

dans laquelle :

- Hal représente un atome de chlore ou de brome ;
- $Pg_1$ représente un radical tétrahydropyran-2-yle, benzoyle ou ($C_1$-$C_4$)alkylcarbonyle ;
- Ar représente un phényle disubstitué par un atome d'halogène ;
- $R_1$ représente un phényle non substitué ou substitué une ou deux fois par un atome d'halogène.

L'invention a aussi pour objet des composés de formule :

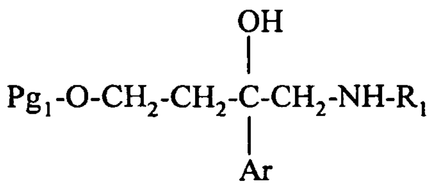 (VIII)

dans laquelle :

- $Pg_1$ représente un radical tétrahydropyran-2-yle, benzoyle ou ($C_1$-$C_4$)alkylcarbonyle ;
- Ar représente un phényle mono- ou disubstitué par un atome d'halogène ;
- $R_1$ représente un phényle non substitué ou substitué une ou deux fois par un atome d'halogène.

L'invention a aussi pour objet des composés de formule :

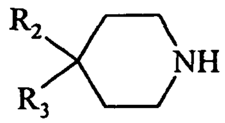 (III)

dans laquelle :

- $R_2$ représente :
    - un pyridyle ;
    - un phényle non substitué ou substitué une ou deux fois par un ou deux substituants choisis indépendamment parmi un atome d'halogène, un ($C_1$-$C_4$)alkyle, un ($C_1$-$C_4$)alcoxy, un groupe trifluorométhyle, un groupe trifluorométhoxy ;
- $R_3$ représente un groupe :

    (11) $-(CH_2)_q-NR_7COOR_9$;

- q est 0 ;
- $R_7$ et $R_9$ ensemble représentent un groupe $-(CH_2)_n-$ ;
- n est 2 ou 3;

à l'état de base ou de sel d'addition à un acide.

La résolution des mélanges racémiques des composés de formule (I) permet d'isoler les énantiomères.

Il est cependant préférable d'effectuer le dédoublement des mélanges racémiques à partir des composés de formule (IX) selon les méthodes décrites dans WO 96/23787 dans WO 00/58292.

Les EXEMPLES suivants décrivent la préparation de certains composés conformes à l'invention. Ces exemples ne sont pas limitatifs et ne font qu'illustrer la présente invention. Les numéros des composés exemplifies renvoient à ceux donnés dans le TABLEAU (I) ci-après, qui illustre les structures chimiques et les propriétés physiques de quelques composés selon l'invention.

Dans les Préparations et dans les Exemples on utilise les abréviations suivantes :

éther : éther diéthylique
éther iso : éther diisopropylique
DMSO : diméthylsulfoxyde
DMF : N,N-diméthylformamide
THF : tétrahydrofurane
DCM : dichlorométhane
AcOEt : acétate d'éthyle
DIPEA : diisopropyléthylamine
TFA : acide trifluoroacétique

BOP : benzotriazol-1-yloxytris(diméthylamino)phosphoniumhexafluoro phosphate
Ether chlorhydrique 2N : solution 2N d'acide chlorhydrique dans l'éther diéthylique
F : point de fusion
TA : température ambiante
Eb : température d'ébullition
CLHP : chromatographie liquide haute performance
Silice H : gel de silice 60 H commercialisé par Merck (DARMSTAD)
Solution tampon pH = 2 : solution de 16,66 g de $KHSO_4$ et 32,32 g de $K_2SO_4$ dans 1 litre d'eau.

Les spectres de résonance magnétique nucléaire du proton (RMN $^1H$) sont enregistrés à 200 MHz dans le DMSO-$d_6$. Les déplacements chimiques $\delta$ sont exprimés en parties par million (ppm). Pour l'interprétation des spectres, on utilise les abréviations suivantes : s : singulet, d : doublet, t : triplet, q : quadruplet : m : massif, mt : multiplet, se : singulet élargi, dd : doublet dédoublé.

Les composés selon l'invention sont analysés par couplage LC/UV/MS (chromatographie liquide/détection UV/ spectrométrie de masse). On mesure le pic moléculaire ($MH^+$) et le temps de rétention (tr) en minutes.

On utilise une colonne Symmetry C18 de 2,1 x 50 mm, 3,5 $\mu$m, à 30°C, débit 0,4 mL/minute.

L'éluant est composé comme suit :

- solvant A : 0,005 % d'acide trifluoroacétique (TFA) dans l'eau à pH 3,15 ;
- solvant B : 0,005 % de TFA dans l'acétonitrile.

Gradient :

| Temps (mn) | % A | % B |
|---|---|---|
| 0 | 100 | 0 |
| 10 | 10 | 90 |
| 15 | 10 | 90 |
| 16 | 100 | 0 |
| 20 | 100 | 0 |

La détection UV est effectuée à $\lambda$ = 210 nM et la détection de masse en mode ionisation chimique ESI positif.

PREPARATIONS

**1. Préparations des composés de formule (X).**

Préparation 1.1

Bis(acétato)triphénylbismuth.

(X) : $R_1$ = 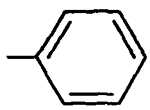

A une solution de 100 g de triphénylbismuthane dans 720 ml d'un mélange DCM/THF (70/30 ; v/v), on ajoute, à TA et goutte à goutte, 56,6 ml d'une solution à 32 % d'acide peracétique dans l'acide acétique et laisse 2 heures sous agitation à TA. On ajoute 200 ml d'éther au mélange réactionnel, essore le précipité formé et le lave à l'éther. On obtient 72,2 g du produit attendu.

Préparation 1.2

Bis(acétato)tris(4-chlorophényl)bismuth.

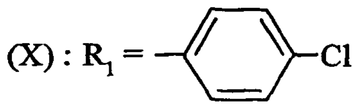

A) Tris(4-chlorophényl)bismuthane.

A un mélange de 2,54 g de magnésium recouvert de THF on ajoute, goutte à goutte et à TA, une solution de 20 g de 1-bromo-4-chlorobenzène dans 150 ml de THF et chauffe à reflux pendant 1 heure. On refroidit le mélange réactionnel à 0°C, ajoute, goutte à goutte, une solution de 9,9 g de $BiCl_3$ dans 50 ml de THF, chauffe à reflux pendant 1 heure et laisse une nuit sous agitation à TA. On verse le mélange réactionnel dans une solution aqueuse à 10 % de $NH_4Cl$ saturée en NaCl, filtre sur Célite®, extrait au DCM, sèche la phase organique sur $MgSO_4$ et évapore le solvant sous vide. On obtient 15,5 g du composé attendu. B) Bis(acétato)tris(4-chlorophényl)bismuth.

A un mélange de 13 g du composé obtenu à l'étape précédente dans 250 ml d'acide acétique on ajoute, à TA, 7,1 g de perborate de sodium monohydrate et laisse 1 heure sous agitation à TA. On verse le mélange réactionnel sur 500 ml d'eau, extrait au DCM, lave la phase organique à l'eau, sèche sur $MgSO_4$ et évapore le solvant sous vide. On obtient 15,5 g du composé attendu.

Préparation 1.3

Bis(acétato)tris(4-fluorophényl)bismuth.

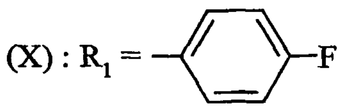

A) Tris(4-fluorophényl)bismuthane.

A un mélange de 5,6 g de magnésium recouvert de THF on ajoute, goutte à goutte et à TA, une solution de 40 g de 1-bromo-4-fluorobenzène dans 250 ml de THF puis chauffe à reflux pendant 1 heure. On refroidit le mélange réactionnel à 0°C, ajoute, goutte à goutte, une solution de 21,6 g de $BiCl_3$ dans 100 ml de THF, chauffe à reflux pendant 1 heure et laisse une nuit sous agitation à TA. On verse le mélange réactionnel dans une solution aqueuse à 10 % de $NH_4Cl$ saturée en NaCl, filtre sur Célite®, extrait au DCM, sèche la phase organique sur $MgSO_4$ et évapore le solvant sous vide. On obtient 29,75 g du composé attendu. B) Bis(acétato)tris(4-fluorophényl)bismuth.

A un mélange de 25 g du composé obtenu à l'étape précédente dans 100 ml d'acide acétique on ajoute 2,3 g de perborate de sodium tétrahydrate et laisse 1 heure sous agitation à TA. On verse le mélange réactionnel sur 1 litre d'eau, extrait au DCM, lave la phase organique à l'eau, sèche sur $MgSO_4$ et évapore le solvant sous vide. On obtient 27,34 g du composé attendu.

Préparation 1.4

Bis(acétato)tris(3,4-difluorophényl)bismuth.

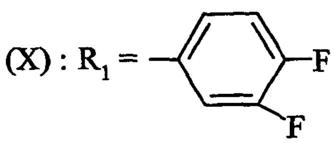

A) Tris(3,4-difluorophényl)bismuthane.

A un mélange de 2,5 g de magnésium recouvert de THF, on ajoute, goutte à goutte et à TA, une solution de 20 g de 1-bromo-3,4-difluorobenzène dans 150 ml de THF puis chauffe à reflux pendant 1 heure. On refroidit le mélange réactionnel à 0°C, ajoute, goutte à goutte, une solution de 9,8 g de $BiCl_3$ dans 50 ml de THF, chauffe à reflux pendant 1 heure et laisse une nuit sous agitation à TA. On verse le mélange réactionnel dans une solution aqueuse à 10 % de $NH_4Cl$ saturée en NaCl, filtre sur Célite®, extrait le filtrat au DCM, sèche la phase organique sur $MgSO_4$ et évapore le solvant sous vide. On obtient 15,75 g du composé attendu. B) Bis(acétato)tris(3,4-difluorophényl)bismuth.

A un mélange de 13,5 g du composé obtenu à l'étape précédente dans 250 ml d'acide acétique on ajoute 7,4 g de perborate de sodium monohydrate et laisse 1 heure sous agitation à TA. On verse le mélange réactionnel sur 500 ml d'eau, extrait au DCM, lave la phase organique à l'eau, sèche sur $MgSO_4$ et évapore le solvant sous vide. On obtient 15,5 g du composé attendu.

**2. Préparations des composés de formule (IX).**

Préparation 2.1

Benzoate de 4-amino-3-(3,4-dichlorophényl)-3-hydroxybutyle, isomère lévogyre.

(IX) : Ar = 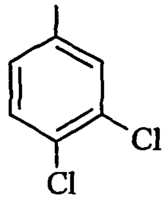 ; $Pg_1$ = 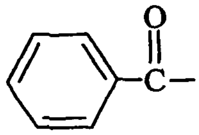

On prépare ce composé en suivant les modes opératoires décrits aux étapes A, B, C, D, E et F de la Préparation 1.1 dans WO 00/58292.

Préparation 2.2

Benzoate de 4-amino-3-(3,4-difluorophényl)-3-hydroxybutyle, isomère lévogyre.

(IX) : Ar = 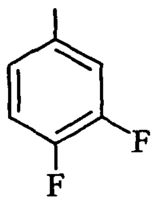 ; $Pg_1$ = 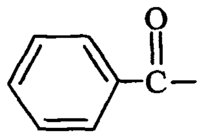

On prépare ce composé selon le mode opératoire décrit à l'étape A de la Préparation 1.16 dans WO 96/23787.

Préparation 2.3

Benzoate de 4-amino-3-(3,4-dichlorophényl)-3-hydroxybutyle.

(IX) : Ar = 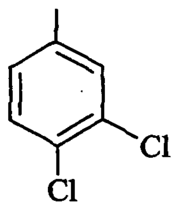 ; $Pg_1$ = 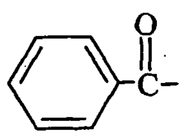

On prépare ce composé en suivant les modes opératoires décrits aux étapes A, B, C, D et E de la Préparation 1.1 dans WO 00/58292.

**3. Préparations des composés de formule (VIII).**

Préparation 3.1

Benzoate de 4-anilino-3-(3,4-dichlorophényl)-3-hydroxybutyle, isomère unique.

(VIII) : Ar = 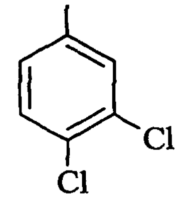 ; $R_1$ = 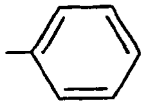 ; $Pg_1$ = 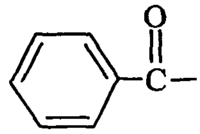

A) Dipivaloate de cuivre II.

A une suspension de 13,6 g de $CuCO_3.Cu(OH)_2$ dans 50 ml d'eau, on ajoute, à TA et goutte à goutte, une solution de 18,6 g d'acide pivalique dans 100 ml d'eau chaude et laisse sous agitation jusqu'à la fin du dégagement gazeux. On essore le mélange réactionnel et lave quatre fois le précipité à l'eau. On reprend le précipité dans 300 ml de THF, filtre un insoluble et concentre le filtrat sous vide. On obtient 7,2 g du produit attendu après séchage sous vide.

B) Benzoate de 4-anilino-3-(3,4-dichlorophényl)-3-hydroxybutyle, isomère unique.

On laisse une nuit sous agitation à TA un mélange de 17,7 g du composé obtenu à la Préparation 2.1, 30,7 g du composé obtenu à la Préparation 1.1 et 1,25 g du composé obtenu à l'étape précédente dans 960 ml de DCM. On concentre sous vide le mélange réactionnel, reprend le résidu à l'éther, filtre un insoluble et concentre le filtrat sous vide. On chromatographie le résidu sur gel de silice H en éluant au DCM. On obtient 18,6 g du produit attendu.

Préparation 3.2

Benzoate de 4-anilino-3-(3,4-diffuorophényl)-3-hydroxybutyle, isomère unique.

(VIII) : Ar = 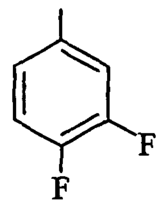 ; $R_1$ = 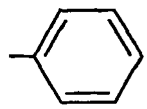 ; $Pg_1$ = 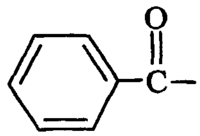

A un mélange de 0,5 g du composé obtenu à la Préparation 2.2 et 0,04 g du composé obtenu à l'étape A de la Préparation 3.1 dans 40 ml de DCM on ajoute à TA 0,95 g du composé obtenu à la Préparation 1.1 et laisse 1 heure sous agitation à TA. On acidifie le mélange réactionnel par ajout d'une solution tampon pH = 2, neutralise à pH = 7 par ajout d'une solution de $Na_2CO_3$ à 10 %, filtre un insoluble, décante le filtrat, lave la phase organique à l'eau, sèche sur $Na_2SO_4$ et évapore le solvant sous vide. On chromatographie le résidu sur gel de silice en éluant au DCM. On obtient 0,3 g du produit attendu.

Préparation 3.3

Benzoate de 4-[(4-chlorophényl)amino]-3-(3,4-dichlorophényl)-3-hydroxybutyle.

(VIII) : Ar = 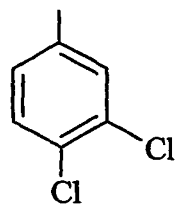 ; $R_1$ = 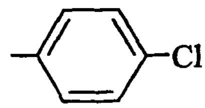 ; $Pg_1$ = 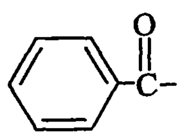

On laisse 18 heures sous agitation à TA un mélange de 15,5 g du composé obtenu à la Préparation 1.2, 8,3 g du composé obtenu à la Préparation 2.3 et 0,62 g du composé obtenu à l'étape A de la Préparation 3.1 dans 750 ml de DCM. On concentre sous vide le mélange réactionnel, reprend le résidu au DCM, filtre un insoluble et chromatographie le filtrat sur gel de silice en éluant au DCM. On obtient 4,62 g du composé attendu.

Préparation 3.4

Benzoate de 3-(3,4-dichlorophényl)-4-([(4-ffuorophényl)amino]-3-hydroxybutyle.

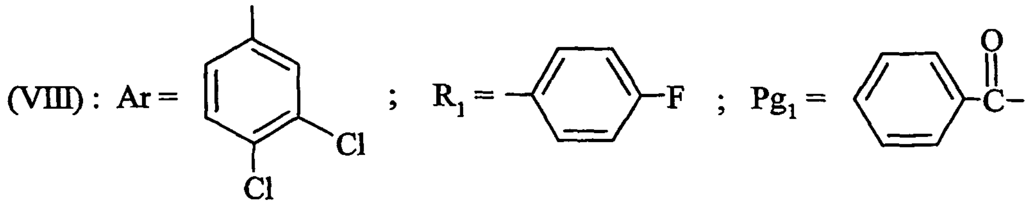

On laisse 18 heures sous agitation à TA un mélange de 10 g du composé obtenu à la Préparation 1.3, 5,78 g du composé obtenu à la Préparation 2.3 et 0,43 g du composé obtenu à l'étape A de la Préparation 3.1 dans 500 ml de DCM. On concentre le mélange réactionnel sous vide, reprend le résidu au DCM, filtre un insoluble et chromatographie le filtrat sur gel de silice en éluant au DCM. On obtient 5,84 g du composé attendu.

Préparation 3.5

Benzoate de 3-(3,4-dichlorophényl)-4-[(3,4-difluorophényl)amino]-3-hydroxybutyle.

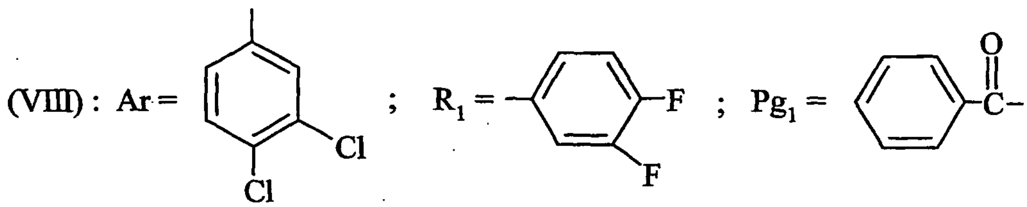

On laisse 18 heures sous agitation à TA un mélange de 15,5 g du composé obtenu à la Préparation 1.4, 8,2 g du composé obtenu à la Préparation 2.3 et 0,62 g du composé obtenu à l'étape A de la Préparation 3.1 dans 750 ml de DCM. On concentre sous vide, reprend le résidu au DCM, filtre un insoluble et chromatographie le filtrat sur gel de silice en éluant au DCM. On obtient 5,42 g du composé attendu.

**4. Préparations des composés de formule (VII).**

Préparation 4.1

Benzoate de 4-[(chloroacétyl)(phényl)amino]-3-(3,4-dichlorophényl)-3-hydroxybutyle, isomère lévogyre.

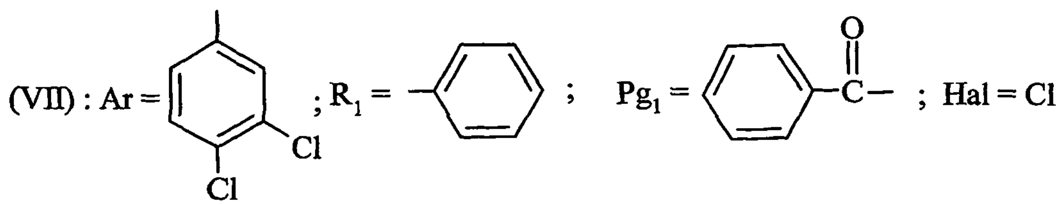

A une solution de 7,2 g du composé obtenu à la Préparation 3.1 et 1,7 g de triéthylamine dans 100 ml de DCM on ajoute, à TA, 1,9 g de chlorure de chloroacétyle et laisse 1 heure sous agitation à TA. On rajoute 0,19 g de chlorure de chloroacétyle et laisse 1 heure sous agitation à TA. On concentre le mélange réactionnel sous vide, extrait le résidu à l'AcOEt, lave la phase organique par une solution tampon pH = 2, à l'eau, sèche sur $Na_2SO_4$ et évapore le solvant sous vide. On obtient 6,42 g du produit attendu. $\alpha_D^{20} = -119,9°$ (c=1; MeOH).

Préparation 4.2

Benzoate de 4-[(chloroacétyl)(phényl)amino]-3-(3,4-difluorophényl)-3-hydroxybutyle, isomère unique.

(VII) : Ar = 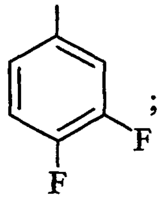 ; $R_1$ = 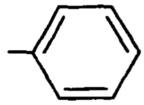 ; $Pg_1$ = 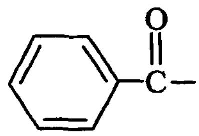 ; Hal = Cl

On refroidit à 0°C un mélange de 3,1 g du composé obtenu à la Préparation 3.2 et 0,79 g de triéthylamine dans 60 ml de DCM, ajoute goutte à goutte une solution de 0,88 g de chlorure de chloroacétyle dans 10 ml de DCM et laisse 2 heures sous agitation. On concentre le mélange réactionel sous vide, extrait le résidu à l'AcOEt, lave la phase organique par une solution tampon pH = 2, à l'eau, sèche sur $Na_2SO_4$ et évapore le solvant sous vide. On obtient 3,5 g du produit attendu.

Préparation 4.3

Benzoate de 4-[(chloroacétyl)(4-chlorophényl)amino]-3-(3,4-dichlorophényl)-3-hydroxybutyle.

(VII) : Ar = 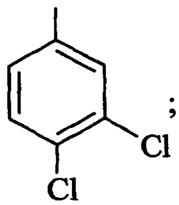 ; $R_1$ = 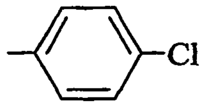 ; $Pg_1$ = 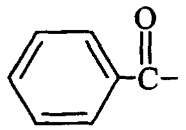 ; Hal = Cl

A une solution de 4,6 g du composé obtenu à la Préparation 3.3 et 1 g de triéthylamine dans 150 ml de DCM on ajoute 1,34 g de chlorure de chloroacétyle et laisse une nuit sous agitation à TA. On concentre sous vide le mélange réactionnel, extrait le résidu à l'AcOEt, lave la phase organique par une solution tampon pH = 2, à l'eau, par une solution saturée de NaCl, sèche sur $MgSO_4$ et évapore le solvant sous vide. On obtient 5,25 g du composé attendu.

Préparation 4.4

Benzoate de 4-[(chloroacétyl)(4-fluorophényl)amino]-3-(3,4-dichlorophényl)-3-hydroxybutyle.

(VII) : Ar = 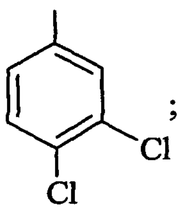 ; $R_1$ = 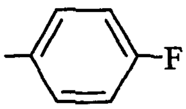 ; $Pg_1$ = 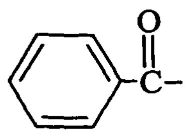 ; Hal = Cl

A un mélange de 8,5 g du composé obtenu à la Préparation 3.4 et 2,64 ml de triéthylamine dans 150 ml de DCM on ajoute 2,6 g de chlorure de chloroacétyle et laisse une nuit sous agitation à TA. On concente le mélange réactionnel sous vide, extrait le résidu à l'AcOEt, lave la phase organique par une solution tampon pH = 2, à l'eau, par une solution saturée de NaCl, sèche sur $MgSO_4$ et évapore le solvant sous vide. On obtient 9,5 g du composé attendu.

Préparation 4.5

Benzoate de 4-[(chloroacétyl)(3,4-difluorophényl)amino]-3-(3,4-dichlorophényl)-3-hydroxybutyle.

(VII) : Ar = 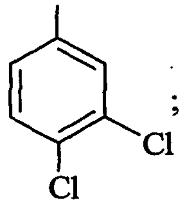 ; $R_1$ = 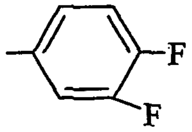 ; $Pg_1$ = 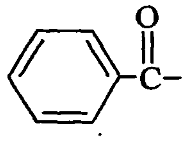 ; Hal = Cl

A un mélange de 5,4 g du composé obtenu à la Préparation 3.5 et 1,17 g de triéthylamine dans 150 ml de DCM on ajoute 1,57 g de chlorure de chloroacétyle et laisse une nuit sous agitation à TA. On concentre sous vide, extrait le résidu à l'AcOEt, lave la phase organique par une solution tampon pH = 2, à l'eau, par une solution saturée de NaCl, sèche sur $MgSO_4$ et évapore le solvant sous vide. On obtient 6,23 g du composé attendu.

**5. Préparations des composés de formule (VI).**

Préparation 5.1

Benzoate de 2-[2-(3,4-dichlorophényl)-5-oxo-4-phénylmorpholin-2-yl]éthyle, isomère unique.

(VI) : Ar = 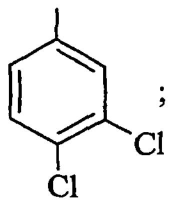 ; $R_1$ = 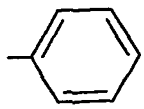 ; $Pg_1$ = 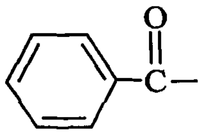

On refroidit à -60°C une solution de 16 g du composé obtenu à la Préparation 4.1 dans 750 ml de THF, ajoute 3,54 g de *tert*-butylate de potassium, laisse sous agitation en laissant revenir la température à -30°C puis laisse 30 minutes sous agitation à - 30°C. On verse le mélange réactionnel sur une solution tampon pH = 2 préalablement refroidie, extrait à l'AcOEt, lave la phase organique à l'eau, sèche sur $Na_2SO_4$ et évapore le solvant sous vide. On obtient 14,85 g du produit attendu.

Préparation 5.2

Benzoate de 2-[2-(3,4-difluorophényl)-5-oxo-4-phénylmorpholin-2-yl]éthyle, isomère unique.

(VI) : Ar = 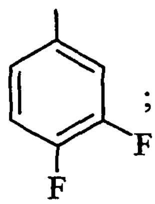 ; $R_1$ = 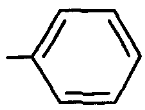 ; $Pg_1$ = 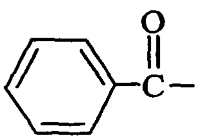

On refroidit à -60°C une solution de 3,5 g du composé obtenu à la Préparation 4.2 dans 120 ml de THF, ajoute 1,75 g de *tert*-butylate de potassium et laisse 1 heure sous agitation. On verse le mélange réactionnel sur une solution tampon pH = 2, extrait à l'AcOEt, lave la phase organique à l'eau, sèche sur $Na_2SO_4$ et évapore le solvant sous vide. On obtient le produit attendu que l'on utilise tel quel à la Préparation 6.2.

Préparation 5.3

Benzoate de 2-[4-(4-chlorophényl)-2-(3,4-dichlorophényl)-5-oxomorpholin-2-yl] éthyle.

(VI) : Ar = 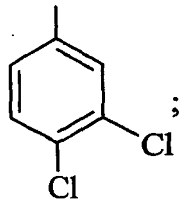 ; $R_1$ = 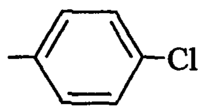 ; $Pg_1$ = 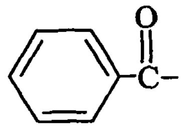

On refroidit à -60°C une solution de 1,28 g de *tert*-butylate de potassium dans 250 ml de THF, puis ajoute, goutte à goutte, une solution de 6,2 g du composé obtenu à la Préparation 4.3 dans 40 ml de THF, laisse sous agitation en laissant remonter la température à -30°C et laisse 1 heure sous agitation à -30°C. On verse le mélange réactionnel sur une solution tampon pH = 2 préalablement refroidie, extrait à l'AcOEt, lave la phase organique à l'eau, par une solution saturée de NaCl, sèche sur $MgSO_4$ et évapore le solvant sous vide. On obtient 5,8 g du composé attendu.

Préparation 5.4

Benzoate de 2-[2-(3,4-dichlorophényl)-4-(4-fluorophényl)-5-oxomorpholin-2-yl] éthyle.

(VI) : Ar = 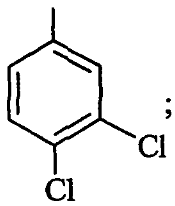 ; $R_1$ = 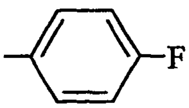 ; $Pg_1$ = 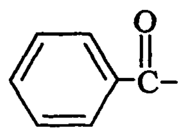

On refroidit à -60°C une solution de 2 g de *tert*-butylate de potassium dans 450 ml de THF, puis ajoute, goutte à goutte, une solution de 9,5 g du composé obtenu à la Préparation 4.4 dans 40 ml de THF, laisse sous agitation en laissant remonter la température à -30°C et laisse 1 heure sous agitation à -30°C. On verse le mélange réactionnel sur une solution tampon pH = 2 préalablement refroidie, extrait à l'AcOEt, lave la phase organique à l'eau, par une solution saturée de NaCl, sèche sur $MgSO_4$ et évapore le solvant sous vide. On obtient 8,4 g du composé attendu.

Préparation 5.5

Benzoate de 2-[2-(3,4-dichlorophényl)-4-(3,4-difluorophényl)-5-oxomorpholin-2-yl]éthyle.

(VI) : Ar = 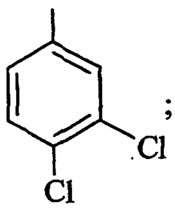 ; $R_1$ = 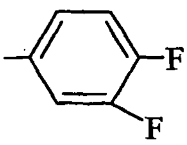 ; $Pg_1$ = 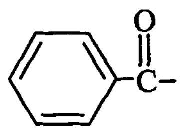

On refroidit à -60°C une solution de 1,08 g de *tert*-butylate de potassium dans 250 ml de THF, puis ajoute, goutte à goutte, une solution du composé obtenu à la Préparation 4.5 dans 40 ml de THF, laisse sous agitation en laissant remonter la température à -30°C et laisse 1 heure sous agitation à -30°C. On verse le mélange réactionnel sur une solution tampon pH = 2 préalablement refroidie, extrait à l'AcOEt, lave la phase organique à l'eau, par une solution saturée de NaCl, sèche sur $MgSO_4$ et évapore le solvant sous vide. On obtient 4,78 g du composé attendu.

**6. Préparations des composés de formule (V).**

Préparation 6.1

6-(3,4-Dichlorophényl)-6-(2-hydroxyéthyl)-4-phénylmorpholin-3-one, isomère dextrogyre.

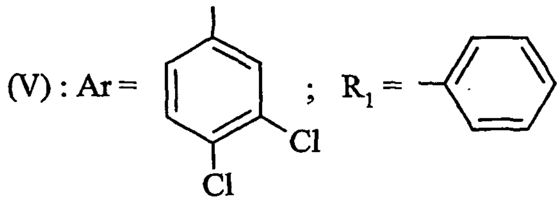

A une solution de 14,5 g du composé obtenu à la Préparation 5.1 dans 100 ml de MeOH, on ajoute, à TA, 0,74 g d'hydroxyde de lithium monohydrate et laisse 1 heure sous agitation à TA. On concentre le mélange réactionnel sous vide, reprend le résidu à l'eau, extrait à l'éther, lave la phase organique à l'eau, sèche sur $Na_2SO_4$ et évapore le solvant sous vide. On chromatographie le résidu sur gel de silice H en éluant au DCM puis par le gradient du mélange DCM/MeOH jusqu'à (98,5/1,5 ; v/v). On obtient 9 g du produit attendu. $\alpha_D^{20} = +96,6°$ (c=1; MeOH).

Préparation 6.2

6-(3,4-Diffuorophényl)-6-(2-hydroxyéthyl)-4-phénylmorpholin-3-one, isomère unique.

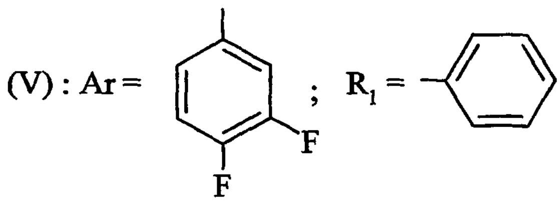

A une solution du composé obtenu à la Préparation 5.2 dans 30 ml de MeOH, on ajoute, à TA, 3 ml d'une solution de NaOH concentrée et laisse 1 heure sous agitation à TA. On concentre sous vide, reprend le résidu à l'eau, extrait à l'AcOEt, lave la phase organique à l'eau, sèche sur $Na_2SO_4$, et évapore le solvant sous vide. On chromatographie le résidu sur gel de silice H en éluant par le mélange DCM/MeOH (100/1 ; v/v). On obtient 1,3 g du produit attendu.

Préparation 6.3

4-(4-Chlorophényl)-6-(3,4-dichlorophényl)-6-(2-hydroxyéthyl)morpholin-3-one.

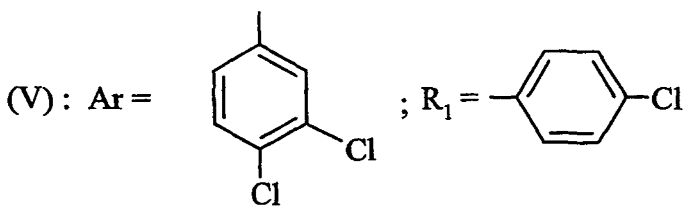

A une solution de 5,8 g du composé obtenu à la Préparation 5.3 dans 75 ml de MeOH on ajoute, à TA, 0,48 g d'hydroxyde de lithium monohydrate et 2 ml d'eau puis laisse 3 heures sous agitation à TA. On concentre le mélange réactionnel sous vide, reprend le résidu à l'eau, extrait à l'éther, lave la phase organique à l'eau, par une solution saturée de NaCl, sèche sur $MgSO_4$ et évapore le solvant sous vide. On obtient 4,7 g du composé attendu.

Préparation 6.4

6-(3,4-Dichlorophényl)-4-(4-fluorophényl)-6-(2-hydroxyéthyl)morpholin-3-one.

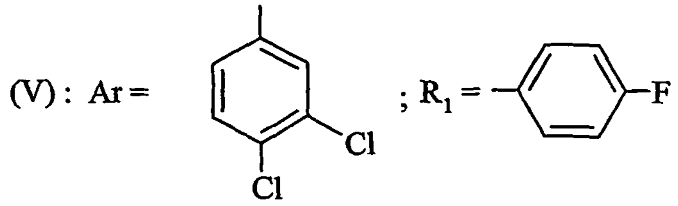

A une solution de 8,4 g du composé obtenu à la Préparation 5.4 dans 75 ml de MeOH, on ajoute 0,72 g d'hydroxyde de lithium monohydrate et 2 ml d'eau et laisse 3 heures sous agitation à TA. On concentre le mélange réactionnel sous vide, reprend le résidu à l'eau, extrait à l'éther, lave la phase organique à l'eau, par une solution saturée de NaCl, sèche sur $MgSO_4$ et évapore le solvant sous vide. On obtient 6,06 g du composé attendu.

Préparation 6.5

6-(3,4-Dichlorophenyl)-4-(3,4-difluorophényl)-6-(2-hydroxyéthyl)morpholin-3-one.

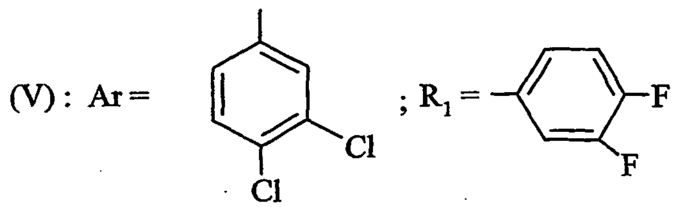

A une solution de 4,75 g du composé obtenu à la Préparation 5.5 dans 75 ml de MeOH on ajoute 0,4 g d'hydroxyde de lithium monohydrate et 2 ml d'eau puis laisse 3 heures sous agitation à TA. On concentre sous vide, reprend le résidu à l'eau, extrait à l'éther, lave la phase organique à l'eau, par une solution saturée de NaCl, sèche sur $MgSO_4$ et évapore le solvant sous vide. On obtient 3,8 g du composé attendu.

**7. Préparations des composés de formule (II).**

Préparation 7.1

[2-(3,4-Dichlorophényl)-5-oxo-4-phénylmorpholin-2-yl]acétaldéhyde, isomère unique.

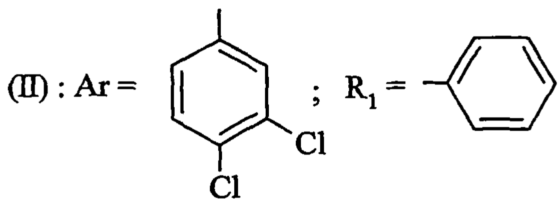

On refroidit à -60°C une solution de 1,6 g du composé obtenu à la Préparation 6.1 et 1,86 ml de DMSO dans 40 ml de DCM, ajoute, goutte à goutte, une solution de 1,1 g de chlorure d'oxalyle dans 20 ml de DCM et laisse 2 heures sous agitation à -60°C. On ajoute ensuite 1,4 g de triéthylamine et laisse sous agitation en laissant remonter la température à TA. On lave le mélange réactionnel par une solution d'HCl 1N, à l'eau, sèche la phase organique sur $Na_2SO_4$ et évapore le solvant sous vide. On obtient 1,6 g du produit attendu.

Préparation 7.2

[2-(3,4-Difluorophényl)-5-oxo-4-phénylmorpholin-2-yl]acétaldéhyde, isomère unique.

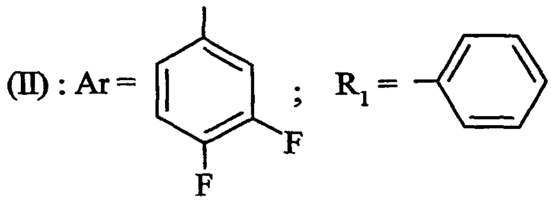

On refroidit à -70°C une solution de 1,14 g de chlorure d'oxalyle dans 20 ml de DCM, ajoute, goutte à goutte, une solution de 1,5 g du composé obtenu à la Préparation 6.2 et 2,1 g de DMSO dans 40 ml de DCM et laisse 3 heures sous agitation à -70°C. On ajoute ensuite 6,5 g de triéthylamine et laisse sous agitation en laissant remonter la température à TA. On lave le mélange réactionnel par une solution d'HCl 2N, à l'eau, sèche sur $Na_2SO_4$ et évapore le solvant sous vide. On obtient 1,4 g du produit attendu.

Préparation 7.3

[4-(4-Chlorophényl)-2-(3,4-dichlorophényl)-5-oxomorpholin-2-yl]acétaldéhyde.

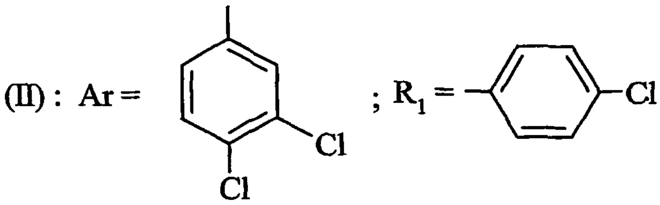

On refroidit à -60°C une solution de 2,05 ml de chlorure d'oxalyle dans 80 ml de DCM, ajoute, goutte à goutte, une solution de 2,50 ml de DMSO dans 20 ml de DCM, puis, goutte à goutte, une solution de 4,7 g du composé obtenu à la Préparation 6.3 et 3,5 ml de DMSO dans 40 ml de DCM et laisse 1 heure sous agitation à -60°C. On ajoute ensuite 10,1 ml de triéthylamine et laisse sous agitation en laissant remonter la température à TA. On lave le mélange réactionnel par une solution d'HCl 1N, par une solution de $Na_2CO_3$ à 10 %, par une solution saturée de NaCl, sèche sur $MgSO_4$ et évapore le solvant sous vide. On chromatographie le résidu sur gel de silice en éluant au DCM puis par le gradient du mélange DCM/MeOH de (99/1 ; v/v) à (95/5 ; v/v). On obtient 2,58 g du composé attendu.

Préparation 7.4

[2-(3,4-Dichlorophényl)-4-(4-fluorophényl)-5-oxomorpholin-2-yl]acétaldéhyde.

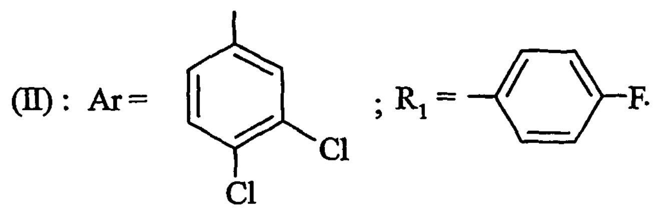

On refroidit à -60°C un mélange de 2,72 ml de chlorure d'oxalyle dans 80 ml de DCM, ajoute, goutte à goutte, une solution de 3,32 ml de DMSO dans 20 ml de DCM, puis, goutte à goutte, une solution de 6 g du composé obtenu à la Préparation 6.4 et 4,65 ml de DMSO dans 40 ml de DCM et laisse une heure sous agitation à froid. On ajoute ensuite 13,5 ml de triéthylamine et laisse sous agitation en laissant remonter la température à TA. On lave le mélange réactionnel par une solution d'HCl 1N, par une solution de $Na_2CO_3$ à 10 %, par une solution saturée de NaCl, sèche sur $MgSO_4$ et évapore le solvant sous vide. On chromatographie le résidu sur gel de silice en éluant au DCM puis par le gradient du mélange DCM/MeOH de (99/1 ; v/v) à (95/5 ; v/v). On obtient 3,68 g du composé attendu.

Préparation 7.5

[2-(3,4-Dichlorophényl)-4-(3,4-difluorophényl)-5-oxomorpholin-2-yl] acétaldéhyde.

(II) : Ar = 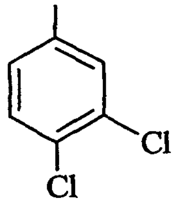 ; $R_1$ = 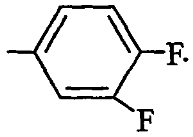

On refroidit à -60°C un mélange de 1,65 ml de chlorure d'oxalyle dans 80 ml de DCM, ajoute, goutte à goutte, une solution de 2 ml de DMSO dans 20 ml de DCM, puis, goutte à goutte, une solution de 3,8 g du composé obtenu à la Préparation 6.5 et 2,8 ml de DMSO dans 40 ml de DCM et laisse 1 heure sous agitation à froid. On ajoute ensuite 8,15 ml de triéthylamine et laisse sous agitation en laissant remonter la température à TA. On lave le mélange réactionnel par une solution d'HCl 1N, par une solution de $Na_2CO_3$ à 10 %, par une solution saturée de NaCl, sèche sur $MgSO_4$ et évapore le solvant sous vide. On chromatographie le résidu sur gel de silice en éluant par le gradient du mélange DCM/MeOH de (99/1 ; v/v) à (95/5 ; v/v). On obtient 3 g du composé attendu.

**8. Préparations des composés de formule (III).**

Préparation 8.1

N-(4-Phénylpipéridin-4-yl)acétamide.

(III) : $R_2$ = 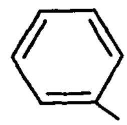 ;

$R_3$ = $NHCOCH_3$

On prépare ce composé selon le mode opératoire décrit dans EP 474 561.

Préparation 8.2

Formiate de N-[4-(3-ffuorophényl)pipéridin-4-yl]acétamide.

(III), HCOOH :

$R_2$ = 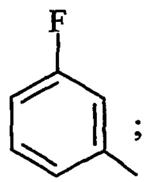 ;

$R_3$ = $-NHCOCH_3$

A) 1-Benzyl-4-(3-fluorophényl)pipéridin-4-ol.

A une suspension de 5,86 g de magnésium dans 25 ml de THF, on ajoute lentement une solution de 42,16 g de 1-bromo-3-fluorobenzène dans 100 ml de THF de manière à atteindre puis à maintenir le reflux du THF et laisse 1 heure à reflux sous agitation. Après refroidissement à TA, on ajoute ensuite, goutte à goutte, une solution de 38 g de 1-benzyl-4-pipéridinone dans 175 ml de THF et chauffe à reflux pendant 1 heure. Après refroidissement à TA, on verse le mélange réactionnel dans de la glace, extrait à l'AcOEt, lave la phase organique à l'eau, sèche sur $Na_2SO_4$ et évapore le solvant sous vide. On obtient 38 g du produit attendu après cristallisation dans le cyclohexane. B) N-[1-Benzyl-4-(3-fluorophényl)pipéridin-4-yl]acétamide.

A une solution de 38 g du composé obtenu à l'étape précédente dans 152 ml d'acétonitrile, on ajoute, goutte à goutte et à une température inférieure à 30°C, 133 ml d'une solution d'$H_2SO_4$ à 95 % puis laisse 3 heures sous agitation à une température comprise entre 25° et 30°C. On verse le mélange réactionnel sur de le glace, alcalinise par ajout d'une solution concentrée de NaOH, essore le précipité formé et le lave à l'eau. On obtient 42,8 g du produit attendu après séchage. C) Formiate de N-[4-(3-fluorophényl)pipéridin-4-yl]acétamide.

On laisse 30 minutes à 40°C puis 1 heure 30 minutes à TA un mélange de 14 g du composé obtenu à l'étape précédente, 16,32 g de formiate d'ammonium et 2,3 g de palladium sur charbon à 10 % dans 100 ml d'éthanol. On filtre le catalyseur et concentre le filtrat sous vide. On obtient 12 g du produit attendu.

Préparation 8.3

Formiate de N-[4-(3,4-difluorophényl)pipéridin-4-yl]acétamide.
(III), HCOOH :

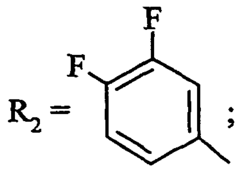

$R_3$ =-NHCOCH$_3$

A) 1-Benzyl-4-(3,4-difluorophényl)pipéridin-4-ol.

A une suspension de 7,55 g de magnésium dans 50 ml de THF, on ajoute, goutte à goutte, une solution de 50 g de 1-bromo-3,4-difluorobenzène dans 100 ml de THF, de manière à atteindre puis à maintenir le reflux et laisse 2 heures à reflux sous agitation. Après refroidissement à TA, on ajoute, goutte à goutte, une solution de 49 g de 1-benzyl-4-pipéridinone dans 100 ml de THF puis laisse une nuit sous agitation à TA. On verse le mélange réactionnel sur une solution saturée de $NH_4Cl$, extrait à l'éther, sèche la phase organique sur $Na_2SO_4$ et évapore le solvant sous vide. On obtient 38,3 g du produit attendu après cristallisation dans le cyclohexane. B) N-[1-Benzyl-4-(3,4-difluorophényl)pipéridin-4-yl]acétamide.
A une solution de 37 g du composé obtenu à l'étape précédente dans 140 ml d'acétonitrile, on ajoute, goutte à goutte et à une température inférieure à 30°C, 120 ml d'une solution d'$H_2SO_4$ à 95 % puis laisse une nuit sous agitation à TA. On verse le mélange réactionnel sur de la glace, alcalinise par ajout d'une solution concentrée de NaOH, extrait au DCM, lave la phase organique à l'eau, par une solution saturée de NaCl, sèche sur $Na_2SO_4$ et évapore le solvant sous vide. On obtient 38,6 g du produit attendu après séchage. C) Formiate de N-[4-(3,4-diffuorophényl)pipéridin-4-yl] acétamide.
On laisse 4 heures sous agitation à TA un mélange de 5 g du composé obtenu à l'étape précédente, 4,65 g de formiate d'ammonium et 0,93 g de palladium sur charbon à 10 % dans 100 ml d'EtOH. On filtre le catalyseur et concentre sous vide le filtrat. On obtient 4,3 g du produit attendu.

Préparation 8.4

Formiate de N-[4-(4-méthylphényl)pipéridin-4-yl]acétamide.
(III), HCOOH:

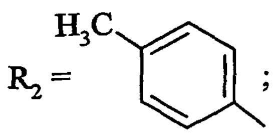

$R_3$= -NHCOCH$_3$

A) 1-Benzyl-4-(4-méthylphényl)pipéridin-4-ol.

On prépare ce composé selon le mode opératoire décrit à l'étape A) de la Préparation 8.3 à partir de 2,6 g de magnésium dans 15 ml de THF, 15 g de 1-bromo-4-méthylbenzène dans 100 ml de THF et 16,6 g de 1-benzyl-4-pipéridinone dans 100 ml de THF. On obtient 22,9 g du produit attendu.

B) N-[1-Benzyl-4-(4-méthylphényl)pipéridin-4-yl]acétamide.

A une solution de 5,4 g du composé obtenu à l'étape précédente dans 30 ml d'acétonitrile, on ajoute, goutte à goutte et à une température inférieure à 30°C, 19 ml d'une solution d'$H_2SO_4$ à 95 % puis laisse une nuit sous agitation à TA. On verse le mélange réactionnel sur de le glace, alcalinise par ajout d'une solution concentrée de NaOH et essore le précipité formé. On obtient 4,51 g du produit attendu. C) Formiate de N-[4-(4-méthylphényl)pipéridin-4-yl]acétamide.

On refroidit au bain de glace un mélange de 3,5 g du composé obtenu à l'étape précédente et 2 g de formiate d'ammonium dans 80 ml de MeOH, ajoute 0,06 g de palladium sur charbon à 10 % et laisse une nuit sous agitation en laissant remonter la température à TA. On filtre le catalyseur et concentre sous vide le filtrat. On obtient 1,63 g du produit attendu après séchage.

Préparation 8.5

Formiate de N-[4-(4-méthoxyphényl)pipéridin-4-yl]acétamide.

(III), HCOOH :

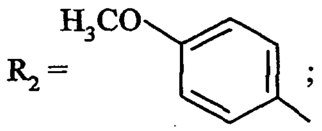

$R_3$ =-NHCOCH$_3$

A) 1-Benzyl-4-(4-méthoxyphényl)pipéridin-4-ol.

On refroidit à -78°C une solution de 44,8 g de 1-bromo-4-méthoxybenzène dans 700 ml de THF, ajoute, goutte à goutte, 168 ml d'une solution 1,6M de n-butyllithium dans l'hexane puis laisse 30 minutes sous agitation à -78°C. On ajoute ensuite, goutte à goutte, une solution de 45,3 ml de 1-benzyl-4-pipéridinone dans 100 ml de THF et laisse 2 heures sous agitation à -78°C. On verse le mélange réactionnel sur 400 ml d'une solution saturée de $NH_4Cl$, extrait à l'éther, lave la phase organique à l'eau, par une solution saturée de NaCl, sèche sur $MgSO_4$ et évapore le solvant sous vide. On obtient 48,6 g du produit attendu après cristallisation dans l'hexane. B) N-[1-Benzyl-4-(4-méthoxyphényl)pipéridin-4-yl]acétamide.

A une solution de 48,6 g du composé obtenu à l'étape précédente dans 189 ml d'acétonitrile, on ajoute, goutte à goutte et à une température inférieure à 27°C, 160 ml d'une solution d'$H_2SO_4$ à 95 % puis laisse 2 heures sous agitation et une nuit au froid. On verse le mélange réactionnel sur de la glace, alcalinise par ajout d'une solution de NaOH à 30 %, essore le précipité formé et le lave à l'eau. On obtient 49,4 g du produit attendu que l'on utilise tel quel. C) Formiate de N-[4-(4-méthoxyphényl)pipéridin-4-yl]acétamide.

On laisse une nuit sous agitation à TA un mélange de 2,58 g du composé obtenu à l'étape précédente, 2,88 g de formiate d'ammonium et 1,7 g de palladium sur charbon à 10 % dans 60 ml d'EtOH. On filtre le catalyseur et concentre sous vide le filtrat. On obtient 0,7 g du produit attendu.

Préparation 8.6

Formiate de N-[4-[4-(trifluorométhoxy)phényl]pipéridin-4-yl]acétamide.

(III), HCOOH :

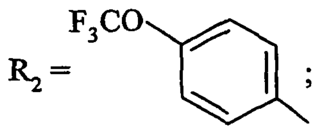

$R_3$ =-NHCOCH$_3$

A) 1-Benzyl-4-[4-(trifluorométhoxy)phényl]pipéridin-4-ol.

On prépare ce composé selon le mode opératoire décrit à l'étape A de la Préparation 8.5 à partir de 20 g de

1-bromo-4-(trifluorométhoxy)benzène dans 300 ml de THF, 89,3 ml d'une solution 1,6M de n-butyllithium dans l'hexane et 24,1 ml de 1-benzyl-4-pipéridinone dans 50 ml de THF. On obtient 18,5 g du produit attendu.

B) N-[1-Benzyl-4-[4-(trifluorométhoxy)phényl]pipéridin-4-yl]acétamide.

A un mélange de 18,43 g du composé obtenu à l'étape précédente dans 61 ml d'acétonitrile, on ajoute, goutte à goutte et à 25°C, 120 ml d'une solution d'$H_2SO_4$ à 95 % puis laisse 3 heures sous agitation. On verse le mélange réactionnel sur de la glace, alcalinise par ajout d'une solution concentrée de NaOH, extrait à l'AcOEt, lave la phase organique à l'eau, sèche sur $Na_2SO_4$ et évapore le solvant sous vide. On reprend le résidu à l'éther et essore le précipité formé. On obtient 9,5 g du produit attendu.

C) Formiate de N-[4-[4-(trifluorométhoxy)phényl]pipéridin-4-yl]acétamide.

On laisse une nuit sous agitation à TA un mélange de 2,75 g du composé obtenu à l'étape précédente, 1,97 g de formiate d'ammonium et 1,2 g de palladium sur charbon à 10 % dans 50 ml d'EtOH. On filtre le catalyseur et concentre sous vide le filtrat. On obtient 2 g du produit attendu.

Préparation 8.7

1-[4-(3-Fluorophényl)pipéridin-4-yl]pyrrolidin-2-one.

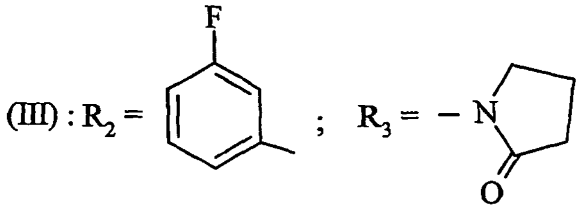

A) 1-Benzyl-4-(3-fluorophényl)pipéridin-4-amine.

On chauffe à reflux pendant 48 heures un mélange de 42,5 g du composé obtenu à l'étape B de la Préparation 8.2 et 250 ml d'une solution d'HCl 2N. On rajoute 100 ml d'une solution d'HCl 4N et poursuit le reflux pendant 72 heures. Après refroidissement à TA, on neutralise le mélange réactionnel par ajout d'une solution concentrée de NaOH, extrait à l'AcOEt, lave la phase organique à l'eau, sèche sur $Na_2SO_4$ et évapore le solvant sous vide. On obtient 37 g du produit attendu. B) N-[1-Benzyl-4-(3-fluorophényl)pipéridin-4-yl]-4-chlorobutanamide.

On laisse 30 minutes sous agitation à TA un mélange de 2,7 g du composé obtenu à l'étape précédente et 1,32 ml de triéthylamine dans 100 ml d'acétonitrile, puis ajoute, goutte à goutte, 1,34 g de chlorure de 4-chlorobutyryle et laisse 2 heures sous agitation à TA. On concentre sous vide, reprend le résidu à l'eau, extrait à l'AcOEt, sèche la phase organique sur $MgSO_4$ et évapore le solvant sous vide. On obtient 3 g du produit attendu. C) 1-[1-Benzyl-4-(3-fluorophényl)pipéridin-4-yl]pyrrolidin-2-one.

On laisse 2 heures sous agitation à TA un mélange de 3 g du composé obtenu à l'étape précédente et 0,617 g d'hydrure de sodium à 60 % dans l'huile dans 60 ml de DMF. On ajoute de l'eau au mélange réactionnel, extrait à l'AcOEt, sèche la phase organique sur $Na_2SO_4$ et évapore le solvant sous vide. On obtient 2,65 g du produit attendu. D) 1-[4-(3-Fluorophényl)pipéridin-4-yl]pyrrolidin-2-one.

On laisse une nuit sous agitation à TA un mélange de 2,65 g du composé obtenu à l'étape précédente, 1,43 g de formiate d'ammonium et 1,2 g de palladium sur charbon à 10 % dans 50 ml d'EtOH. On filtre le catalyseur et concentre sous vide le filtrat. On reprend le résidu par une solution de $Na_2CO_3$ à 10 %, extrait à l'AcOEt, sèche la phase organique sur $Na_2SO_4$ et évapore le solvant sous vide. On obtient 1,6 g du produit attendu après cristallisation dans l'éther.

Préparation 8.8

1-[4-(3,4-Difluorophényl)pipéridin-4-yl]pyrrolidin-2-one.

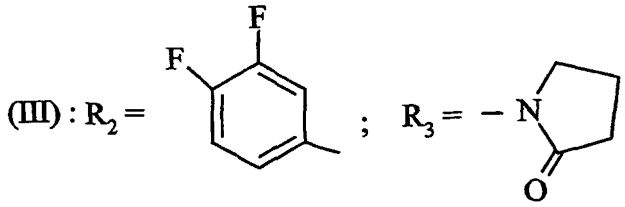

A) 1-Benzyl-4-(3,4-difluorophényl)pipéridin-4-amine.

On chauffe à reflux pendant 48 heures un mélange de 14,5 g du composé obtenu à l'étape B) de la Préparation 8.3 et 100 ml d'une solution d'HCl à 8 %. On concentre sous vide le mélange réactionnel, reprend le résidu dans l'éther et essore le précipité formé. On reprend le précipité par une solution de $Na_2CO_3$ à 10 %, extrait au DCM, sèche la phase organique sur $Na_2SO_4$ et évapore le solvant sous vide. On obtient 10,75 g du produit attendu.

B) N-[1-Benzyl-4-(3,4-difluorophényl)pipéridin-4-yl]-4-chlorobutanamide.

On laisse 30 minutes sous agitation à TA un mélange de 3,11 g du composé obtenu à l'étape précédente et 1,43 ml de triéthylamine dans 50 ml d'acétonitrile, puis ajoute, goutte à goutte, 1,16 g de chlorure de 4-chlorobutyryle et laisse 5 heures sous agitation à TA. On concentre sous vide, extrait le résidu à l'AcOEt, lave la phase organique à l'eau, sèche sur $Na_2SO_4$ et évapore le solvant sous vide. On obtient 3,73 g du produit attendu. C) 1-[1-Benzyl-4-(3,4-difluorophényl)pipéridin-4-yl]pyrrolidin-2-one.

On laisse 3 heures sous agitation à TA un mélange de 3,73 g du composé obtenu à l'étape précédente et 0,73 g d'hydrure de sodium à 60 % dans l'huile dans 60 ml de DMF. On ajoute de l'eau au mélange réactionnel, extrait à l'AcOEt, sèche la phase organique sur $Na_2SO_4$ et évapore le solvant sous vide. On obtient 3,7 g du produit attendu sous forme d'huile orange. D) 1-[4-(3,4-Difluorophényl)pipéridin-4-yl]pyrrolidin-2-one.

On laisse 4 heures sous agitation à TA un mélange de 3,7 g du composé obtenu à l'étape précédente, 1,9 g de formiate d'ammonium et 1,4 g de palladium sur charbon à 10 % dans 60 ml de MeOH. On filtre le catalyseur, concentre sous vide le filtrat, reprend le résidu à l'éther et essore le précipité formé. On obtient 2,5 g du produit attendu.

Préparation 8.9

1-[4-(4-méthylphényl)pipéridin-4-yl]pyrrolidin-2-one.

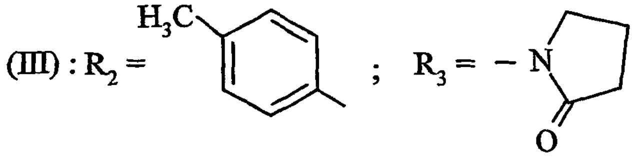

A) Chlorhydrate de N-[1-benzyl-4-(4-méthylphényl)pipéridin-4-yl]-4-chlorobutanamide.

On refroidit à 0°C un mélange de 20 g du composé obtenu à l'étape A de la Préparation 8.4 et 120,7 ml de 4-chlorobutyronitrile, ajoute, goutte à goutte, 50,5 ml d'une solution d'$H_2SO_4$ à 95 % et laisse 2 heures sous agitation à 0-5°C. On verse le mélange réactionnel dans l'eau, alcalinise à pH = 14 par ajout d'une solution concentrée de NaOH, extrait au DCM, lave la phase organique à l'eau, sèche sur $MgSO_4$ et concentre en partie le solvant. On filtre un insoluble, acidifie le filtrat à pH = 1 par ajout d'une solution d'éther chlorhydrique 2N, laisse sous agitation et essore le précipité formé. On obtient 9,8 g du produit attendu après séchage sous vide. B) 1-[1-Benzyl-4-(4-méthylphényl)pipéridin-4-yl] pyrrolidin-2-one.

A une solution de 1,9 g du composé obtenu à l'étape précédente (base libre) dans 60 ml de DMF, on ajoute à TA 0,396 g d'hydrure de sodium à 60 % dans l'huile et laisse une nuit sous agitation à TA. On ajoute de l'eau au mélange réactionnel, extrait à l'AcOEt, sèche la phase organique sur $Na_2SO_4$ et évapore le solvant sous vide. On obtient 1,7 g du produit attendu. C) 1-[4-(4-méthylphényl)pipéridin-4-yl]pyrrolidin-2-one.

On laisse 3 heures sous agitation à TA un mélange de 1,7 g du composé obtenu à l'étape précédente, 0,934 g de formiate d'ammonium et 0,026 g de palladium sur charbon à 10 % dans 100 ml de MeOH. On filtre le catalyseur et concentre sous vide le filtrat. On reprend le résidu dans l'acétone, essore le précipité formé et le lave à l'acétone. On obtient 1,232 g du produit attendu.

Préparation 8.10

1-[4-(4-méthylphényl)pipéridin-4-yl]pipéridin-2-one

(III) : $R_2$ = 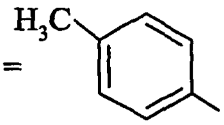 ; $R_3$ = 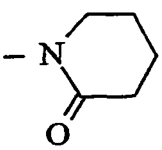

A) Chlorhydrate de N-[1-benzyl-4-(4-méthylphényl)pipéridin-4-yl]-5-chloropentanamide.

On refroidit à 0°C un mélange de 6,8 g du composé obtenu à l'étape A de la Préparation 8.4 et 50,3 g de 5-chloro-n-valéronitrile, ajoute rapidement 17,16 ml d'une solution d'$H_2SO_4$ à 95 % et laisse 4 heures sous agitation à 0-5°C. On verse le mélange réactionnel dans l'eau, alcalinise à pH = 14 par ajout d'une solution concentrée de NaOH, extrait au DCM, lave la phase organique à l'eau, sèche sur $MgSO_4$ et évapore le solvant sous vide. On reprend le résidu par une solution d'éther chlorhydrique 2N, laisse sous agitation et essore le précipité formé. On obtient 2 g du produit attendu.

B) 1-[1-Benzyl-4-(4-méthylphényl)pipéridin-4-yl]pipéridin-2-one.

A une solution de 1 g du composé obtenu à l'étape précédente (base libre) dans 30 ml de DMF, on ajoute à TA 0,2 g d'hydrure de sodium à 60 % dans l'huile et laisse une nuit sous agitation à TA. On ajoute de l'eau au mélange réactionnel, extrait au DCM, lave la phase organique à l'eau, sèche sur $Na_2SO_4$ et évapore le solvant sous vide. On reprend le résidu à l'éther iso et essore le précipité formé. On obtient 0,15 g du produit attendu.

C) 1-[4-(4-méthylphényl)pipéridin-4-yl]pipéridin-2-one

On laisse une nuit sous agitation à TA un mélange de 2 g du composé obtenu à l'étape précédente, 1,04 g de formiate d'ammonium et 0,29 g de palladium sur charbon à 10 % dans 50 ml de MeOH. On filtre le catalyseur et concentre le filtrat sous vide. On obtient 0,7 g du produit attendu.

Préparation 8.11

1-[4-(3-méthylphényl)pipéridin-4-yl]pyrrolidin-2-one.

(III) : $R_2$ = 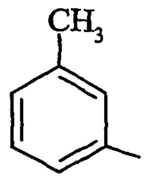 ; $R_3$ = 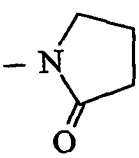

A) 1-Benzyl-4-(3-méthylphényl)pipéridin-4-ol.

On prépare ce composé selon le mode opératoire décrit à l'étape A de la Préparation 8.3 à partir de 3,4 g de magnésium dans 15 ml de THF, d'une solution de 20 g de 1-bromo-3-méthylbenzène dans 100 ml de THF et d'une solution de 22,13 g de 1-benzyl-4-pipéridone dans 100 ml de THF. On obtient 26,54 g du produit attendu.

B) Chlorhydrate de N-[1-benzyl-4-(3-méthylphényl)pipéridin-4-yl]-4-chlorobutanamide.

On prépare ce composé selon le mode opératoire décrit à l'étape A de la Préparation 8.9 à partir de 24 g du composé obtenu à l'étape précédente, 14,5 ml de 4-chlorobutyronitrile et 60,6 ml d'une solution d'$H_2SO_4$ à 95 %. On obtient 8,3 g du produit attendu.

C) 1-[1-Benzyl-4-(3-méthylphényl)pipéridin-4-yl]pyrrolidin-2-one.

A une solution de 7 g du composé obtenu à l'étape précédente (base libre) dans 150 ml de DMF, on ajoute à TA 1,56 g d'hydrure de sodium à 60 % dans l'huile et laisse une nuit sous agitation à TA. On extrait le mélange réactionnel au DCM, lave la phase organique à l'eau, sèche sur $Na_2SO_4$ et évapore le solvant sous vide. On obtient 6,7 g du produit attendu.

D) 1-[4-(3-méthylphényl)pipéridin-4-yl]pyrrolidin-2-one.

On laisse une nuit sous agitation à TA un mélange de 6,5 g du composé obtenu à l'étape précédente, 3,53 g de formiate d'ammonium et 0,1 g de palladium sur charbon à 10 % dans 150 ml de MeOH. On filtre le catalyseur, concentre le filtrat sous vide, reprend le résidu à l'acétone et essore le précipité formé. On obtient 3,5 g du produit attendu.

Préparation 8.12

3-(4-Phénylpipéridin-4-yl)-1,3-oxazolidin-2-one.

(III) : $R_2$ = 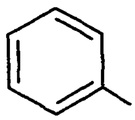 ; $R_3$ = 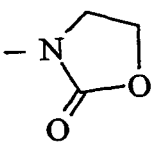

A) Acide 1-[(benzyloxy)carbonyl]-4-phénylpipéridine-4-carboxylique.

On refroidit à 5°C un mélange de 151 g de p-toluènesulfonate de l'acide 4-phénylpipéridine-4-carboxylique et 213 g d'une solution aqueuse de NaOH à 30 % dans 500 ml d'eau. On ajoute, goutte à goutte, une solution de 68,2 g de chloroformiate de benzyle dans 150 ml d'acétone et laisse 6 jours sous agitation en laissant remonter la température à TA. On rajoute 150 ml d'acétone et verse le mélange réactionnel sur un mélange de 200 g d'une solution d'HCl concentrée, 300 ml d'eau et 300 ml d'acétone préalablement refroidi au bain de glace. On essore le précipité formé, le lave à l'eau puis à l'éther. On obtient 89,7 g du produit attendu. B) 4-(Chlorocarbonyl)-4-phénylpipéridine-1-carboxylate de benzyle.

On chauffe à reflux, jusqu'à la fin du dégagement gazeux un mélange de 50,89 g du composé obtenu à l'étape précédente et 71,4 g de chlorure de thionyle dans 400 ml de 1,2-dichloroéthane. On concentre le mélange réactionnel sous vide, reprend le résidu à l'acétone et évapore le solvant sous vide. On obtient 55,3 g du produit attendu sous forme d'huile verte. C) 4-Isocyanato-4-phénylpipéridine-1-carboxylate de benzyle.

On refroidit à 5°C une solution de 55,3 g du composé obtenu à l'étape précédente dans 200 ml d'acétone, ajoute, goutte à goutte, une solution de 19,5 g d'azidure de sodium dans 60 ml d'eau et laisse 2 heures sous agitation en laissant remonter la température à TA. On concentre sous vide, reprend le résidu par une solution à 5 % de $NaHCO_3$, extrait au toluène, lave la phase organique à l'eau, par une solution saturée de NaCl, sèche sur $Na_2SO_4$ et concentre deux tiers du solvant sous vide. On chauffe à reflux pendant 1 heure le reste de la solution toluénique puis concentre sous vide. On obtient 54 g du produit attendu sous forme d'huile orange qui cristallise. D) 4-[[(2-Chloroéthoxy)carbonyl] amino]-4-phénylpipéridine-1-carboxylate de benzyle.

On chauffe à reflux pendant 5 heures un mélange de 3,36 g du composé obtenu à l'étape précédente et 8,04 g de 2-chloroéthanol dans 50 ml de 1,2-dichloroéthane et laisse une nuit sous agitation à TA. On concentre sous vide, reprend le résidu au pentane, triture, essore le précipité formé et le lave au pentane. On obtient 3,7 g du produit attendu. E) 4-(2-Oxo-1,3-oxazolidin-3-yl)-4-phénylpipéridine-1-carboxylate de benzyle.

On laisse une nuit sous agitation à TA un mélange de 3,7 g du composé obtenu à l'étape précédente et 0,7 g d'hydrure de sodium à 60 % dans l'huile dans 50 ml de DMF. On concentre sous vide, reprend le résidu par une solution tampon pH = 2, extrait à l'AcOEt, lave la phase organique à l'eau, sèche sur $Na_2SO_4$ et évapore le solvant sous vide. On reprend le résidu dans un mélange éther iso/pentane, triture, essore le précipité formé et le lave à l'éther iso. On obtient 2,7 g du produit attendu. F) 3-(4-Phénylpipéridin-4-yl)-1,3-oxazolidin-2-one.

On chauffe à 50°C pendant 1 heure un mélange de 2,7 g du composé obtenu à l'étape précédente, 1,34 g de formiate d'ammonium et 0,45 g de palladium sur charbon à 10 % dans 50 ml d'EtOH. On filtre le catalyseur et concentre le filtrat sous vide. On obtient 1,7 g du produit attendu.

Préparation 8.13

3-[4-(3-Fluorophényl)pipéridin-4-yl]-1,3-oxazolidin-2-one.

(III) : $R_2$ = 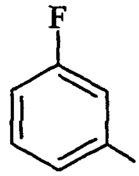 ; $R_3$ = 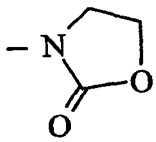

A) [1-Benzyl-4-(3-fluorophényl)pipéridin-4-yl]carbamate de 2-chloroéthyle.

A une solution de 9 g du composé obtenu à l'étape A de la Préparation 8.7 et 3,84 g de triéthylamine dans 100 ml de 1,2-dichloroéthane on ajoute, à TA, 4,52 g de chloroformiate de 2-chloroéthyle et laisse 1 heure sous agitation à TA. On concentre sous vide, extrait le résidu à l'AcOEt, lave la phase organique à l'eau, sèche sur $Na_2SO_4$ et évapore le solvant sous vide. On chromatographie le résidu sur gel de silice H en éluant par le mélange DCM/MeOH (100/1 ; v/v). On obtient 3,2 g du produit attendu.

B) 3-[1-Benzyl-4-(3-fluorophényl)pipéridin-4-yl]-1,3-oxazolidin-2-one.

A une solution de 3,2 g du composé obtenu à l'étape précédente dans 30 ml de DMF, on ajoute, à TA, 0,65 g d'hydrure de sodium à 60 % dans l'huile, laisse 1 heure sous agitation à TA puis chauffe pendant 1 heure à 30°C. On concentre sous vide, reprend le résidu à l'eau, essore le précipité formé et le lave à l'eau. On obtient 2,4 g du produit attendu après séchage sous vide.

C) 3-[4-(3-Fluorophényl)pipéridin-4-yl]-1,3-oxazolidin-2-one.

On chauffe à 50-60°C pendant 3 heures un mélange de 2,4 g du composé obtenu à l'étape précédente, 1,28 g de formiate d'ammonium et 0,43 g de palladium sur charbon à 10 % dans 150 ml d'EtOH. Après refroidissement à TA, on filtre le catalyseur et concentre le filtrat sous vide. On obtient 1,2 g du produit attendu.

Préparation 8.14

3-[4-(4-Fluorophényl)pipéridin-4-yl]-1,3-oxazolidin-2-one.

(III) : $R_2$ = 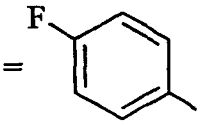 ; $R_3$ = 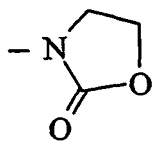

A) Bis-(2-chloroéthyl)carbamate de *tert*-butyle.

A un mélange de 75 g de chlorhydrate de bis-(2-chloroéthyl)amine et 91,7 g de di-*tert*-butyldicarbonate dans 1000 ml de DCM, on ajoute, à TA et goutte à goutte, 61,2 ml de triéthylamine et laisse une nuit sous agitation à TA. On concentre le mélange réactionnel sous vide, extrait le résidu à l'éther, lave la phase organique à l'eau, par une solution tampon pH = 2, par une solution à 5 % de $NaHCO_3$, par une solution saturée de NaCl, sèche sur $Na_2SO_4$ et évapore le solvant sous vide. On obtient 101 g du produit attendu sous forme d'huile que l'on utilise tel quel.

B) 4-Cyano-4-(4-fluorophényl)pipéridine-1-carboxylate de *tert*-butyle.

A un mélange de 33,3 g d'hydrure de sodium à 60 % dans l'huile dans 200 ml de DMSO et 100 ml de THF, on ajoute, goutte à goutte et à TA, une solution de 50 ml de 4-fluorophénylacétonitrile et 101 g du composé obtenu à l'étape précédente dans 200 ml de THF, laisse 2 heures sous agitation à TA, puis chauffe à reflux pendant 1 heure et laisse une nuit sous agitation à TA. On verse le mélange réactionnel dans une solution saturée de $NH_4Cl$, on concentre le

THF sous vide, dilue la phase aqueuse par ajout d'eau, extrait à l'éther, sèche la phase organique sur $Na_2SO_4$ et évapore le solvant sous vide. On reprend l'huile orange obtenue dans un minimum d'éther et essore le produit cristallisé formé. On obtient 87,6 g du produit attendu.

C) Chlorhydrate de 4-(4-fluorophényl)pipéridine-4-carbonitrile.

On chauffe à reflux pendant 4 heures un mélange de 87,6 g du composé obtenu à l'étape précédente et 33 ml d'une solution d'HCl concentrée dans 330 ml de MeOH. On concentre le mélange réactionnel sous vide, reprend le résidu à l'acétone et essore le produit cristallisé formé. On obtient 54,71 g du produit attendu.

D) Acide 4-(4-fluorophényl)pipéridine-4-carboxylique.

On chauffe à reflux pendant 7 heures un mélange de 54,71 g du composé obtenu à l'étape précédente et 57 g de potasse dans 360 ml de diéthylèneglycol puis laisse une nuit sous agitation à TA. On amène le volume du mélange réactionnel jusqu'à 900 ml par ajout de glace, neutralise à pH = 7 par ajout d'une solution d'HCl concentrée et laisse sous agitation pendant 2 heures. On essore le précipité formé et le lave à l'acétone. On obtient 31,56 g du produit attendu.

E) Acide 1-[(benzyloxy)carbonyl]-4-(4-fluorophényl)pipéridine-4-carboxylique.

A un mélange de 31,56 g du composé obtenu à l'étape précédente et 56,4 g d'une solution de NaOH à 30 % dans 320 ml d'eau, on ajoute 50 ml d'acétone, refroidit au bain de glace, ajoute goutte à goutte 20,12 ml de chloroformiate de benzyle et laisse une nuit sous agitation à TA. On lave le mélange réactionnel à l'éther, acidifie la phase aqueuse à pH = 1 par ajout d'une solution d'HCl concentrée, extrait à l'AcOEt, lave la phase organique à l'eau, par une solution saturée de NaCl, sèche sur $Na_2SO_4$ et évapore le solvant sous vide. On chromatographie le résidu sur gel de silice H en éluant au DCM puis par le mélange DCM/MeOH (100/2 ; v:v). On obtient 30,39 g du produit attendu.

F) 4-(Cblorocarbonyl)-4-(4-fluorophényl)pipéridine-1-carboxylate de benzyle.

A une solution de 30,39 g du composé obtenu à l'étape précédente dans 200 ml de 1,2-dichloroéthane, on ajoute 25 ml de chlorure de thionyle puis chauffe à reflux pendant 4 heures. On concentre sous vide, reprend le résidu au DCM et évapore le solvant sous vide. On obtient 31,8 g du produit attendu.

G) 4-(4-Fluorophényl)-4-isocyanatopipéridine-1-carboxylate de benzyle.

On refroidit au bain de glace une solution de 31,8 g du composé obtenu à l'étape précédente dans 200 ml d'acétone, ajoute goutte à goutte une solution de 11,1 g d'azidure de sodium dans 15 ml d'eau et laisse 1 heure sous agitation à TA. On concentre sous vide, extrait le résidu au toluène, lave la phase organique par une solution à 5 % de $NaHCO_3$, par une solution saturée de NaCl et sèche sur $Na_2SO_4$. On chauffe à reflux pendant 1 heure la solution toluénique résultante puis concentre sous vide. On reprend le résidu au pentane et essore le précipité formé. On obtient 26 g du produit attendu.

H) 4-[[(2-chloroéthoxy)carbonyl]amino]-4-(4-fluorophényl)pipéridine-1-carboxylate de benzyle.

On chauffe à reflux pendant 4 heures un mélange de 1,77 g du composé obtenu à l'étape précédente et 0,4 g de 2-chloroéthanol dans 50 ml de 1,2-dichloroéthane puis laisse une nuit sous agitation à TA et concentre sous vide. On obtient 2,1 g du produit attendu.

I) 4-(4-Fluorophényl)-4-(2-oxo-1,3-oxazolidin-3-yl)pipéridine-1-carboxylate de benzyle.

On laisse 2 heures sous agitation à TA un mélange de 2,1 g du composé obtenu à l'étape précédente et 0,386 g d'hydrure de sodium à 60 % dans l'huile dans 20 ml de DMF. On concentre sous vide, reprend le résidu par une solution tampon pH = 2, extrait à l'AcOEt, lave la phase organique à l'eau, sèche sur $Na_2SO_4$ et évapore le solvant sous vide. On reprend le résidu au pentane, triture et essore le précipité formé. On obtient 1,5 g du produit attendu.

J) 3-[4-(4-Fluorophényl)pipéridin-4-yl]-1,3-oxazolidin-2-one.

On laisse 1 heure sous agitation à 40°C un mélange de 1,5 g du composé obtenu à l'étape précédente, 0,7 g de formiate d'ammonium et 0,2 g de palladium sur charbon à 10 % dans 20 ml d'EtOH. On filtre le catalyseur et concentre

le filtrat sous vide. On obtient 0,9 g du produit attendu.

Préparation 8.15

3-[4-(3,4-diméthylphényl)pipéridin-4-yl]-1,3-oxazolidin-2-one.

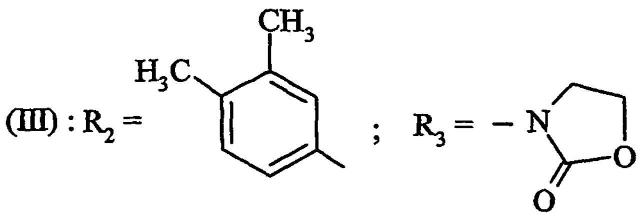

A) Benzyl[bis(2-chloroéthyl)]amine.

On refroidit au bain de glace un mélange de 150 g de chlorhydrate de bis(2-chloroéthyl)amine et 144 g de bromure de benzyle dans 1000 ml de DMF, ajoute, goutte à goutte, 120 ml de triéthylamine puis laisse 5 heures sous agitation à TA. On concentre sous vide, reprend le résidu à l'eau, extrait à l'éther, sèche la phase organique sur $Na_2SO_4$ et évapore le solvant sous vide. On obtient 110 g du produit attendu.

B) 1-Benzyl-4-(3,4-diméthylphényl)pipéridine-4-carbonitrile.

A une suspension de 5,78 g d'hydrure de sodium à 60 % dans l'huile dans 20 ml de THF et 10 ml de DMSO, on ajoute, goutte à goutte, une solution de 10 g de 3,4-diméthylphénylacétonitrile dans 50 ml de DMSO puis laisse 30 minutes sous agitation à TA. On ajoute ensuite, goutte à goutte, une solution de 14 g de composé obtenu à l'étape précédente dans 50 ml de DMSO et laisse une nuit sous agitation à TA. On concentre le mélange réactionnel sous vide, reprend le résidu à l'eau, extrait à l'éther, sèche la phase organique sur $Na_2SO_4$ et évapore le solvant sous vide. On reprend le résidu dans une solution d'éther chlorhydrique 2N, essore le précipité formé et le lave au pentane. On obtient 18 g du produit attendu, F = 265-267°C.

C) Acide 1-benzyl-4-(3,4-diméthylphényl)pipéridine-4-carboxylique.

On chauffe à reflux pendant 24 heures un mélange de 15 g du composé obtenu à l'étape précédente et 11,06 g de KOH en pastilles dans 100 ml d'éthylèneglycol. Après refroidissement à TA, on ajoute de l'eau au mélange réactionnel, lave la phase aqueuse à l'éther, neutralise la phase aqueuse à pH = 7 par ajout d'une solution d'HCl concentrée et essore le précipité formé. On obtient 10,6 g du produit attendu.

D) Chlorure de 1-benzyl-4-(3,4-diméthylphényl)pipéridine-4-carbonyle, chlorhydrate.

On chauffe à reflux pendant 6 heures un mélange de 10,6 g du composé obtenu à l'étape précédente et 8,76 ml de chlorure de thionyle dans 100 ml de 1,2-dichloroéthane puis laisse une nuit sous agitation à TA. On concentre sous vide, reprend le résidu à l'éther, essore le précipité formé, le lave à l'éther et au pentane. On obtient 9 g du produit attendu que l'on utilise tel quel.

E) 1-Benzyl-4-(3,4-diméthylphényl)-4-isocyanatopipéridine.

On refroidit à 0°C une solution de 9 g du composé obtenu à l'étape précédente et 5,16 ml de triéthylamine dans 200 ml d'acétone, ajoute, goutte à goutte, une solution de 3,1 g d'azidure de sodium dans 15 ml d'eau et laisse sous agitation en laissant remonter la température à TA. On concentre sous vide, extrait le résidu au toluène, lave la phase organique à l'eau, sèche sur $Na_2SO_4$, et chauffe à reflux pendant 3 heures la phase toluènique. On concentre sous vide et obtient 4 g du produit attendu que l'on utilise tel quel.

F) [1-Benzyl-4-(3,4-diméthylphényl)pipéridin-4-yl]carbamate de 2-chloroéthyle.

On chauffe à reflux pendant 4 heures un mélange de 4 g du composé obtenu à l'étape précédente et 10,05 g de 2-chloroéthanol dans 50 ml de 1,2-dichloroéthane. On concentre sous vide et chromatographie le résidu sur gel de silice en éluant par le mélange DCM/MeOH (100/5 ; v/v). On obtient 2 g du produit attendu que l'on utilise tel quel.

G) 3-[1-Benzyl-4-(3,4-diméthylphényl)pipéridin-4-yl]-1,3-oxazolidin-2-one.

On laisse 2 heures sous agitation à TA un mélange de 2 g du composé obtenu à l'étape précédente et 0,4 g d'hydrure de sodium à 60 % dans l'huile dans 20 ml de DMF. On concentre sous vide, reprend le résidu à l'eau, extrait à l'AcOEt, lave la phase organique à l'eau, sèche sur $MgSO_4$ et évapore le solvant sous vide. On obtient 1,4 g du produit attendu.

H) 3-[4-(3,4-diméthylphényl)pipéridin-4-yl]-1,3-oxazolidin-2-one.

On laisse 2 heures sous agitation à 30°C un mélange de 1,4 g du composé obtenu à l'étape précédente, 0,73 g de formiate d'ammonium et 0,41 g de palladium sur charbon à 10 % dans 30 ml d'EtOH. On filtre le catalyseur et concentre le filtrat sous vide. On obtient 1 g du produit attendu.

Préparation 8.16

Formiate de 3-(4-phénylpipéridin-4-yl)-1,3-oxazinan-2-one.
(III), HCOOH :

$R_2$ = 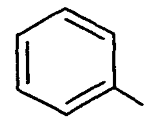 ; $R_3$ = 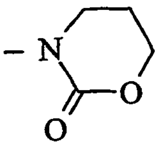

A) 4-[[(3-Chloropropoxy)carbonyl]amino]-4-phénylpipéridine-1-carboxylate de benzyle.

On chauffe à reflux pendant 4 heures un mélange de 4 g du composé obtenu à l'étape C de la Préparation 8.12 et 5 ml de 3-chloro-1-propanol dans 100 ml de 1,2-dichloroéthane. On rajoute 5 ml de 3-chloro-1-propanol puis poursuit le reflux pendant 1 heure. On rajoute 5 ml de 3-chloro-1-propanol et laisse une nuit sous agitation à TA. On concentre sous vide et obtient 4,14 g du produit attendu.

B) 4-(2-Oxo-1,3-oxazinan-3-yl)-4-phénylpipéridine-1-carboxylate de benzyle.

On laisse 2 heures sous agitation à TA un mélange de 4,14 g du composé obtenu à l'étape précédente et 0,769 g d'hydrure de sodium à 60 % dans l'huile dans 50 ml de DMF. On concentre sous vide, reprend le résidu dans l'éther iso, et essore le précipité formé. On obtient 3,19 g du produit attendu après séchage sous vide.

C) Formiate de 3-(4-phénylpipéridin-4-yl)-1,3-oxazinan-2-one.

On laisse 3 heures sous agitation à TA un mélange de 3,19 g du composé obtenu à l'étape précédente, 1,53 g de formiate d'ammonium et 0,43 g de palladium sur charbon à 10 % dans 60 ml d'EtOH. On filtre le catalyseur et concentre le filtrat sous vide. On obtient 1 g du produit attendu.

Préparation 8.17

Formiate de 3-[4-(4-fluorophényl)pipéridin-4-yl]-1,3-oxazinan-2-one.
(III), HCOOH :

$R_2$ = 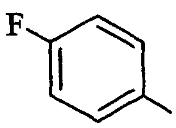 ; $R_3$ = 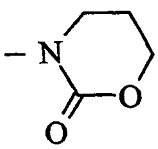

A) 4-[[(3-Chloropropoxy)carbonyl]amino]-4-(4-fluorophényl)pipéridine-1-carboxylate de benzyle.

On chauffe à reflux pendant 1 heure un mélange de 2 g du composé obtenu à l'étape G de la Préparation 8.14

et 8 g de 3-chloro-1-propanol dans 20 ml de 1,2-dichloroéthane. On concentre le mélange réactionnel sous vide, reprend le résidu au pentane, laisse 1 heure sous agitation et essore le précipité formé. On obtient 2,5 g du produit attendu après séchage sous vide.

B) 4-(4-Fluorophényl)-4-(2-oxo-1,3-oxazinan-3-yl)pipéridine-1-carboxylate de benzyle.

On laisse 1 heure sous agitation à TA un mélange de 2,5 g du composé obtenu à l'étape précédente et 0,445 g d'hydrure de sodium à 60 % dans l'huile dans 20 ml de DMF. On concentre le mélange réactionnel sous vide, reprend le résidu par une solution tampon pH = 2, extrait à l'AcOEt, lave la phase organique à l'eau, sèche sur $Na_2SO_4$ et évapore le solvant sous vide. On reprend le résidu dans le mélange éther iso/pentane, triture et essore le précipité formé. On obtient 1,1 g du produit attendu.

C) Formiate de 3-[4-(4-fluorophényl)pipéridin-4-yl]-1,3-oxazinan-2-one.

On laisse 1 heure sous agitation à TA un mélange de 1,1 g du composé obtenu à l'étape précédente, 0,5 g de formiate d'ammonium et 0,14 g de palladium sur charbon à 10 % dans 20 ml d'EtOH. On filtre le catalyseur et concentre sous vide le filtrat. On obtient 0,7 g du produit attendu.

Préparation 8.18

3-[4-(3,4-Difluorophényl)pipéridin-4-yl]-1,3-oxazinan-2-one.

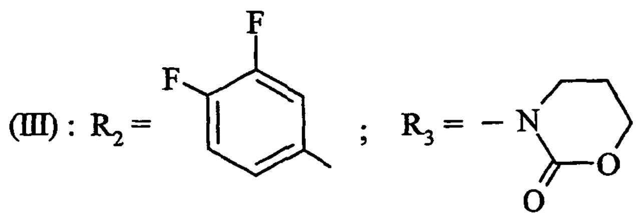

A) 4-(3,4-Difluorophényl)pipéridin-4-amine.

On refroidit au bain de glace un mélange de 33 g du composé obtenu à l'étape A de la Préparation 8.8 et 18,42 g de formiate d'ammonium dans 500 ml de MeOH, ajoute 0,5 g de palladium sur charbon à 10 %, laisse 3 heures sous agitation en laissant remonter la tempérautre à TA puis chauffe à 40°C pendant 1 heure. On filtre le catalyseur et concentre le filtrat sous vide. On obtient 28 g du produit attendu.

B) 4-Amino-4-(3,4-difluorophényl)pipéridine-1-carboxylate de benzyle.

A un mélange de 27,5 g du composé obtenu à l'étape précédente et 57,7 ml de triéthylamine dans 500 ml de DCM, on ajoute, goutte à goutte et à TA, 22,1 g de chloroformiate de benzyle et laisse une nuit sous agitation à TA. On concentre le mélange réactionnel sous vide, extrait le résidu au DCM, lave la phase organique à l'eau, par une solution saturée de NaCl, sèche sur $Na_2SO_4$ et évapore le solvant sous vide. On chromatographie le résidu sur gel de silice en éluant par DCM/MeOH (95/5 ; v/v). On obtient 11,51 g du produit attendu.

C) 4-[[(3-Chloropropoxy)carbonyl]amino]-4-(3,4-difluorophényl)pipéridine-1-carboxylate de benzyle

A un mélange de 5 g du composé obtenu à l'étape précédente et 4,42 ml de triéthylamine dans 100 ml de DCM, on ajoute, goutte à goutte et à TA, 2,27 g de chloroformiate de 3-chloropropyle et laisse une nuit sous agitation à TA. On concentre le mélange réactionnel sous vide, extrait le résidu au DCM, lave la phase organique à l'eau, par une solution saturée de NaCl, sèche sur $Na_2SO_4$ et évapore le solvant sous vide. On chromatographie le résidu sur gel de silice H en éluant au DCM puis par le mélange DCM/MeOH (100/2 ; v/v). On obtient 1,9 g du produit attendu.

D) 4-(3,4-Difluorophényl)-4-(2-oxo-1,3-oxazinan-3-yl)pipéridine-1-carboxylate de benzyle

On laisse 2 heures sous agitation à TA, un mélange de 1,9 g du composé obtenu à l'étape précédente et 0,33 g d'hydrure de sodium à 60 % dans l'huile dans 30 ml de DMF. On concentre le mélange réactionnel sous vide, reprend le résidu à l'éther iso et essore le précipité formé. On obtient 1,5 g du produit attendu après séchage sous vide.

E) 3-[4-(3,4-Difluorophényl)pipéridin-4-yl]-1,3-oxazinan-2-one.

On laisse une nuit sous agitation à TA, un mélange de 1,5 g du composé obtenu à l'étape précédente, 0,66 g de formiate d'ammonium et 0,18 g de palladium sur charbon à 10 % dans 50 ml d'EtOH. On filtre le catalyseur et concentre le filtrat sous vide. On obtient 0,87 g du produit attendu.

Préparation 8.19

4-[(4-Phénylpipéridin-4-yl)carbonyl]morpholine.

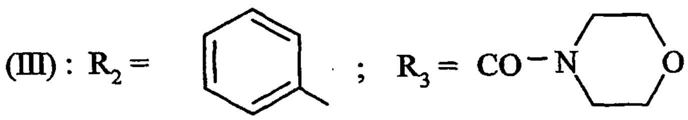

On prépare ce composé selon les modes opératoires décrits à la Préparation 1.29 dans WO 97/10211.

Préparation 8.20

Formiate de 1,4'-bipipéridine-4'-carboxamide.

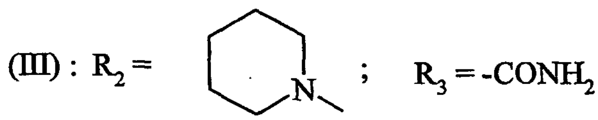

A) 1'-Benzyl-1,4'-bipipéridine-4'-carboxamide.

On prépare ce composé selon le mode opératoire décrit à l'étape B de la Préparation 3.1 dans WO 02/094821.

B) Formiate de 1,4'-bipipéridine-4'-carboxamide.

On laisse 4 heures et 30 minutes un mélange de 50 g du composé obtenu à l'étape précédente, 31,4 g de formiate d'ammonium et 12,5 g de palladium sur charbon à 50 % dans 200 ml de MeOH. On filtre le catalyseur et concentre le filtrat sous vide. On obtient 22,5 g du produit attendu.

Préparation 8.21

3-[4-(3,4-Difluorophényl)pipéridin-4-yl]-1,3-oxazolidin-2-one.

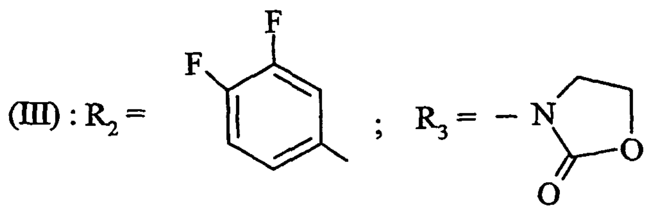

A) 4-[[(2-Chloroéthoxy)carbonyl]amino]-4-(3,4-diffuorophényl)pipéridine-1-carboxylate de benzyle.

A une solution de 5 g du composé obtenu à l'étape B de la Préparation 8.18 et 4,42 ml de triéthylamine dans 100 ml de DCM, on ajoute, goutte à goutte à TA, 2,06 g de chloroformiate de 2-chloroéthyle et laisse une nuit sous agitation à TA. On concentre le mélange réactionnel sous vide, extrait le résidu au DCM, lave la phase organique à l'eau, par une solution saturée de NaCl, sèche sur $Na_2SO_4$ et évapore le solvant sous vide. On chromatographie le résidu sur gel de silice H en éluant au DCM puis par le mélange DCM/MeOH (97/3 ; v/v) On obtient 1,55 g du produit attendu.

B) 4-(3,4-Difluorophényl)-4-(2-oxo-1,3-oxazolidin-3-yl)pipéridine-1-carboxylate de benzyle.

A un mélange de 1,5 g du composé obtenu à l'étape précédente dans 50 ml de DMF, on ajoute 0,16 g d'hydrure de sodium à 60 % dans l'huile et laisse 2 heures sous agitation à TA. On concentre le mélange réactionnel sous vide, reprend le résidu à l'éther iso, essore le précipité formé et le sèche sous vide. On obtient 1,16 g du produit attendu.

C) 3-[4-(3,4-Difluorophényl)pipéndin-4-yl]-1,3-oxazolidin-2-one.

On laisse une nuit sous agitation à TA un mélange de 1,15 g du composé obtenu à l'étape précédente, 0,52 g de formiate d'ammonium et 0,15 g de palladium sur charbon à 10 % dans 60 ml d'EtOH. On filtre le catalyseur et concentre le filtrat sous vide. On obtient 0,73 g du produit attendu.

Préparation 8.22

Formiate de N-[4-[3-(triffuorométhyl)phényl]pipéridin-4-yl]acétamide.

(III) : $R_2$ = 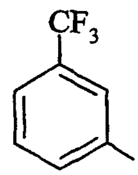 ; $R_3$ = -NHCOCH$_3$

A) 1-Benzyl-4-[3-(trifluorométhyl)phényl]pipéridin-4-ol.

A un mélange de 24,5 g de 4-hydroxy-4-[3-(trifluorométhyl)phényl]pipéridine (commercial) et 27,6 g de $K_2CO_3$ dans 20 ml de DMF, on ajoute, goutte à goutte et à TA, 17,1 g de bromure de benzyle puis chauffe à 40°C pendant 2 heures et laisse une nuit sous agitation à TA. On concentre le mélange réactionnel sous vide, reprend le résidu à l'eau, extrait à l'AcOEt, lave la phase organique à l'eau, sèche sur $Na_4SO_4$ et évapore le solvant sous vide. On obtient 31 g du produit attendu.

B) N-[1-Benzyl-4-[3-(bùiuorométhyl)phényl]pipéridin-4-yl]acétamide.

A une solution de 31 g du composé obtenu à l'étape précédente dans 107 ml d'acétonitrile, on ajoute, goutte à goutte et à une température inférieure à 20°C, 88,65 ml d'$H_2SO_4$ à 95 % et laisse 6 heures sous agitation à TA. On verse le mélange réactionnel dans de la glace, neutralise par ajout d'une solution concentrée de NaOH, essore le précipité formé, le lave à l'eau et le sèche. On obtient 17,7 g du produit attendu après cristallisation dans le propan-2-ol.

C) Formiate de N-[4-[3-(trifluorométhyl)phényl]pipéridin-4-yl]acétamide.

On laisse une nuit sous agitation à TA un mélange de 3 g du composé obtenu à l'étape précédente, 1,5 g de formiate d'ammonium et 0,4 g de palladium sur charbon à 10 % dans 60 ml de MeOH. On filtre le catalyseur et concentre le filtrat sous vide. On obtient 3 g du produit attendu.

Préparation 8.23

Formiate de 4-(pyridin-2-yl)pipéridin-4-ol.

(III) : $R_2$ = 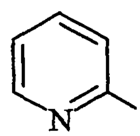 ;

$R_3$ = -OH

A) 1-Benzyl-4-(pyridin-2-yl)pipéridin-4-ol.

On refroidit à -70°C une solution de 22,2 g de 2-bromopyridine dans 100 ml de THF, ajoute, goutte à goutte, 87,8 ml de n-butyllithium et laisse 30 minutes sous agitation. On ajoute ensuite, goutte à goutte et à -60°C, une solution de 25,6 g de 1-benzyl-4-pipéridinone dans 100 ml de THF et laisse une nuit sous agitation en laissant remonter la température à TA. On verse le mélange réactionnel dans l'eau, extrait à l'éther, lave la phase organique à l'eau, sèche sur $Na_2SO_4$ et évapore le solvant sous vide. On chromatographie le résidu sur gel de silice H en éluant au DCM puis par le gradient du mélange DCM/MeOH de (99/1 ; v/v) à (90/10 ; v/v). On obtient 21,5 g du produit attendu.

B) Formiate de 4-(pyridin-2-yl)pipéridin-4-ol.

On laisse 4 heures sous agitation à TA un mélange de 5 g du composé obtenu à l'étape précédente, 3,5 g de formiate d'ammonium et 0,1 g de palladium sur charbon à 10 % dans 50 ml de MeOH. On filtre le catalyseur et concentre le filtrat sous vide. On obtient 3,4 g du produit attendu.

Préparation 8.24

Chlorhydrate de 3-[4-(4-chlorophényl)pipéridin-4-yl]-1,3-oxazolidin-2-one.

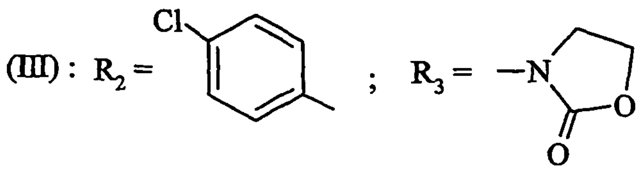

A) 1-Benzyl-4-(4-chlorophényl)pipéridine-4-carbonitrile.

A une suspension de 9,8g d'hydrure de sodium à 60 % dans l'huile dans 140 ml de THF et 60 ml de DMSO, on ajoute, goutte à goutte et à TA, une solution de 11,3 g de 4-chlorophénylacétonitrile et 20 g de chlorhydrate de benzyl [bis(2-chloroéthyl)]amine dans 140 ml de THF et 60 ml de DMSO puis chauffe à 70-80°C pendant 2 heures. Après refroidissement, on verse le mélange réactionnel sur de la glace, extrait à l'éther, lave la phase organique à l'eau, sèche sur $Na_2SO_4$ et évapore le solvant sous vide. On chromatographie le résidu sur gel de silice en éluant par le mélange heptane/AcOEt (90/10 ; v/v). On obtient 19,6 g du composé attendu.

B) Acide 1-benzyl-4-(4-chlorophényl)pipéridine-4-carboxylique.

On chauffe à 200°C pendant 18 heures un mélange de 19,6 g du composé obtenu à l'étape précédente et 14,15 g de KOH en pastilles dans 150 ml d'éthylèneglycol. Après refroidissement à TA, on verse le mélange réactionnel sur de la glace, lave la phase aqueuse à l'éther, acidifie la phase aqueuse à pH = 6,5 par ajout d'une solution d'HCl concentrée, essore le précipité formé, le lave à l'eau puis à l'acétone et le sèche. On obtient 19 g du composé attendu.

C) Chlorure de 1-benzyl-4-(4-chlorophényl)pipéridine-4-carbonyle, chlorhydrate.

On chauffe à reflux pendant 4 heures un mélange de 19 g du composé obtenu à l'étape précédente et 40,5 ml de chlorure de thionyle, puis laisse une nuit sous agitation à TA. On concentre le mélange réactionnel sous vide, reprend le résidu au toluène et évapore le solvant sous vide. On obtient 21 g du composé attendu que l'on utilise tel quel.

D) 1-Benzyl-4-(4-chlorophényl)-4-isocyanatopipéridine.

On refroidit à 0°C une suspension de 20,5 g du composé obtenu à l'étape précédente et 13 ml de triéthylamine dans 200 ml d'acétone, ajoute, goutte à goutte, une solution de 9,6 g d'azidure de sodium dans 50 ml d'eau, laisse 30 minutes sous agitation puis 1 heure à TA. On concentre sous vide, extrait le résidu au toluène, lave la phase organique à l'eau, sèche sur $Na_2SO_4$ et chauffe à reflux pendant 4 heures la phase toluénique. Après une nuit sous agitation à TA, on évapore le solvant sous vide et obtient 10,5 g du composé attendu que l'on utilise tel quel.

E) [1-Benzyl-4-(4-chlorophényl)pipéridin-4-yl]carbamate de 2-chloroéthyl.

On chauffe à 80°C pendant 1 heure un mélange de 10,5 g du composé obtenu à l'étape précédente et 20 ml de 2-chloroéthanol puis laisse une nuit sous agitation à TA. On concentre sous vide, extrait le résidu à l'AcOEt, lave la phase organique à l'eau, sèche sur $Na_2SO_4$ et évapore le solvant sous vide. On chromatographie le résidu sur gel de silice en éluant au DCM puis par le gradient du mélange DCM/MeOH de (99/1 ; v/v) à (90/10 ; v/v). On obtient 8,3 g du composé attendu.

F) 3-[1-Benzyl-4-(4-chlorophényl)pipéridin-4-yl]-1,3-oxazolidin-2-one.

A une solution de 8 g du composé obtenu à l'étape précédente dans 45 ml de DMF, on ajoute, par portions et à TA, 1,73 g d'hydrure de sodium à 60 % dans l'huile puis chauffe à 50°C pendant 1 heure. On concentre sous vide, reprend le résidu à l'eau, extrait à l'AcOEt, lave la phase organique à l'eau, sèche sur $Na_2SO_4$ et évapore le solvant sous vide. On reprend le résidu au pentane, triture, essore le précipité formé et le lave au pentane. On obtient 6,2 g du composé attendu. G) Chlorhydrate de 3-[4-(4-chlorophényl)pipéridin-4-yl]-1,3-oxazolidin-2-one.

On refroidit à 0°C un mélange de 6,2 g du composé obtenu à l'étape précédente et 2,3 g de $K_2CO_3$ dans 60 ml de DCM, ajoute, goutte à goutte, 2,87 g de chloroformiate de 1-chloroéthyle, laisse 1 heure sous agitation à froid puis 1 heure à TA. On filtre l'insoluble et concentre sous vide le filtrat. On reprend le résidu par 50 ml de MeOH et laisse une nuit sous agitation à TA. On concentre sous vide, dissout le résidu dans un mélange DCM/MeOH, ajoute sur un mélange éther/pentane, essore le précipité formé et le lave à l'éther. On obtient 4,2 g du composé attendu.

Préparation 8.25

N,N-Diméthyl-1,4'-bipipéridine-4'-carboxamide.

(III) : $R_2$ = 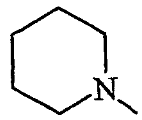 ;

$R_3$ = -CON($CH_3$)$_2$

On prépare ce composé selon les modes opératoires décrits à la Préparation 3.1 dans WO 02/094821.

EXEMPLE 1 : Composé n° 1

Chlorhydrate de 6-(3,4-dichlorophényl)-6-[2-[4-hydroxy-4-[3-(trifluorométhyl) phényl]pipéridin-1-yl]éthyl]-4-phénylmorpholin-3-one, isomère dextrogyre.

(I), HCl :

Ar = 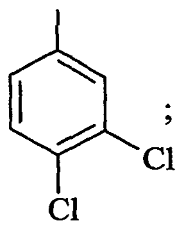 ; $R_1$ = 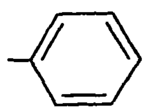 ; $R_2$ = 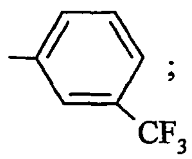 ;

$R_3$ = -OH

A une solution de 0,5 g du composé obtenu à la Préparation 7.1 dans 20 ml de DCM, on ajoute 0,337 g de 4-hydroxy-4-[3-trifluorométhyl)phényl]pipéridine (commercial) puis 0,61 g de triacétoxyborohydrure de sodium et 0,0085 g d'acide acétique et laisse une nuit sous agitation à TA. On alcalinise le mélange réactionnel par ajout d'une solution à 10 % de $Na_2CO_3$, extrait au DCM, lave la phase organique à l'eau, sèche sur $Na_2SO_4$ et évapore le solvant sous vide. On chromatographie le résidu sur gel de silice H en éluant au DCM puis par le gradient du mélange DCM/EtOH jusqu'à (98/2 ;v/v). On reprend le produit obtenu dans l'éther chlorhydrique 2N, concentre sous vide, dissout le résidu dans le DCM, ajoute de l'éther et essore le précipité formé. On obtient 0,42 g du produit attendu. $\alpha_D^{20} = +30°$ (c = 0,25 ;

MeOH).

EXEMPLE 2 : Composé n° 3

Chlorhydrate de N-[1-[2-[2-(3,4-dichlorophényl)-5-oxo-4-phénylmorpholin-2-yl]éthyl]-4-(3-fluorophényl)pipéridin-4-yl]acétamide, isomère dextrogyre.
(I), HCl:

Ar = 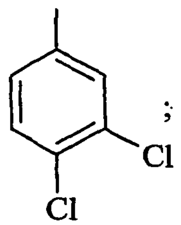 ; $R_1$ = 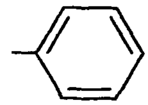 ; $R_2$ = 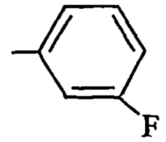 ;

$R_3$= -NHCOCH$_3$

A une solution de 0,5 g du composé obtenu à la Préparation 7.1 dans 20 ml de DCM, on ajoute 0,387 g du composé obtenu à la Préparation 8.2 puis 0,61 g de triacétoxyborohydrure de sodium et 0,0085 g d'acide acétique et laisse une nuit sous agitation à TA. On alcalinise le mélange réactionnel par ajout d'une solution à 10 % de $Na_2CO_3$, extrait au DCM, lave la phase organique à l'eau, sèche sur $Na_2SO_4$ et évapore le solvant sous vide. On chromatographie le résidu sur gel de silice H en éluant au DCM puis par le gradient du mélange DCM/MeOH jusqu'à (97/3 ; v/v). On reprend le produit obtenu dans l'éther chlorhydrique 2N, concentre sous vide, dissout le résidu dans le DCM, ajoute de l'éther et essore le précipité formé. On obtient 0,57 g du produit attendu. $\alpha_D^{20} = +29{,}8°$ (c = 0,5 ; MeOH).

RMN$^1$H : DMSO-d$_6$ : δ (ppm) : 1,9 : s : 3H ; 2,3 : mt : 2H ; 2,4-3,7 : m : 10H ; 4,0-4,75 : m : 4H ; 7,0-7,9 : m : 12H ; 8,2 : s : 1H; 11 : se : 1H.

EXEMPLE 3 : Composé n° 4

Fumarate de N-[1-[2-[2-(3,4-dichlorophényl)-5-oxo-4-phénylmorpholin-2-yl]éthyl]-4-(3-fluorophényl)pipéridin-4-yl]acétamide, isomère dextrogyre.
(I), $C_4H_4O_4$:

Ar = 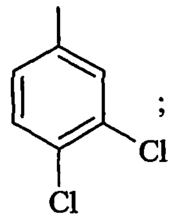 ; $R_1$ = 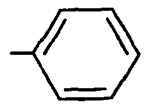 ; $R_2$ = 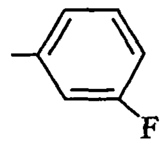 ;

$R_3$ = -NHCOCH$_3$

On chauffe à reflux une solution de 4,5 g du composé n° 3 sous forme de base libre dans 72 ml d'acétonitrile, ajoute 0,896 g d'acide fumarique et poursuit le reflux pendant 30 minutes. On refroidit le mélange réactionnel à TA et laisse 1 heure sous agitation à TA. On essore le produit cristallisé formé et le sèche sous vide. On obtient 2,3 g du produit attendu sous forme de fumarate, F = 244-245°C. $\alpha_D^{20} = +24{,}4°$ (c = 0,25 ; MeOH).

EXEMPLE 4 : Composé n° 14

Chlorhydrate de 6-(3,4-dichlorophényl)-6-[2-[4-(3-fluorophényl)-4-(2-oxo-1,3-oxazolidin-3-yl)pipéridin-1-yl]éthyl]-4-phénylmorpholin-3-one, isomère dextrogyre. (I), HCl :

Ar = 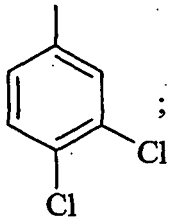 ; $R_1$ = 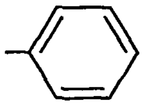 ; $R_2$ = 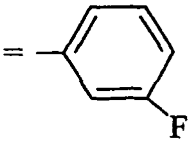 ; $R_3$ = 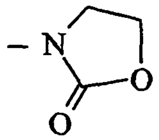

On laisse une nuit sous agitation à TA un mélange de 0,5 g du composé obtenu à la Préparation 7.1, 0,36 g du composé obtenu à la Préparation 8.13, 0,58 g de triacétoxyborohydrure de sodium et 0,0085 g d'acide acétique dans 50 ml de DCM. On lave le mélange réactionnel par une solution à 10 % de $Na_2CO_3$, lave la phase organique à l'eau, sèche sur $Na_2SO_4$ et évapore le solvant sous vide. On chromatographie le résidu sur gel de silice H en éluant au DCM puis par le mélange DCM/MeOH (99/1 ; v/v). On dissout le produit obtenu dans du DCM, ajoute de l'éther chlorhydrique 2N, essore le précipité formé et le lave à l'éther. On obtient 0,4 g du produit attendu. $\alpha_D^{20} = +32,4°$ (c = 0,25 ; MeOH).

RMN[1]H : DMSO-$d_6$ : $\delta$ (ppm) : 2,3 : mt : 2H ; 2,4-3,7 : m : 12H ; 4,0-4,75 : m : 6H ; 7,0-7,9 : m : 12H ; 11 : se : 1H.

EXEMPLE 5 : Composé n° 15

Chlorhydrate de 6-(3,4-dichlorophényl)-6-[2-[4-(4-fluorophényl)-4-52-oxo-1,3-oxazolidin-3-yl)pipéridin-1-yl] éthyl]-4-phénylmorpholin-3-one, isomère dextrogyre.
(I), HCl :

Ar = 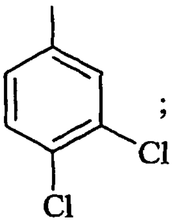 ; $R_1$ = 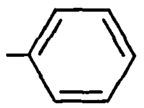 ; $R_2$ = 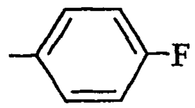 ; $R_3$ = 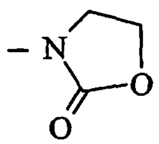

On laisse une nuit sous agitation à TA un mélange de 0,5 g du composé obtenu à la Préparation 7.1, 0,36 g du composé obtenu à la Préparation 8.14, 0,61 g de triacétoxyborohydrure de sodium et 0,0085 g d'acide acétique dans 50 ml de DCM. On lave le mélange réactionnel par une solution à 10 % de $Na_2CO_3$, à l'eau, sèche sur $Na_2SO_4$ et évapore le solvant sous vide. On chromatographie le résidu sur gel de silice H en éluant au DCM puis par le mélange DCM/MeOH jusqu'à (100/3 ; v/v). On dissout le produit obtenu dans du DCM, ajoute de l'éther chlorhydrique 2N, essore le précipité formé et le lave à l'éther. On obtient 0,56 g du produit attendu. $\alpha_D^{20} = +31°$ (c = 0,5 ; MeOH).

RMN[1]H : DMSO-$d_6$ : $\delta$ (ppm) : 2,3 : mt : 2H ; 2,4-3,7 : m : 12H ; 4,0-4,75 : m : 6H ; 7,0-7,9 : m : 12H ; 11 : se : 1H.

Le TABLEAU I qui suit illustre les structures chimiques et les propriétés physiques de quelques exemples de composés selon l'invention. Dans ce tableau :
- dans la colonne "Sel", "-" représente un composé sous forme de base libre, alors que "HCl" représente un composé sous forme de chlorhydrate et "$C_4H_4O_4$" représente un composé sous forme de fumarate :

TABLEAU I

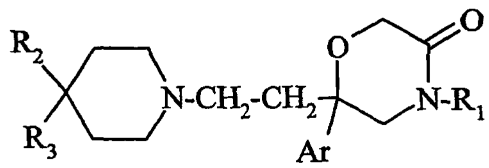 (I)

| Composés n° | Ar | $R_1$ | $R_2$ | $R_3$ | Sel | RMN $\alpha_D^{20}$ = (c ; MeOH) MH+, tr |
|---|---|---|---|---|---|---|
| 1 | 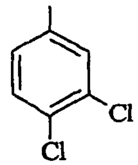 | 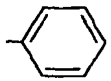 | 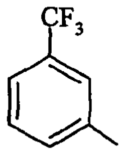 | -OH | HCl | + 30° (c = 0,2) |
| 2 | 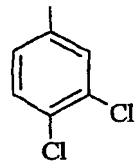 | 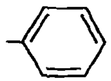 | 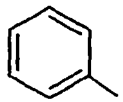 | $-NHCOCH_3$ | HCl | + 31,8° (c = 0,5) |
| 3 | 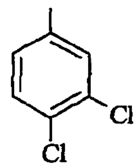 | 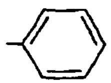 | 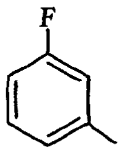 | $-NHCOCH_3$ | HCl | + 29,8° (c = 0,5) |
| 4 | 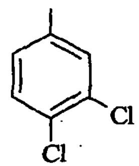 | 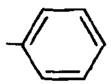 | 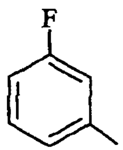 | $-NHCOCH_3$ | $C_4H_4O_4$ | + 24,4° (c = 0,2) |

(suite)

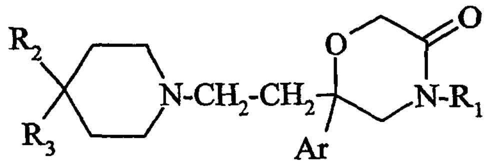 (I)

| Composés n° | Ar | $R_1$ | $R_2$ | $R_3$ | Sel | RMN<br>$\alpha_D^{20} = (c ; MeOH)$<br>$MH^+$, tr |
|---|---|---|---|---|---|---|
| 5 | 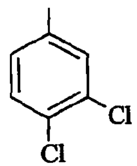 | 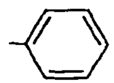 | 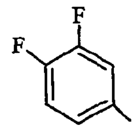 | $-NHCOCH_3$ | HCl | + 30,4° (c = 0,5) |
| 6 | 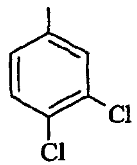 | 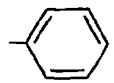 | 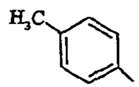 | $-NHCOCH_3$ | HCl | RMN<br>$MH^+$ = 580, tr = 6,75 |
| 7 | 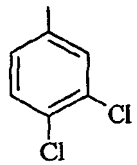 | 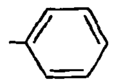 | 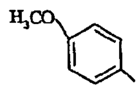 | $-NHCOCH_3$ | HCl | + 11,9° (c = 0,5) |
| 8 | 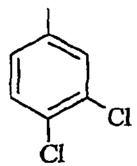 | 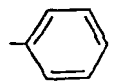 | 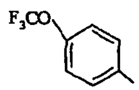 | $-NHCOCH_3$ | HCl | RMN<br>$MH^+$ = 650, tr = 7,17 |

(suite)

| Composés n° | Ar | $R_1$ | $R_2$ | $R_3$ | Sel | RMN<br>$\alpha_D^{20} = (c\,;\,MeOH)$<br>$MH^+$, tr |
|---|---|---|---|---|---|---|
| 9 | 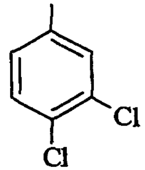 | 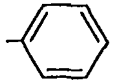 | 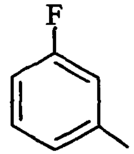 | 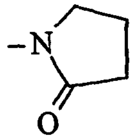 | HCl | + 31,4° (c = 0,5) |
| 10 | 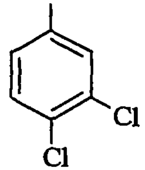 | 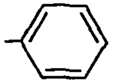 | 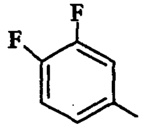 | 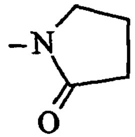 | HCl | + 26,8° (c = 0,5) |
| 11 | 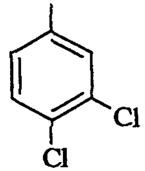 | 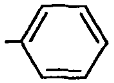 | 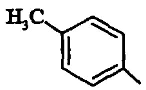 | 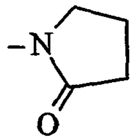 | HCl | RMN<br>$MH^+$ = 606, tr=7,1 |
| 12 | 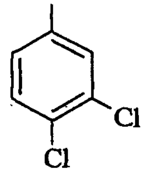 | 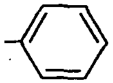 | 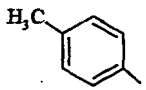 | 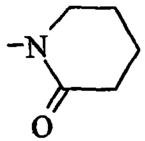 | HCl | + 33,2° (c = 0,5) |
| 13 | 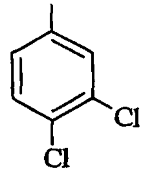 | 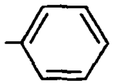 | 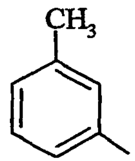 | 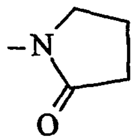 | HCl | + 35,2° (c = 1) |

(suite)

| Composés n° | Ar | $R_1$ | $R_2$ | $R_3$ | Sel | RMN<br>$\alpha_D^{20}$ = (c ; MeOH)<br>$MH^+$, tr |
|---|---|---|---|---|---|---|
| 14 | 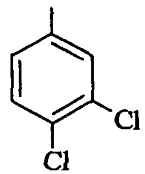 | 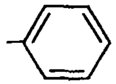 | 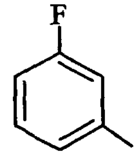 | 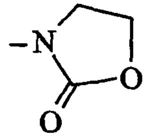 | HCl | + 32,4° (c = 0,5) |
| 15 | 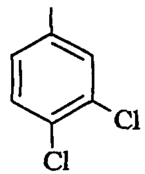 | 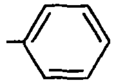 | 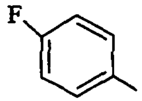 | 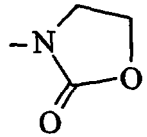 | HCl | + 31° (c = 0,5) |
| 16 | 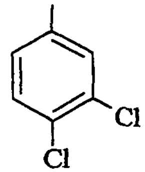 | 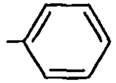 | 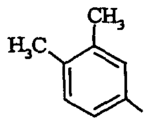 | 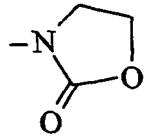 | HCl | + 28° (c = 0,5) |
| 17 | 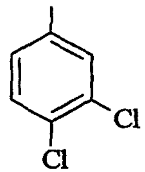 | 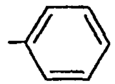 | 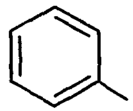 | 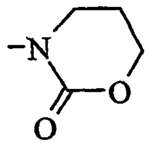 | HCl | + 33,2° (c = 0,5) |
| 18 | 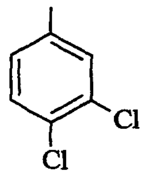 | 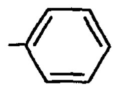 | 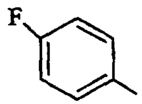 | 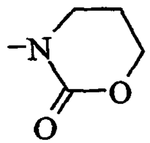 | HCl | + 37° (c = 0,5) |

(suite)

| Composés n° | Ar | $R_1$ | $R_2$ | $R_3$ | Sel | RMN<br>$\alpha_D^{20}$ = (c ; MeOH)<br>$MH^+$, tr |
|---|---|---|---|---|---|---|
| 19 | 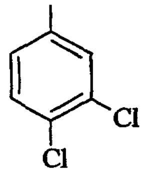 | 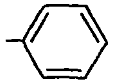 | 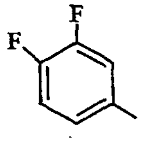 | 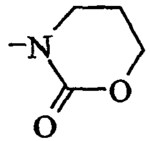 | HCl | RMN<br>$MH^+$= 644, tr = 6,46 |
| 20 | 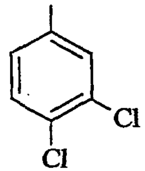 | 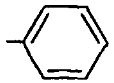 | 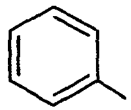 | 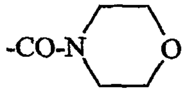 | HCl | + 29,2° (c = 0,2) |
| 21 | 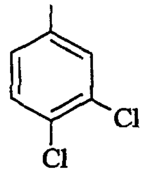 | 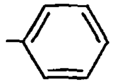 | 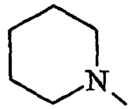 | -CONH2 | 2HCl | + 32,4° (c = 0,2) |
| 22 | 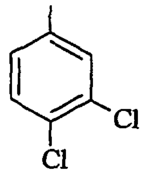 | 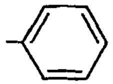 | 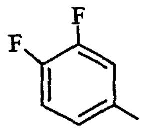 | 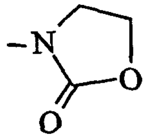 | HCl | + 29,6° (c =1) |
| 23 | 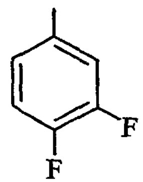 | 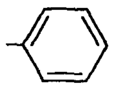 | 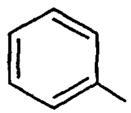 | $-NHCOCH_3$ | HCl | + 23,4° (c = 0,5) |

(suite)

| Composés n° | Ar | $R_1$ | $R_2$ | $R_3$ | Sel | RMN $\alpha_D^{20}$ = (c ; MeOH) $MH^+$, tr |
|---|---|---|---|---|---|---|
| 24 | 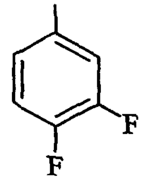 | 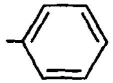 | 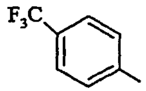 | $-NHCOCH_3$ | HCl | + 17° (c = 0,5) |
| 25 | 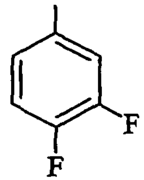 | 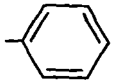 | 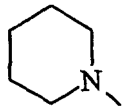 | $-CONH_2$ | 2HCl | + 18,2° (c = 0,5) |
| 26 | 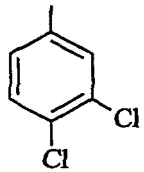 | 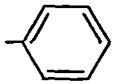 | 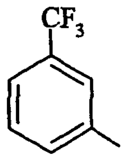 | $-NHCOCH_3$ | HCl | + 29,2° (c = 0,1) |
| 27 | 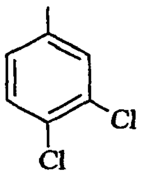 | 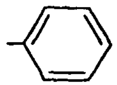 | 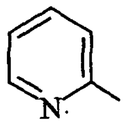 | -OH | HCl | + 30,4° (c =1) |
| 28 | 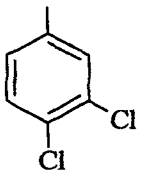 | 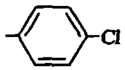 | 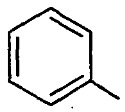 | $-NHCOCH_3$ | HCl | $MH^+$ = 600, tr = 7,02 |

(suite)

| Composés n° | Ar | $R_1$ | $R_2$ | $R_3$ | Sel | RMN $\alpha_D^{20} = (c ; MeOH)$ $MH^+$, tr |
|---|---|---|---|---|---|---|
| 29 | 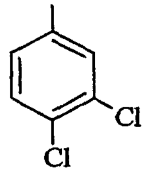 | 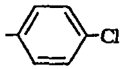 | 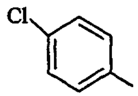 | 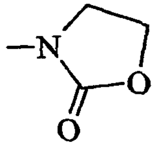 | HCl | $MH^+$ = 662, tr = 7,54 |
| 30 | 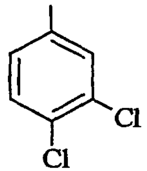 | 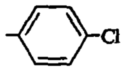 | 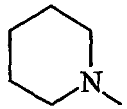 | $-CON(CH_3)_2$ | 2HCl | $MH^+$ = 621, tr = 7,45 |
| 31 | 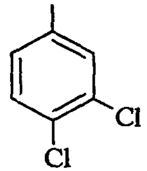 | 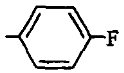 | 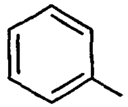 | $-NHCOCH_3$ | HCl | $MH^+$ = 584, tr = 6,69 |
| 32 | 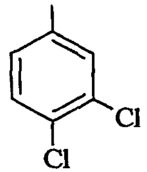 | 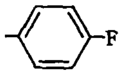 | 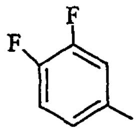 | $-NHCOCH_3$ | HCl | $MH^+$ = 620, tr = 6,93 |
| 33 | 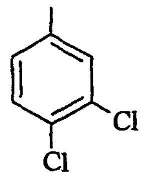 | 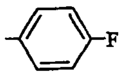 | 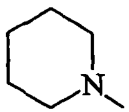 | $-CON(CH_3)_2$ | 2HCl | $MH^+$ = 605, tr = 7,27 |

(suite)

| Composés n° | Ar | $R_1$ | $R_2$ | $R_3$ | Sel | RMN $\alpha_D^{20}$ = (c ; MeOH) $MH^+$, tr |
|---|---|---|---|---|---|---|
| 34 | 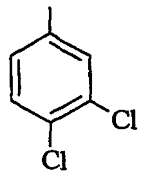 | 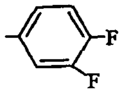 | 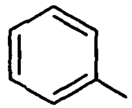 | $-NHCOCH_3$ | HCl | $MH^+$ = 602, tr = 6,90 |

RMN$^1$H du composé n°6:DMSO-$d_6$ : $\delta$(ppm) : 1,87 : s : 3H ; 1,9-2,1 : t : 2H ; 2,25 : s : 3H ; 2,3-3,2 : m : 8H; 3,3-3,5 : t : 2H; 4,13 : s : 2H ; 4,42 : dd : 2H ; 7,0-7,6 : m : 10H; 7,6-7,8 : m : 2H ; 8,0 : se : 1H; 10,2 : se : 1H.

RMN$^1$H du composé n° 8 : DMSO-$d_6$ : $\delta$(ppm) : 1,9 : s : 3H ; 2,1-2,4 : m : 2H ; 2,5-3,2 : m : 8H ; 3,3-3,6 : m : 2H ; 4,1 : s : 2H ; 4,4 : dd : 2H ; 7,2-7,6 : m : 10H ; 7,7-7,8 : 2d : 2H ; 8,2 : s : 1H; 10,6 : se : 1H.

RMN$^1$H du composé n° 11 : DMSO-$d_6$ : $\delta$(ppm) : 1,7-2,2 : m ; 4H ; 2,2-2,4 : t+d : 5H; 2,4-3,2 : m : 10H; 3,4-3,6 : t : 2H ; 4,14 : s : 2H ; 4,42 : dd : 2H ; 7,1-7,6 : m : 10H; 7,6-7,8 : m:2H; 10,2 : se : 1H.

RMN$^1$H du composé n° 19 : DMSO-$d_6$ : $\delta$(ppm) : 1,8-1,9 : m : 2H ; 2,19 : t : 2H ; 2,3-3,2 : m : 9H ; 3,2-3,6 : m : 3H ; 3,9-4,2 : m : 4H ; 4,4 : dd : 2H ; 7,1-7,6 : m : 9H ; 7,6-7,8 : m : 2H ; 10,5 : se : 1H.

Les composés selon l'invention ont fait l'objet d'essais pharmacologiques.

L'affinité des composés pour les récepteurs aux tachykinines $NK_2$ a été évaluée in vitro en mesurant l'inhibition de la liaison de la [$^{125}$I]His-neurokinine A ([$^{125}$I]His-NKA) aux récepteurs clonés $NK_2$ humains exprimés par des cellules CHO (Y. Takeda et al., J. Neurochem., 1992, 59, 740-745).

Les essais ont été effectués selon Emonds-Alt et al. (J. Pharmacol. Exp. Ther., 2002, 303,1171-1179).

Les composés selon l'invention inhibent fortement la liaison [$^{125}$I]His-NKA aux récepteurs $NK_2$ humains clonés et exprimés dans les cellules CHO avec un constante d'inhibition Ki comprise entre 50 nM et 0,05 nM. Ainsi, le composé n° 3 possède une constante d'inhibition (Ki) égale à 0.16 nM.

Les composés de la présente invention ont également été évalués in vivo dans un modèle de pharmacodynanie.

Chez la souris, l'injection dans le striatum d'un agoniste spécifique du récepteur $NK_2$ la [Nle$^{10}$]NKA(4-10) provoque un comportement de rotation qui est inhibé de manière dose-dépendante par les composés selon l'invention administrés par voie orale. Ce test a été effectué selon Poncelet et al. (Neurosci. Lett., 1993, 149, 40-42). Dans ce test, le composé n° 3 possède une dose efficace 50 ($DE_{50}$) de 0.023 mg par kg par voie orale.

Ces résultats pharmacologiques montrent que les composés selon l'invention, en particulier le composé n° 3, sont des antagonistes des récepteurs $NK_2$ en bloquant les effets pharmacologiques provoqués par la neurokinine A. De plus, ces résultats montrent que les composés selon l'invention passent bien la barrière hématoencéphalique et sont actifs par voie orale.

Selon un autre de ses aspects, la présente invention concerne l'utilisation des composés de formule (I), ou de l'un de leurs sels, solvats et/ou hydrates pharmaceutiquement acceptables pour la préparation de médicaments destinés à traiter et à prévenir toute pathologie centrale et/ou périphérique où la neurokinine A par l'intermédiare de son récepteur $NK_2$, est impliquée.

Les composés selon l'invention peuvent donc être utilisés pour la préparation de médicaments, en particulier de médicaments antagonistes des récepteurs $NK_2$ de la neurokinine A.

Ainsi selon un autre de ses aspects, l'invention a pour objet des médicaments qui comprennent un composé de formule (I), ou un sel d'addition de ce dernier à un acide pharmaceutiquement acceptable ou encore un hydrate ou un solvat du composé de formule (I).

Ces médicaments trouvent leur emploi en thérapeutique et sont notamment utiles:

- comme analgésique, en particulier dans le traitement de douleurs traumatiques telles que les douleurs post-opératoires ; de la névralgie du plexus brachial ; de douleurs chroniques telles que les douleurs arthritiques due à l'ostéoarthrite, l'arthrite rhumatoïde ou l'arthrite psoriatique ; de douleurs neuropathiques telles que la névralgie post-herpétique, la névralgie du trijumeau, la névralgie segmentale ou intercostale, la fibromyalgie, la causalgie, la neuropathie périphérique, la neuropathie diabétique, les neuropathies induites par une chimiothérapie, les neuropathies liées au SIDA, la névralgie occipitale, la névralgie géniculée, la névralgie glossopharyngienne ; de douleurs de l'illusion des amputés ; de diverses formes de mal de tête telle que la migraine chronique ou aiguë, de douleur temporomandibulaire, de douleur du sinus maxillaire, du névralgisme facial, d'odontalgie ; de douleur du cancéreux ; de douleur d'origine viscérale ; de douleur gastro-intestinale ; de douleur due à la compression nerveuse, de douleurs dues à la pratique intensive d'un sport ; de la dysménorrhée ; de douleur menstruelle ; de douleur due à une méningite, à une arachnoïdite ; de douleur muscosquelettique ; de douleurs du bas du dos dues à une sténose spinale, à un disque prolabé, à une sciatique ; de douleur de l'angineux ; de douleurs dues à une spondylite ankylosante ; de douleurs liées à la goutte ; de douleurs liées aux brûlures, à la cicatrisation, à une dermatose prurigineuse ; de douleur thalamique ;

- comme antiinflammatoire en particulier pour traiter les inflammations dans l'asthme, l'influenza, les bronchites chroniques (en particulier la bronchite chronique obstructive, COPD de l'anglais chronic obstructive pulmonary disease), les toux, les allergies, le bronchospasme et l'arthrite rhumatoïde ; les maladies inflammatoires du système gastro-intestinal par exemple la maladie de Crohn, les colites ulcératives, les pancréatites, les gastrites, l'inflammation des intestins, les troubles causés par les antiinflammatoires non-stéroidiens, les effets inflammatoires et secrétoires dûs aux infections bactériennes par exemple dûe au Clostridium difficile ; les maladies inflammatoires de la peau, par exemple l'herpès et l'eczéma ; les maladies inflammatoires de la vessie telles que la cystite et l'incontinence ; les

inflammations ophtalmiques telles que la conjonctivite, la vitréorétinopathie ; les inflammations dentaires telles que gingivite et périodontite ;

- dans le traitement des maladies allergiques en particulier de la peau comme l'urticaire, les dermatites de contact, les dermatites atopiques et des maladies respiratoires comme les rhinites ;
- dans le traitement des maladies du système nerveux central en particulier des psychoses telles que la schizophrénie, la manie et la démence ; des désordres cognitifs telle que la maladie d'Alzheimer, l'anxiété, la démence liée au SIDA ; des neuropathies diabétiques ; de la dépression ; de la maladie de Parkinson ; de la dépendance aux drogues; des abus de substances ; des troubles de la vigilance, du sommeil, du rythme circadien, de l'humeur, de l'épilepsie ; du syndrôme de Down ; de la chorée d'Huntington ; des désordres somatiques liés au stress ; des maladies neurodégénératives telles que la maladie de Pick, la maladie de Creutzfeldt-Jacob ; des désordres liés à la panique, à la phobie, au stress ; des troubles obsessionnels compulsifs ; des tics ; des troubles bipolaires ; des troubles schizoaffectifs ; des troubles de la personnalité ; des troubles liés aux déficits d'attention ou d'hyperactivité ;
- dans le traitement des modifications de la perméabilité de la barrière hématoencéphalique durant les processus inflammatoires et auto-immuns du système nerveux central, par exemple lors d'infections liées au SIDA ;
- comme myorelaxant et antispasmodique ;
- dans le traitement des nausées et vomissements aigus ou retardés et anticipés, par exemple les nausées et vomissements induits par des drogues comme les agents utilisés en chimiothérapie lors d'un cancer ; par thérapies par rayons lors d'irradiations du thorax ou de l'abdomen dans le traitement du cancer, de carcinoïdose ; par ingestion de poison ; par des toxines dues à des désordres métaboliques ou infectieux comme les gastrites, ou produites lors d'infection gastro-intestinale bactérienne ou virale ; lors d'une grossesse ; lors de troubles vestibulaires telles que le mal des transports, les vertiges, le syndrome de Ménière ; lors des maladies post-opératoires ; les nausées et vomissements induits par dialyse, par les prostaglandines ; par obstructions gastro-intestinales ; lors de motilité gastro-intestinale réduite ; lors de douleurs viscérales par infarctus du myocarde ou péritonite ; lors de migraine ; lors du mal d'altitude ; par ingestion d'analgésiques opiacés comme la morphine ; lors de reflux gastro-oesophagien ; lors d'indigestion acide ou de surconsommation de nourriture ou de boisson, lors d'acidité ou aigreur gastrique, régurgitation, brûlure d'estomac, par exemple épisodiques, nocturnes ou induites par un repas et dyspepsie ;
- dans le traitement des maladies du système gastro-intestinal telles que le syndrome du colon irritable, les ulcères gastriques et duodénaux, les ulcères oesophagiques, les diarrhées, les hypersécrétions, les lymphomes, les gastrites, les reflux gastro-oesophagiens, l'incontinence fécale, la maladie de Hirschsprung ;
- dans le traitement des maladies de la peau telles que le psoriasis, les prurits, les brûlures, notamment les coups de soleil ;
- dans le traitement des maladies du système cardiovasculaire telles que l'hypertension, les aspects vasculaires de la migraine, les oedèmes, la thrombose, l'angine de poitrine, les spasmes vasculaires, les maladies circulatoires dûes à une vasodilatation, la maladie de Raynaud, les fibroses, les maladies du collagène, l'athérosclérose, la préeclampsie ;
- dans le traitement des cancers du poumon à petites cellules et à grandes cellules ; des cancers du sein ; des tumeurs cérébrales ; des adéno-carcinomes de la sphère urogénitale ; dans le traitement adjuvant pour prévenir les métastases ;
- les maladies de démyelination telles que la sclérose en plaques ou la sclérose latérale amyotrophique ;
- dans le traitement des maladies du système immunitaire liées à la suppression ou à la stimulation des fonctions des cellules immunes par exemple l'arthrite rhumatoïde, le psoriasis, la maladie de Crohn, le diabète, le lupus, les réactions de rejet après transplantation ;
- dans le traitement des troubles de la miction en particulier la pollakiurie, l'incontinence d'effort, l'incontinence d'urgence, l'incontinence post-partum ;
- dans le traitement de l'hypertrophie de la prostate ;
- dans le traitement de la réticulose histiocytaire comme dans les tissus lymphatiques ;
- comme anorexigène ;
- dans le traitement de l'emphysème ; du syndrome de Reiter ; des hémorroïdes ;
- dans le traitement des troubles oculaires telles que le glaucome, l'hypertension oculaire, le myosis, l'excès de larmes ;
- dans le traitement ou la prévention d'une attaque, de l'épilepsie, des traumatismes de la tête, des traumatismes du cordon spinal, des lésions ischémiques cérébrales dûe à une attaque ou à une occlusion vasculaire ;
- dans le traitement des troubles de la fréquence et du rythme cardiaque, en particulier ceux qui sont occasionnés par la douleur ou le stress ;
- dans le traitement des peaux sensibles et pour prévenir ou combattre les irritations de la peau ou des muqueuses, les pellicules, les érythèmes ou les prurits ;
- dans le traitement des troubles neurologiques de la peau tels que lichens, prurigos, toxidermies prurigineuses, prurits sévères d'origine neurogène ;
- dans le traitement des ulcères et de toutes maladies causées par Helicobacter Pylori ou une bactérie uréase positive

gram négative ;

- dans le traitement des maladies causées par l'angiogénèse ou dont l'angiogénèse est un symtôme ;
- dans le traitement des algies oculaires et/ou palbébrales et/ou les dysesthésies oculaires ou palpébrales ;
- comme antiperspirant.

Selon la présente invention, les composés de formule (I) sont tout particulièrement utiles pour le traitement du syndrome du colon irritable (IBS) ; de la fibromyalgie ; de la douleur neuropathique ; du syndrome de fatigue chronique ; de la migraine ; de la douleur faciale atypique ; de la maladie de Crohn ; des colites ulcératives ; de la constipation ; des diarrhées ; du reflux gastro-oesophagien ; des gastrites ; des pancréatites ; de la dépression majeure ; de l'anxiété tel que l'anxiété généralisée, l'anxiété sociale, phobique et la panique ; des troubles obsessionnels compulsifs ; des tics ; de la manie ; des troubles bipolaires ; de la schizophrénie ; des troubles schizoaffectifs ; des troubles de la personnalité ; des désordres psychotiques ; des troubles liés aux déficits d'attention ou d'hyperactivité ; des troubles dus à l'usage de substances addictives ; de l'hypertrophie de la prostate.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un solvat ou hydrate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | : 50,0 mg |
| Mannitol | : 223,75 mg |
| Croscarmellose sodique | : 6,0 mg |
| Amidon de maïs | : 15,0 mg |
| Hydroxypropyl-méthylcellulose | : 2,25 mg |
| Stéarate de magnésium | : 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

**1.** Composé répondant à la formule (I) :

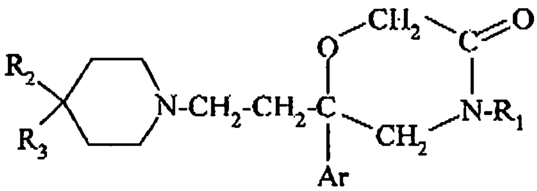

(I)

dans laquelle :

- Ar représente un phényle disubstitué par un atome d'halogène ;
- $R_1$ représente un phényle non substitué ou substitué une ou deux fois par un atome d'halogène ;
- $R_2$ représente :

  . un pyridyle ;
  . un phényle non substitué ou substitué une ou deux fois par un ou deux substituants choisis indépendamment parmi un atome d'halogène, un $(C_1-C_4)$alkyle, un $(C_1-C_4)$alcoxy, un groupe trifluorométhyle, un groupe trifluorométhoxy;
  . $R_2$ peut de plus représenter un radical hétérocyclique choisi parmi l'azétidine, la pyrrolidine, la pipéridine, la morpholine, la thiomorpholine ou la perhydroazétidinc lorsque $R_3$ représente un groupe $-CONR_{11}R_{12}$ ;

- $R_3$ représente un groupe choisi parmi :

  (5) $-(CH_2)_q-OH$ dans lequel q est 0 ;
  (10) $-(CH_2)_q-NR_7COR_8$ dans lequel q est 0 ;
  (11) $-(CH_2)_q-NR_7COOR_9$ dans lequel q est 0 ;
  (18) $-CONR_{11}R_{12}$ ;

- $R_7$ représente un atome d'hydrogène ou un $(C_1-C_4)$alkyle ;
- $R_8$ représente un atome d'hydrogène ; un $(C_1-C_4)$alkyle ; un vinyle ; un phényle ; un benzyle ; un pyridyle ; un $(C_3-C_7)$cycloalkyle non substitué ou substitué par un ou plusieurs méthyles ; un furyle ; un thiényle ; un pyrrolyle ; un imidazolyle ;
- ou $R_7$ et $R_8$ ensemble représentent un groupe $-(CH_2)_p-$ ;
- p est 3 ou 4 ;
- $R_9$ représente un $(C_1-C_4)$alkyle ou un phényle ;
- ou $R_7$ et $R_9$ ensemble représentent un groupe $-(CH_2)_n-$ ;
- n est 2 ou 3 ;
- $R_{11}$ et $R_{12}$ représentent chacun indépendamment un hydrogène ou un $(C_1-C_4)$alkyle ; $R_{12}$ peut de plus représenter un $(C_3-C_7)$cycloalkyle, un $(C_3-C_7)$cycloalkylméthyle, un hydroxy, un $(C_1-C_4)$alcoxy, un benzyle ou un phényle ; ou $R_{11}$ et $R_{12}$ ensemble avec l'atome d'azote auquel ils sont liés constituent un hétérocycle choisi parmi l'azétidine, la pyrrolidine, la pipéridine, la morpholine, la thiomorpholine ou la perhydroazépine ;

à l'état de base ou de sel d'addition à un acide, ainsi qu'à l'état d'hydrate ou de solvat.

**2.** Composé de formule (I) selon la revendication 1, **caractérisé en ce que** :

- Ar représente un 3,4-dichlorophényle ou un 3,4-difluorophényle ;
- $R_1$ représente un phényle, un 4-chlorophényle, un 4-fluorophényle, un 3,4-difluorophényle ;
- $R_2$ représente :

  . un pyridin-2-yle ;
  . un phényle, un 4-chlorophényle, un 3-fluorophényle, un 4-fluorophényle, un 3,4-difluorophényle, un 3-méthylphényle, un 3,4-diméthylphényle, un 4-méthoxyphényle, un 3-(trifluorométhyl)phényle, un 4-(trifluorométhyl)phényle, un 4-(trifluorométhoxy)phényle ;
  . $R_2$ peut de plus représenter un pipéridin-1-ylc lorsque $R_3$ représente un groupe-$CONH_2$ ou un groupe $CON(CH_3)_2$ ;

- $R_3$ représente un groupe choisi parmi :

. un hydroxy ;
. $-NHCOCH_3$ ;

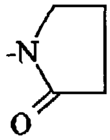 ; 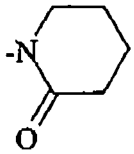 ;

.

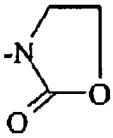 ; 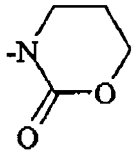 ;

. $-CONH_2$ ;

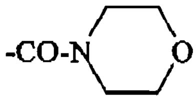 ;

$-CON(CH_3)_2$ ;

à l'état de base ou de sel d'addition à un acide, ainsi qu'à l'état d'hydrate ou de solvat.

**3.** Composé selon la revendication 1 de formule (I) choisi parmi :

- 6-(3,4-dichlorophényl)-6-[2-[4-hydroxy-4-[3-(trifluorométhyl)phényl]pipéridin-1-yl]éthyl]-4-phénylmorpholin-3-one, isomère dextrogyre ;
- N-[1-[2-[2-(3,4-dichlorophényl)-5-oxo-4-phénylmorpholin-2-yl]éthyl]-4-phénylpipéridin-4-yl]acétamide, isomère dextrogyre ;
- N-[1-[2-[2-(3,4-dichlorophényl)-5-oxo-4-phénylmorpholin-2-yl]éthyl]-4-(3-fluorophényl)pipéridin-4-yl]acétamide, isomère dextrogyre ;
- N-[1-[2-[2-(3,4-dichlorophényl)-5-oxo-4-phénylmorpholin-2-yl]éthyl]-4-(3,4-difluorophényl)pipéridin-4-yl]acétamide, isomère dextrogyre ;
- N-[1-[2-[2-(3,4-dichlorophényl)-5-oxo-4-phénylmorpholin-2-yl]éthyl]-4-(4-méthylphényl)pipéridin-4-yl]acétamide, isomère dextrogyre ;
- N-[1-[2-[2-(3,4-dichlorophényl)-5-oxo-4-phénylmorpholin-2-yl]éthyl]-4-(4-méthoxyphényl)pipéridin-4-yl]acétamide, isomère dextrogyre ;
- N-[1-[2-[2-(3,4-dichlorophényl)-5-oxo-4-phénylmorpholin-2-yl]éthyl]-4-[4-(trifluorométhoxy)phényl]pipéridin-4-yl]acétamide, isomère dextrogyre ;
- 6-(3,4-dichlorophényl)-6-[2-[4-(3-fluorophényl)-4-(2-oxopyrrolidin-1-yl)pipéridin-1-yl]éthyl]-4-phénylmorpholin-3-one, isomère dextrogyre ;
- 6-(3,4-dichlorophényl)-6-[2-[4-(3,4-difluorophényl)-4-(2-oxopyrrolidin-1-yl)pipéridin-1-yl]éthyl]-4-phénylmorpholin-3-one, isomère dextrogyre ;
- 6-(3,4-dichlorophényl)-6-[2-[4-(4-méthylphényl)-4-{2-oxopyrrolidin-1-yl)pipéridin-1-yl]éthyl]-4-phénylmorpholin-3-one, isomère dextrogyre ;
- 1'-[2-[2-(3,4-dichlorophényl)-5-oxo-4-phénylmorpholin-2-yl]éthyl]-4'-(4-méthylphényl)-1-4'-bipipéridin-2-ane, isomère dextrogyre ;
- 6-(3,4-dichlorophényl)-6-[2-[4-(3-méthylphényl)-4-(2-oxopyrrolidin-1-yl)pipéridin-1-yl]éthyl]-4-phénylmorpholin-3-one, isomère dextrogyre ;
- 6-(3,4-dichlorophényl)-6-[2-[4-(3-fluorophényl)-4-(2-oxo-1,3-oxazolidin-3-yl)pipéridin-1-yl]éthyl]-4-phénylmorpholin-3-one, isomère dextrogyre ;

- 6-(3,4-dichlorophényl)-6-[2-[4-(4-fluorophényl)-4-(2-oxo-1,3-oxazolidin-3-yl)pipéridin-1-yl]éthyl]-4-phénylmorpholin-3-one, isomère dextrogyre ;
- 6-(3,4-dichlorophényl)-6-[2-[4-(3,4-diméthylphényl)-4-(2-oxo-1,3-oxazolidin-3-yl)pipéridin-1yl]éthyl]-4-phénylmorpholin-3-one, isomère dextrogyre ;
- 3-[1-[2-[2-(3,4-dichlorophényl)-5-oxo-4-phénylmorpholin-2-yl]éthyl]-4-phénylpipéridin-4-yl]-1,3-oxazinan-2-one, isomère dextrogyre ;
- 3-[1-[2-[2-(3,4-dichlorophényl)-5-oxo-4-phénylmorpholin-2-yl]éthyl]-4-(4-fluorophényl)pipéridin-4-yl]-1,3-oxazinan-2-one, isomère dextrogyre ;
- 3-[1-[2-[2-(3,4-dichlorophényl)-5-oxo-4-phénylmorpholin-2-yl]éthyl]-4-(3,4-difluorophényl)pipéridin-4-yl]-1,3-oxazinan-2-one, isomère dextrogyre ;
- 6-(3,4-dichlorophényl)-6-[2-[4-(morpholin-4-ylcarbonyl)-4-phénylpipéridin-1-yl]éthyl]-4-phénylmorpholin-3-one, isomère dextrogyre ;
- 1'-[2-[2-(3,4-dichlorophényl)-5-oxo-4-phénylmorpholin-2-yl]éthyl]-1,4'-bipipéridine-4'-carboxamide, isomère dextrogyre ;
- 6-(3,4-dichlorophényl)-6-[2-[4-(3,4-difluorophényl)-4-(2-oxo-1,3-oxazolidin-3-yl)pipéridin-1-yl]éthyl]-4-phénylmorpholin-3-one, isomère dextrogyre;
- N-[1-[2- [2-(3,4-difluorophényl)-5-oxo-4-phénylmorpholin-2-yl]éthyl]-4-phénylpipéridin-4-yl]acétamide, isomère dextrogyre ;
- N-[1-[2-[2-(3,4-difluorophényl)-5-oxo-4-phénylmorpholin-2-yl]éthyl]-4-[4-(trifluorométhyl)phényl]pipéridin-4-yl]acétamide, isomère dextrogyre ;
- 1'-[2-[2-(3,4-difluorophényl)-5-oxo-4-phénylmorpholin-2-yl]éthyl]-1,4'-bipipéridine-4'-carboxamide, isomère dextrogyre ;
- N-[1-[2-[2-(3,4-dichlorophényl)-5-oxo-4-phénylmorpholin-2-yl]éthyl]-4-[3-(trifluorométhyl)phényl]piperidin-4-yl)acetamide, isomère dextrogyre ;
- 6-(3,4-dichlorophényl)-6-[2-(4-hydroxy-4-pyridin-2-ylpipéridin-1-yl)éthyl]-4-phénylmorpholin-3-one, isomère dextrogyre;
- N-[1-[2-[4-(4-chlorophényl)-2-(3,4-dichlorophényl)-5-oxo-morpholin-2-yl]éthyl]-4-phénylpipéridin-4-yl]acétamide ;
- 4-(4-chlorophényl)-6-[2-[4-(4-chlorophényl)-4-(2-oxo-1,3-oxazolidin-3-yl)pipéridin-1-yl]éthyl]-6-(3,4-dichlorophényl)morpholin-3-one ;
- 1'-[2-[4-(4-chlorophényl)-2-(3,4-dichlorophényl)-5-oxo-morpholin-2-yl]éthyl]-N,N-diméthyl-1,4'-bipipéridine-4'-carboxamide ;
- N-[1-[2-[2-(3,4-dichlorophényl)-4-(4-fluorophényl)-5-oxo-morpholin-2-yl]ethyl]-4-phénylpipéridin-4-yl]acetamide ;
- N-[1-[2-[2-(3,4-dichlorophényl)-4-(4-fluorophényl)-5-oxo-morpholin-2-yl]éthyl]-4-(3,4-difluorophényl)pipéridin-4-yl]acétamide ;
- 1'-[2-[2-(3,4-dichlorophényl)-4-(4-fluorophényl)-5-oxo-morpholin-2-yl]éthyl]-N,N-diméthyl-1,4'-bipipéridine-4-carboxamide ;
- N-[1-[2-[2-(3,4-dichlorophényl)-4-(3,4-difluorophenyl)-5-oxo-morpholin-2-yl]éthyl]-4-phénylpipéridin-4-yl]acétamide ;

à l'état de base ou de sel d'addition à un acide, ainsi qu'à l'état d'hydrate ou de solvat.

**4.** Composé selon la revendication 1 de formule (I) qui est le :

- N-[1-[2-[2-(3,4-dichlorophényl)-5-oxo-4-phénylmorpholin-2-yl]éthyl]-4-(3-fluorophényl)pipéridin-4-yl]acétamide, isomère dextrogyre ;

à l'état de base ou de sel d'addition à un acide, ainsi qu'à l'état d'hydrate ou de solvat.

**5.** Procédé de préparation d'un composé de formule (I) selon l'une quelconque des revendications 1 à 4, **caractérisé en ce que** :

on fait réagir un composé de formule :

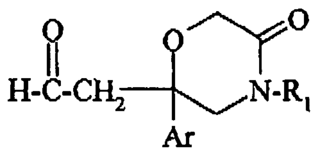

(II)

dans laquelle Ar et $R_1$ sont tels que définis pour un composé de formule (I) dans la revendication 1, avec un composé de formule :

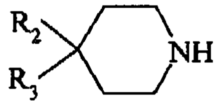

(III)

dans laquelle $R_2$ et $R_3$ sont tels que définis pour un composé de formule (I) dans la revendication 1, en présence d'un acide, dans un solvant, puis on réduit le sel d'iminium formé intermédiairement au moyen d'un agent réducteur.

6. Procédé de préparation d'un composé de formule (T) selon l'une quelconque des revendications 1 à 4, **caractérisé en ce que** :

on fait réagir un composé de formule :

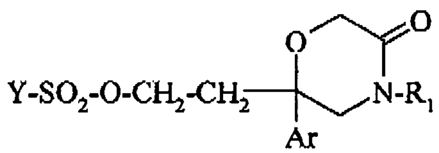

(IV)

dans laquelle Ar et $R_1$ sont tels que définis pour un composé de formule (I) dans la revendication 1, et Y représente un groupe méthyle, phényle, tolyle ou trifluorométhyle, avec un composé de formule :

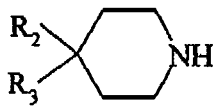

(III)

dans laquelle $R_2$ et $R_3$ sont tels que définis pour un composé de formule (1) dans la revendication 1.

7. Composé de formule :

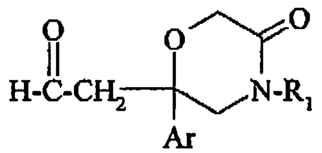

(II)

dans laquelle :

- Ar représente un phényle disubstitué par un atome d'halogène ;
- $R_1$ représente un phényle non substitué ou substitué une ou deux fois par un atome d'halogène.

8. Composé de formule :

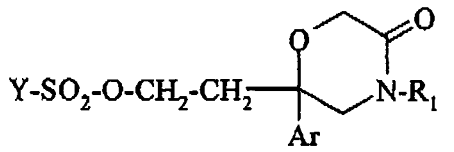

(IV)

dans laquelle :

- Y représente un groupe méthyle, phényle, tolyle, trifluorométhyle ;
- Ar représente un phényle disubstitué par un atome d'halogène ;
- $R_1$ représente un phényle non substitué ou substitué une ou deux fois par un atome d'halogène.

**9.** Composé de formule :

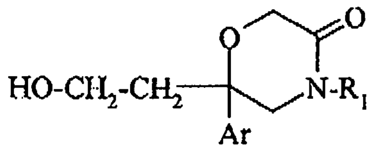

(V)

dans laquelle :

- Ar représente un phényle mono- ou disubstitué par un atome d'halogène ;
- $R_1$ représente un phényle non substitué ou substitué une ou deux fois par un ou deux substituants choisis indépendamment parmi un atome d'halogène, un $(C_1$-$C_4)$alkyle, un $(C_1$-$C_4)$alcoxy.

**10.** Composé de formule:

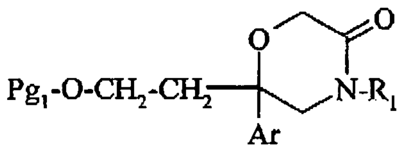

(VI)

dans laquelle :

- $Pg_1$ représente un radical tétrahydropyran-2-yle, benzoyle ou $(C_1$-$C_4)$alkylcarbonyle ;
- Ar représente un phényle disubstitué par un atome d'halogène ;
- $R_1$ représente un phényle non substitué ou substitué une ou deux fois par un atome d'halogène.

**11.** Composé de formule:

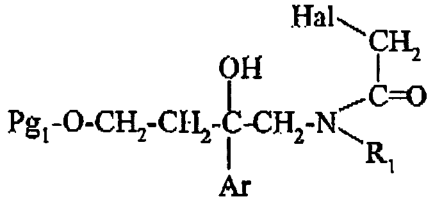

(VII)

dans laquelle :

- Hal représente un atome de chlore ou de brome ;
- $Pg_1$ représente un radical tétrahydropyran-2-yle, benzoyle ou $(C_1$-$C_4)$alkylcarbonyle ;
- Ar représente un phényle disubstitué par un atome d'halogène ;

- $R_1$ représente un phényle non substitué ou substitué une ou deux fois par un atome d'halogène.

**12.** Composé de formule:

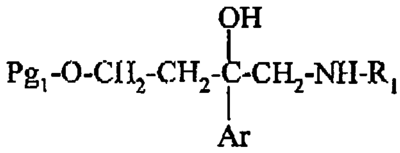

(VIII)

dans laquelle :

- $Pg_1$ représente un radical tétrahydropyran-2-yle, benzoyle ou $(C_1\text{-}C_4)$alkylcarbonyle ;
- Ar représente un phényle disubstitué par un atome d'halogène ;
- $R_1$ représente un phényle non substitué ou substitué une ou deux fois par un atome d'halogène.

**13.** Composé de formule :

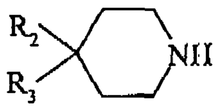

(III)

dans laquelle :

- $R_2$ représente :

  . un pyridyle ;
  . un phényle non substitué ou substitué une ou deux fois par un ou deux substituants choisis indépendamment parmi un atome d'halogène, un $(C_1\text{-}C_4)$alkyle, un $(C_1\text{-}C_4)$alcoxy, un groupe trifluorométhyle, un groupe trifluorométhoxy ;

- $R_3$ représente un groupe :

  (11) $-(CH_2)_q-NR_7COOR_9$;

- q est 0 ;
- $R_7$ et $R_9$ ensemble représentent un groupe $-(CH_2)_n-$ ;
- n est 2 ou 3 ;

à l'état de base ou de sel d'addition à un acide.

**14.** Médicament, **caractérisé en ce qu'**il contient un composé de formule (I) selon l'une quelconque des revendications 1 à 4, ou un sel d'addition de ce composé à un acide pharmaceutiquement acceptable, ou encore un hydrate ou un solvat du composé de formule (1).

**15.** Composition pharmaceutique, **caractérisé en ce qu'**elle comprend un composé de formule (I) selon l'une quelconque des revendications 1 à 4, ou un sel pharmaceutiquement acceptable, un hydrate ou un solvat de ce composé, ainsi qu'au moins un excipient pharmaceutiquement acceptable.

**16.** Utilisation d'un composé de formule (I) selon l'une quelconque des revendications 1 à 4 pour la préparation d'un médicament destiné au traitement et à la prévention du syndrome du colon irritable (IBS) ; de la fibromyalgie ; de la douleur neuropathique ; du syndrome de fatigue chroniques ; de la migraine ; de la douleur faciale atypique ; de la maladie de Crohn ; des colites ulcératives ; de la constipation ; des diarrhées ; du reflux gastro-oesophagien ; des gastrites ; des pancréatites ; de la dépression majeure ; de l'anxiété tel que l'anxiété généralisée, l'anxiété sociale, phobique et la panique ; des troubles obsessionnels compulsifs ; des tics ; de la manie ; des troubles bipolaires ; de la schizophrénie ; des troubles schizoaffectifs ; des troubles de la personnalité ; des désordres

psychotiques ; des troubles liés aux déficits d'attention ou d'hyperactivité ; des troubles dus à l'usage de substances addictives ; de l'hypertrophie de la prostate.

## Patentansprüche

**1.** Verbindung der Formel (I):

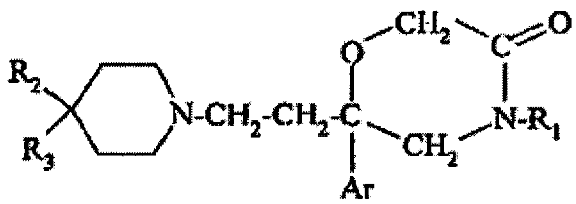

(I)

worin:

- Ar für ein Phenyl, das zweifach durch ein Halogenatom substituiert ist, steht;
- $R_1$ für ein Phenyl, das unsubstituiert oder ein- oder zweifach durch ein Halogenatom substituiert ist, steht;
- $R_2$ für:

  . ein Pyridyl ;
  . ein Phenyl, das unsubstituiert oder ein- oder zweifach durch einen oder zwei Substituenten, die unabhängig voneinander aus einem Halogenatom, einem $(C_1-C_4)$-Alkyl, einem $(C_1-C_4)$-Alkoxy, einer Trifluormethylgruppe und einer Trifluormethoxygruppe ausgewählt sind, substituiert ist;

steht;

  . $R_2$ außerdem für einen heterocyclischen Rest stehen kann, der aus Azetidin, Pyrrolidin, Piperidin, Morpholin, Thiomorpholin oder Perhydroazetidin ausgewählt ist, wenn $R_3$ für eine Gruppe $-CONR_{11}R_{12}$ steht;

- $R_3$ für eine Gruppe steht, die aus:

  (5) $-(CH_2)_q-OH$, worin q für 0 steht;
  (10) $-(CH_2)_q-NR_7COR_8$, worin q für 0 steht;
  (11)$-(CH_2)_q-NR_7COOR_9$, worin q für 0 steht; und
  (18) $-CONR_{11}R_{12}$

ausgewählt ist;

- $R_7$ für ein Wasserstoffatom oder ein $(C_1-C_4)$-Alkyl steht;
- $R_8$ für ein Wasserstoffatom; ein $(C_1-C_4)$-Alkyl; ein Vinyl; ein Phenyl; ein Benzyl; ein Pyridyl; ein $(C_3-C_7)$-Cycloalkyl, das unsubstituiert oder durch eine oder mehrere Methylgruppen substituiert ist; ein Furyl; ein Thienyl; ein Pyrrolyl oder ein Imidazolyl steht;
- oder $R_7$ und $R_8$ zusammen eine Gruppe $-(CH_2)_p$-bilden;
- p für 3 oder 4 steht;
- $R_9$ für ein $(C_1-C_4)$-Alkyl oder ein Phenyl steht;
- oder $R_7$ und $R_9$ zusammen eine Gruppe $-(CH_2)_n$-bilden;
- n für 2 oder 3 steht;
- $R_{11}$ und $R_{12}$ jeweils unabhängig voneinander für einen Wasserstoff oder ein $(C_1-C_4)$-Alkyl stehen; $R_{12}$ außerdem für ein $(C_3-C_7)$-Cycloalkyl, ein $(C_3-C_7)$-Cycloalkylmethyl, ein Hydroxy, ein $(C_1-C_4)$-Alkoxy, ein Benzyl oder ein Phenyl stehen kann; oder $R_{11}$ und $R_{12}$ zusammen mit dem Stickstoffatom, an das sie gebunden sind, einen Heterocyclus bilden, der aus Azetidin, Pyrrolidin, Piperidin, Morpholin, Thiomorpholin oder Perhydroazepin ausgewählt ist;

in Basen- oder Säureadditionssalzform sowie in Hydrat- oder Solvatform.

**2.** Verbindung der Formel (I) nach Anspruch 1, **dadurch gekennzeichnet, dass**:

- Ar für ein 3,4-Dichlorphenyl oder ein 3,4-Difluorphenyl steht;
- $R_1$ für ein Phenyl, ein 4-Chlorphenyl, ein 4-Fluorphenyl oder ein 3,4-Difluorphenyl steht;
- $R_2$ für:

  . ein Pyridin-2-yl;
  . ein Phenyl, ein 4-Chlorphenyl, ein 3-Fluorphenyl, ein 4-Fluorphenyl, ein 3,4-Difluorphenyl, ein 3-Methylphenyl, ein 3,4-Dimethylphenyl, ein 4-Methoxyphenyl, ein 3-(Trifluormethyl)phenyl, ein 4-(Trifluormethyl)phenyl oder ein 4-(Trifluormethoxy)phenyl
  steht;
  . $R_2$ außerdem für ein Piperidin-1-yl stehen kann, wenn $R_3$ für eine Gruppe $-CONH_2$ oder eine Gruppe CON($CH_3$) steht;

- $R_3$ für eine Gruppe steht, die aus:

  .einem Hydroxy;
  .$NHCOCH_3$ ;

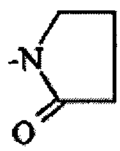 ; 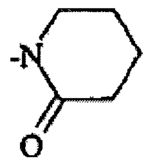 ;

.

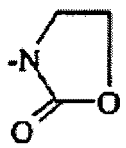 ; 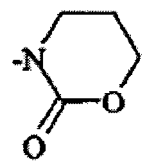 ;

. $-CONH_2$ ;

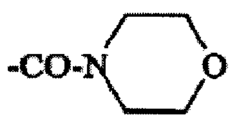

oder $-CON(CH_3)_2$ ausgewählt ist;

in Basen- oder Säureadditionssalzform sowie in Hydrat- oder Solvatform.

**3.** Verbindung nach Anspruch 1 der Formel (I), ausgewählt aus:

- 6-(3,4-Dichlorphenyl)-6-[2-[4-hydroxy-4-[3-(trifluormethyl)phenyl]piperidin-1-yl]ethyl]-4-phenyl-morpholin-3-on, rechtsdrehendes Isomer;
- N-[1-[2-[2-(3,4-Dichlorphenyl)-5-oxo-4-phenyl-morpholin-2-yl]ethyl]-4-phenylpiperidin-4-yl]acetamid, rechtsdrehendes Isomer;
- N-[1-[2-[2-(3,4-Dichlorphenyl)-5-oxo-4-phenyl-morpholin-2-yl]ethyl]-4-(3-fluorphenyl)piperidin-4-yl]acetamid, rechtsdrehendes Isomer;
- N-[1-[2-[2-(3,4-Dichlorphenyl)-5-oxo-4-phenyl-morpholin-2-yl]ethyl]-4-(3,4-difluor-phenyl)piperidin-4-yl]acetamid, rechtsdrehendes Isomer;
- N-[1-[2-[2-(3,4-Dichlorphenyl)-5-oxo-4-phenyl-morpholin-2-yl]ethyl]-4-(4-methylphenyl)piperidin-4-yl]acetamid, rechtsdrehendes Isomer;

- N-[1-[2-[2-(3,4-Dichlorphenyl)-5-oxo-4-phenyl-morpholin-2-yl]ethyl]-4-(4-methoxyphenyl)piperidin-4-yl]acetamid, rechtsdrehendes Isomer;
- N-[1-[2-[2-(3,4-dichlorphenyl)-5-oxo-4-phenyl-morpholin-2-yl]ethyl]-4-[4-(trifluormethoxy)-phenyl]piperidin-4-yl]acetamid, rechtsdrehendes Isomer;
- 6-(3,4-Dichlorphenyl)-6-[2-[4-(3-fluorphenyl)-4-(2-oxopyrrolidin-1-yl)piperidin-1-yl]ethyl]-4-phenylmorpholin-3-on, rechtsdrehendes Isomer;
- 6-(3,4-Dichlorphenyl)-6-[2-[4-(3,4-difluor-phenyl)-4-(2-oxopyrrolidin-1-yl)piperidin-4-yl]-ethyl]-4-phenylmorpholin-3-on, rechtsdrehendes Isomer;
- 6-(3,4-Dichlorphenyl)-6-[2-[4-(4-methylphenyl)-4-(2-oxopyrrolidin-1-yl)piperidin-1-yl]ethyl]-4-phenylmorpholin-3-on, rechtsdrehendes Isomer;
- 1'-[2-[2-(3,4-Dichlorphenyl)-5-oxo-4-phenyl-morpholin-2-yl]ethyl]-4'-(4-methylphenyl)-1,4'-bipiperidin-2-on, rechtsdrehendes Isomer;
- 6-(3,4-Dichlorphenyl)-6-[2-[4-(3-methylphenyl)-4-(2-oxopyrrolidin-1-yl)piperidin-1-yl]ethyl]-4-phenylmorpholin-3-on, rechtsdrehendes Isomer;
- 6-(3,4-Dichlorphenyl)-6-[2-[4-(3-fluorphenyl)-4-(2-oxo-1,3-oxazolidin-3-yl)piperidin-1-yl]ethyl]-4-phenylmorpholin-3-on, rechtsdrehendes Isomer;
- 6-(3,4-Dichlorphenyl)-6-[2-[4-(4-fluorphenyl)-4-(2-oxo-1,3-oxazolidin-3-yl)piperidin-1-yl]ethyl]-4-phenylmorpholin-3-on, rechtsdrehendes Isomer;
- 6-(3,4-Dichlorphenyl)-6-[2-[4-(3,4-dimethylphenyl)-4-(2-oxo-1,3-oxazolidin-3-yl)piperidin-1-yl]ethyl]-4-phenylmorpholin-3-on, rechtsdrehendes Isomer;
- 3-[1-[2-[2-(3,4-Dichlorphenyl)-5-oxo-4-phenyl-morpholin-2-yl]ethyl]-4-phenylpiperidin-4-yl]-1,3-oxazinan-2-on, rechtsdrehendes Isomer;
- 3-[1-[2-[2-(3,4-Dichlorphenyl)-5-oxo-4-phenyl-morpholin-2-yl]ethyl]-4-(4-fluorphenyl)piperidin-4-yl]-1,3-oxazinan-2-on, rechtsdrehendes Isomer;
- 3-[1-[2-[2-(3,4-Dichlorphenyl)-5-oxo-4-phenyl-morpholin-2-yl]ethyl]-4-(3,4-difluorphenyl)piperidin-4-yl]-1,3-oxazinan-2-on, rechtsdrehendes Isomer;
- 6-(3,4-Dichlorphenyl)-6-[2-[4-(morpholin-4-yl-carbonyl)-4-phenylpiperidin-1-yl]ethyl]-4-phenyl-morpholin-3-on, rechtsdrehendes Isomer;
- 1'-[2-[2-(3,4-Dichlorphenyl)-5-oxo-4-phenyl-morpholin-2-yl]ethyl]-1,4'-bipiperidin-4'-carboxamid, rechtsdrehendes Isomer;
- 6-(3,4-Dichlorphenyl)-6-[2-[4-(3,4-difluorphenyl)-4-(2-oxo-1,3-oxazolidin-3-yl)piperidin-1-yl]ethyl]-4-phenylmorpholin-3-on, rechtsdrehendes Isomer;
- N-[1-[2-[2-(3,4-Difluorphenyl)-5-oxo-4-phenyl-morpholin-2-yl]ethyl]-4-phenylpiperidin-4-yl]-acetamid, rechtsdrehendes Isomer;
- N-[1-[2-[2-(3,4-Difluorphenyl)-5-oxo-4-phenyl-morpholin-2-yl]ethyl]-4-[4-(trifluormethyl)-phenyl]piperidin-4-yl] acetamid, rechtsdrehendes Isomer;
- 1'-[2-[2-(3,4-Difluorphenyl)-5-oxo-4-phenyl-morpholin-2-yl]ethyl]-1,4'-bipiperidin-4'-carbox-amid, rechtsdrehendes Isomer;
- N-[1-[2-[2-(3,4-Dichlorphenyl)-5-oxo-4-phenyl-morpholin-2-yl]ethyl]-4-[3-(trifluormethyl)-phenyl]piperidin-4-yl] acetamid, rechtsdrehendes Isomer;
- 6-(3,4-Dichlorphenyl)-6-[2-(4-hydroxy-4-pyridin-2-ylpiperidin-1-yl)ethyl]-4-phenylmorpholin-3-on, rechtsdrehendes Isomer;
- N-[1-[2-[4-(4-Chlorphenyl)-2-(3,4-dichlorphenyl)-5-oxomorpholin-2-yl]ethyl]-4-phenylpiperidin-4-yl]acetamid;
- 4-(4-Chlorphenyl)-6-[2-[4-(4-chlorphenyl)-4-(2-oxo-1,3-oxazolidin-3-yl)piperidin-1-yl]ethyl]-6-(3,4-dichlorphenyl)morpholin-3-on;
- 1'-[2-[4-(4-Chlorphenyl)-2-(3,4-dichlorphenyl)-5-oxomorpholin-2-yl]ethyl]-N,N-dimethyl-1,4'-bipiperidin-4'-carboxamid;
- N-[1-[2-[2-(3,4-Dichlorphenyl)-4-(4-fluor-phenyl)-5-oxomorpholin-2-yl]ethyl]-4-phenylpiperidin-4-yl]acetamid;
- N-[1-[2-[2-(3,4-Dichlorphenyl)-4-(4-fluorphenyl)-5-oxomorpholin-2-yl]ethyl]-4-(3,4-difluor-phenyl)piperidin-4-yl]acetamid;
- 1'-[2-[2-(3,4-Dichlorphenyl)-4-(4-fluorphenyl)-5-oxomorpholin-2-yl]ethyl]-N,N-dimethyl-1,4'-bipiperidin-4'-carboxamid;
- N-[1-[2-[2-(3,4-Dichlorphenyl)-4-(3,4-difluorphenyl)-5-oxomorpholin-2-yl]ethyl]-4-phenylpiperidin-4-yl]acetamid;

in Basen- oder Säureadditionssalzform sowie in Hydrat- oder Solvatform.

**4.** Verbindung nach Anspruch 1 der Formel (I), bei der es sich um:

- N-[1-[2-[2-(3,4-Dichlorphenyl)-5-oxo-4-phenyl-morpholin-2-yl]ethyl]-4-(3-fluorphenyl)-piperidin-4-yl]acetamid, rechtsdrehendes Isomer,

handelt, in Basen- oder Säureadditionssalzform sowie in Hydrat- oder Solvatform.

**5.** Verfahren zur Herstellung einer Verbindung der Formel (I) nach einem der Ansprüche 1 bis 4, **dadurch gekennzeichnet, dass** man:

eine Verbindung der Formel:

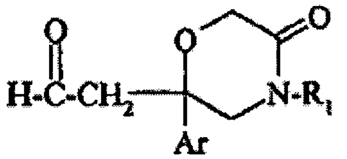

(II)

worin Ar und $R_1$ wie für eine Verbindung der Formel (I) in Anspruch 1 definiert sind, in Gegenwart einer Säure in einem Lösungsmittel mit einer Verbindung der Formel:

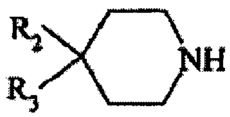

(III)

worin $R_2$ und $R_3$ wie für eine Verbindung der Formel (I) in Anspruch 1 definiert sind, umsetzt und dann das intermediär gebildete Iminiumsalz mit einem Reduktionsmittel reduziert.

**6.** Verfahren zur Herstellung einer Verbindung der Formel (I) nach einem der Ansprüche 1 bis 4, **dadurch gekennzeichnet, dass** man:

eine Verbindung der Formel:

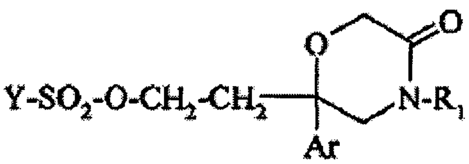

(IV)

worin Ar und $R_1$ wie für eine Verbindung der Formel (I) in Anspruch 1 definiert sind und Y für eine Methyl-, Phenyl-, Tolyl- oder Trifluormethylgruppe steht, mit einer Verbindung der Formel:

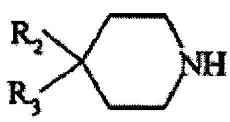

(III)

worin $R_2$ und $R_3$ wie für eine Verbindung der Formel (I) in Anspruch 1 definiert sind, umsetzt.

**7.** Verbindung der Formel:

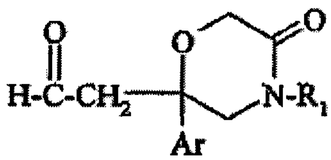

(II)

worin:

- Ar für ein Phenyl, das zweifach durch ein Halogenatom substituiert ist, steht;
- $R_1$ für ein Phenyl, das unsubstituiert oder ein- oder zweifach durch ein Halogenatom substituiert ist, steht.

**8.** Verbindung der Formel:

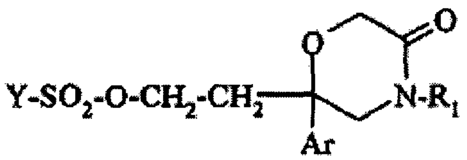

(IV)

worin:

- Y für eine Methyl-, Phenyl-, Tolyl- oder Trifluormethylgruppe steht;
- Ar für ein Phenyl, das zweifach durch ein Halogenatom substituiert ist, steht;
- $R_1$ für ein Phenyl, das unsubstituiert oder ein- oder zweifach durch ein Halogenatom substituiert ist, steht.

**9.** Verbindung der Formel:

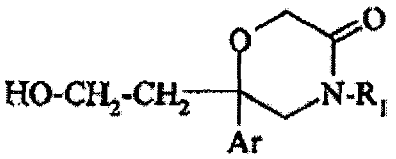

(V)

worin:

- Ar für ein Phenyl, das ein- oder zweifach durch ein Halogenatom substituiert ist, steht;
- $R_1$ für ein Phenyl, das unsubstituiert oder ein- oder zweifach durch einen oder zwei Substituenten, die unabhängig voneinander aus einem Halogenatom, einem ($C_1$-$C_4$)-Alkyl und einem ($C_1$-$C_4$)-Alkoxy ausgewählt sind, substituiert ist, steht.

**10.** Verbindung der Formel:

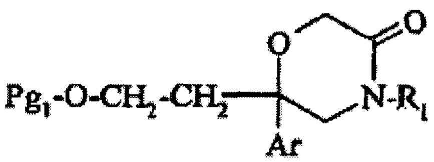

(VI)

worin:

- $Pg_1$ für einen Tetrahydropyran-2-yl-, Benzoyl- oder ($C_1$-$C_4$)-Alkylcarbonylrest steht;
- Ar für ein Phenyl, das zweifach durch ein Halogenatom substituiert ist, steht;
- $R_1$ für ein Phenyl, das unsubstituiert oder ein- oder zweifach durch ein Halogenatom substituiert ist, steht.

**11.** Verbindung der Formel:

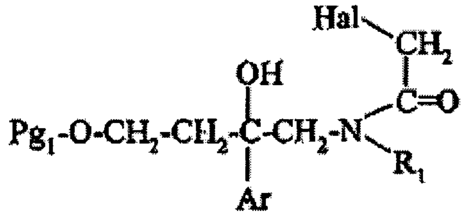

(VII)

worin:

- Hal für ein Chlor- oder Bromatom steht;
- $Pg_1$ für einen Tetrahydropyran-2-yl-, Benzoyl- oder $(C_1-C_4)$-Alkylcarbonylrest steht;
- Ar für ein Phenyl, das zweifach durch ein Halogenatom substituiert ist, steht;
- $R_1$ für ein Phenyl, das unsubstituiert oder ein- oder zweifach durch ein Halogenatom substituiert ist, steht.

**12.** Verbindung der Formel:

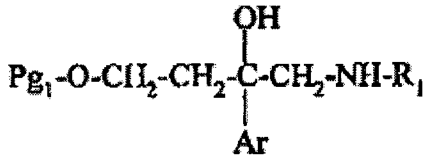

(VIII)

worin:

- $Pg_1$ für einen Tetrahydropyran-2-yl-, Benzoyl- oder $(C_1-C_4)$-Alkylcarbonylrest steht;
- Ar für ein Phenyl, das zweifach durch ein Halogenatom substituiert ist, steht;
- $R_1$ für ein Phenyl, das unsubstituiert oder ein- oder zweifach durch ein Halogenatom substituiert ist, steht.

**13.** Verbindung der Formel:

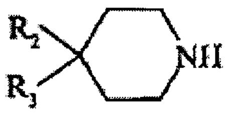

(III)

worin:

- $R_2$ für:

  . ein Pyridyl;
  . ein Phenyl, das unsubstituiert oder ein- oder zweifach durch einen oder zwei Substituenten, die unabhängig voneinander aus einem Halogenatom, einem $(C_1-C_4)$-Alkyl, einem $(C_1-C_4)$-Alkoxy, einer Trifluormethylgruppe und einer Trifluormethoxygruppe ausgewählt sind, substituiert ist;
  steht;

- $R_3$ für eine Gruppe:

  (11) $-(CH_2)_q-NR_7COOR_9$ steht;

- q für 0 steht;
- $R_7$ und $R_9$ zusammen für eine Gruppe $-(CH_2)_n$ stehen;
- n für 2 oder 3 steht;

in Basen- oder Säureadditionssalzform.

**14.** Arzneimittel, **dadurch gekennzeichnet, dass** es eine Verbindung der Formel (I) nach einem der Ansprüche 1 bis 4 oder ein Additionssalz dieser Verbindung mit einer pharmazeutisch unbedenklichen Säure oder auch ein Hydrat oder ein Solvat der Verbindung der Formel (I) enthält.

**15.** Pharmazeutische Zusammensetzung, **dadurch gekennzeichnet, dass** sie eine Verbindung der Formel (I) nach einem der Ansprüche 1 bis 4 oder ein pharmazeutisch unbedenkliches Salz, ein Hydrat oder ein Solvat dieser Verbindung sowie mindestens einen pharmazeutisch unbedenklichen Hilfsstoff umfasst.

**16.** Verwendung einer Verbindung der Formel (I) nach einem der Ansprüche 1 bis 4 zur Herstellung eines Arzneimittels zur Behandlung oder Prävention von Reizkolon (irritable bowel syndrome, IBS); Fibromyalgie; neuropathischen Schmerzen; chronischem Erschöpfungssyndrom; Migräne; atypischem Gesichtsschmerz; Morbis Crohn; Colitis ulcerosa; Verstopfung; Diarrhoe; gastroösophagealem Reflux; Gastritis; Pankreatitis; schwerer Depression; Angst wie generalisierter Angst, sozialer Angst, phobischer Angst und panischer Angst; Zwangsstörungen; Ticks; Manie; bipolaren Störungen; Schizophrenie; schizoaffektiven Störungen; Persönlichkeitsstörungen; psychotischen Störungen; mit Aufmerksamkeitsdefizit oder Hyperaktivität verbundenen Störungen; Störungen infolge des Gebrauchs von abhängig machenden Substanzen; Prostatahypertrophie.

## Claims

**1.** Compound corresponding to formula (I):

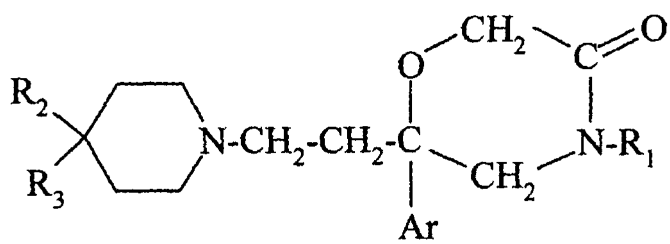

(I)

in which:

- Ar represents a phenyl disubstituted with a halogen atom;
- $R_1$ represents a phenyl that is unsubstituted or substituted once or twice with a halogen atom;
- $R_2$ represents:
  - a pyridyl;
  - a phenyl that is unsubstituted or substituted once or twice with one or two substituents independently chosen from a halogen atom, a $(C_1\text{-}C_9)$alkyl, a $(C_1\text{-}C_4)$alkoxy, a trifluoromethyl group and a trifluoromethoxy group;
- $R_2$ may also represent a heterocyclic radical chosen from azetidine, pyrrolidine, piperidine, morpholine, thiomorpholine and perhydroazetidine when $R_3$ represents a group $-CONR_{11}R_{12}$;
- $R_3$ represents a group chosen from:

  (5) $-(CH_2)_q-OH$ in which q is 0;
  (10) $-(CH_2)_q-NR_7COR_8$ in which q is 0;
  (11) $-(CH_2)q-NR_7COOR_9$ in which q is 0;
  (18) $-CONR_{11}R_{12}$;

- $R_7$ represents a hydrogen atom or a $(C_1\text{-}C_4)$alkyl;
- $R_8$ represents a hydrogen atom; a $(C_1\text{-}C_4)$alkyl; a vinyl; a phenyl; a benzyl; a pyridyl; a $(C_3\text{-}C_7)$cycloalkyl that is unsubstituted or substituted with one or more methyls; a furyl; a thienyl; a pyrrolyl; an imidazolyl;
- or $R_7$ and $R_8$ together represent a group $-(CH_2)_p-$;
- p is 3 or 4;
- $R_9$ represents a $(C_1\text{-}C_9)$alkyl or a phenyl;
- or $R_7$ and $R_9$ together represent a group $-(CH_2)_n-$;

- n is 2 or 3;
- $R_{11}$ and $R_{12}$ each independently represent a hydrogen or a $(C_1-C_4)$alkyl; $R_{12}$ may also represent a $(C_3-C_7)$ cycloalkyl, a $(C_3-C_7)$ cycloalkylmethyl, a hydroxyl, a $(C_1-C_4)$alkoxy, a benzyl or a phenyl; or $R_{11}$ and $R_{12}$, together with the nitrogen atom to which they are attached, constitute a heterocycle chosen from azetidine, pyrrolidine, piperidine, morpholine thiomorpholine and perhydroazepine;

in the form of base or of acid-addition salt, and also in the form of hydrate or solvate.

**2.** Compound of formula (I) according to Claim 1, **characterized in that**:

- Ar represents a 3,4-dichlorophenyl or a 3,4-difluorophenyl;
- $R_1$ represents a phenyl, a 4-chlorophenyl, a 4-fluorophenyl or a 3,4-difluorophenyl;
- $R_2$ represents:

  • a 2-pyridyl;
  • a phenyl, a 4-chlorophenyl, a 3-fluorophenyl, a 4-fluorophenyl, a 3,4-difluorophenyl, a 3-methylphenyl, a 3,4-dimethylphenyl, a 4-methoxyphenyl, a 3-(trifluoromethyl)phenyl, a 4-(trifluoromethyl)phenyl or a 4-(trifluoromethoxy)phenyl;
  • $R_2$ may also represent a 1-piperidyl when $R_3$ represents a $-CONH_2$ group or a $-CON(CH_3)_2$ group;

- $R_3$ represents a group chosen from:

  • a hydroxyl;

  . $-NHCOCH_3$ ;

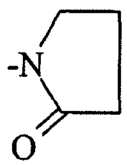 ; 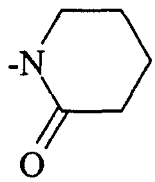 ;

.

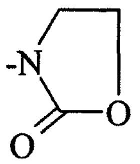 ; 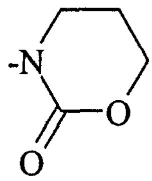 ;

  . $-CONH_2$ ;

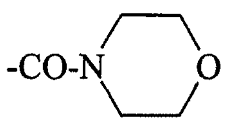 ;

  $-CON(CH_3)_2$ ;

in the form of base or of acid-addition salt, and also in the form of hydrate or solvate.

**3.** Compound according to Claim 1, of formula (I), chosen from:

- 6-(3,4-dichlorophenyl)-6-[2-[4-hydroxy-4-[3-(trifluoromethyl)phenyl]-1-piperidyl]ethyl]-4-phenylmorpholin-3-one, dextrorotatory isomer;
- N-[1-[2-[2-(3,4-dichlorophenyl)-5-oxo-4-phenylmorpholin-2-yl]ethyl]-4-phenyl-4-piperidyl]-acetamide, dextrorotatory isomer;
- N-[1-[2-[2-(3,4-dichlorophenyl)-5-oxo-4-phenylmorpholin-2-yl]ethyl]-4-(3-fluorophenyl)-4-piperidyl]acetamide, dextrorotatory isomer;
- N-[1-[2-[2-(3,4-dichlorophenyl)-5-oxo-4-phenylmorpholin-2-yl]ethyl]-4-(3,4-difluorophenyl)-4-piperidyl]acetamide, dextrorotatory isomer;
- N-[1-[2-[2-(3,4-dichlorophenyl)-5-oxo-4-phenylmorpholin-2-yl]ethyl]-4-(4-methylphenyl)-4-piperidyl]acetamide, dextrorotatory isomer;
- N-[1-[2-[2-(3,4-dichlorophenyl)-5-oxo-4-phenylmorpholin-2-yl]ethyl]-4-(4-methoxyphenyl)-4-piperidyl]acetamide, dextrorotatory isomer;
- N-[1-[2-[2-(3,4-dichlorophenyl)-5-oxo-4-phenylmorpholin-2-yl]ethyl]-4-[4-(trifluoromethoxy)-phenyl]-4-piperidyl]acetamide, dextrorotatory isomer;
- 6-(3,4-dichlorophenyl)-6-[2-[4-(3-fluorophenyl)-4-(2-oxopyrrolidin-1-yl)-1-piperidyl]ethyl]-4-phenylmorpholin-3-one, dextrorotatory isomer;
- 6-(3,4-dichlorophenyl)-6-[2-[4-(3,4-difluorophenyl)-4-(2-oxopyrrolidin-1-yl)-1-piperidyl]-ethyl]-4-phenylmorpholin-3-one, dextrorotatory isomer;
- 6-(3,4-dichlorophenyl)-6-[2-[4-(4-methylphenyl)-4-(2-oxopyrrolidin-1-yl)-1-piperidyl]ethyl]-4-phenylmorpholin-3-one, dextrorotatory isomer;
- 1'-[2-[2-(3,4-dichlorophenyl)-5-oxo-4-phenylmorpholin-2-yl]ethyl]-4'-(4-methylphenyl)-1,4'-bipiperidin-2-one, dextrorotatory isomer;
- 6-(3,4-dichlorophenyl)-6-[2-[4-(3-methylphenyl)-4-(2-oxopyrrolidin-1-yl)-1-piperidyl]ethyl]-4-phenylmorpholin-3-one, dextrorotatory isomer;
- 6-(3,4-dichlorophenyl)-6-[2-[4-(3-fluorophenyl)-4-(2-oxo-1,3-oxazolidin-3-yl)-1-piperidyl]-ethyl]-4-phenylmorpholin-3-one, dextrorotatory isomer;
- 6-(3,4-dichlorophenyl)-6-[2-[4-(4-fluorophenyl)-4-(2-oxo-1,3-oxazolidin-3-yl)-1-piperidyl]-ethyl]-4-phenylmorpholin-3-one, dextrorotatory isomer;
- 6-(3,4-dichlorophenyl)-6-[2-[4-(3,4-dimethylphenyl)-4-(2-oxo-1,3-oxazolidin-3-yl)-1-piperidyl]-ethyl]-4-phenylmorpholin-3-one, dextrorotatory isomer;
- 3-[1-[2-[2-(3,4-dichlorophenyl)-5-oxo-4-phenylmorpholin-2-yl]ethyl]-4-phenyl-4-piperidyl]-1,3-oxazinan-2-one, dextrorotatory isomer;
- 3-[1-[2-[2-(3,4-dichlorophenyl)-5-oxo-4-phenylmorpholin-2-yl]ethyl]-4-(4-fluorophenyl)-4-piperidyl]-1,3-oxazinan-2-one, dextrorotatory isomer;
- 3-[1-[2-[2-(3,4-dichlorophenyl)-5-oxo-4-phenylmorpholin-2-yl]ethyl]-4-(3,4-difluorophenyl)-4-piperidyl]-1,3-oxazinan-2-one, dextrorotatory isomer;
- 6-(3,4-dichlorophenyl)-6-[2-[4-(morpholin-4-ylcarbonyl)-4-phenyl-1-piperidyl]ethyl]-4-phenylmorpholin-3-one, dextrorotatory isomer;
- 1'-[2-[2-(3,4-dichlorophenyl)-5-oxo-4-phenylmorpholin-2-yl]ethyl]-1,4'-bipiperidin-4'-carboxamide, dextrorotatory isomer;
- 6-(3,4-dichlorophenyl)-6-[2-[4-(3,4-difluorophenyl)-4-(2-oxo-1,3-oxazolidin-3-yl)-1-piperidyl]ethyl]-4-phenylmorpholin-3-one, dextrorotatory isomer;
- N-[1-[2-[2-(3,4-difluorophenyl)-5-oxo-4-phenylmorpholin-2-yl]ethyl]-4-phenyl-4-piperidyl]-acetamide, dextrorotatory isomer;
- N-[1-[2-[2-(3,4-difluorophenyl)-5-oxo-4-phenylmorpholin-2-yl]ethyl]-4-[4-(trifluoromethyl)-phenyl]-4-piperidyl] acetamide, dextrorotatory isomer;
- 1'-[2-[2-(3,4-difluorophenyl)-5-oxo-4-phenylmorpholin-2-yl]ethyl]-1,4'-bipiperidine-4'-carboxamide, dextrorotatory isomer;
- N-[1-[2-[2-(3,4-dichlorophenyl)-5-oxo-4-phenylmorpholin-2-yl]ethyl]-4-[3-(trifluoromethyl)-phenyl]-4-piperidyl] acetamide, dextrorotatory isomer;
- 6-(3,4-dichlorophenyl)-6-[2-(4-hydroxy-4-pyridin-2-yl-1-piperidyl)ethyl]-4-phenylmorpholin-3-one, dextrorotatory isomer;
- N-[1-[2-[4-(4-chlorophenyl)-2-(3,4-dichlorophenyl)-5-oxomorpholin-2-yl]ethyl]-4-phenyl-4-piperidyl]acetamide;
- 4-(4-chlorophenyl)-6-[2-[4-(4-chlorophenyl)-4-(2-oxo-1,3-oxazolidin-3-yl)-1-piperidyl]ethyl]-6-(3,4-dichlorophenyl)morpholin-3-one;
- 1'-[2-[4-(4-chlorophenyl)-2-(3,4-dichloro-phenyl)-5-oxomorpholin-2-yl]ethyl]-N,N-dimethyl-1,4'-bipiperidine-4'-carboxamide;

- N-[1-[2-[2-(3,4-dichlorophenyl)-4-(4-fluorophenyl)-5-oxomorpholin-2-yl]ethyl]-4-phenyl-4-piperidyl]acetamide;
- N-[1-[2-[2-(3,4-dichlorophenyl)-4-(4-fluoro-phenyl)-5-oxomorpholin-2-yl]ethyl]-4-(3,4-difluoro-phenyl)-4-piperidyl]acetamide;
- 1'-[2-[2-(3,4-dichlorophenyl)-4-(4-fluorophenyl)-5-oxomorpholin-2-yl]ethyl]-N,N-dimethyl-1,4'-bipiperidine-4'-carboxamide;
- N-[1-[2-[2-(3,4-dichlorophenyl)-4-(3,4-difluorophenyl)-5-oxomorpholin-2-yl]ethyl]-4-phenyl-4-piperidyl]acetamide;

in the form of base or of acid-addition salt, and also in the form of hydrate or solvate.

**4.** Compound according to Claim 1 of formula (I), which is:

- N-[1-[2-[2-(3,4-dichlorophenyl)-5-oxo-4-phenylmorpholin-2-yl]ethyl]-4-(3-fluorophenyl)-piperid-4-yl]acetamide, dextrorotatory isomer;

in the form of base or of acid-addition salt, and also in the form of hydrate or solvate.

**5.** Process for preparing a compound of formula (I) according to any one of Claims 1 to 4, **characterized in that**:

a compound of formula:

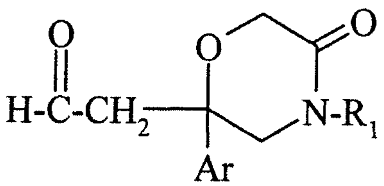

(II)

in which Ar and $R_1$ are as defined for a compound of formula (I) in Claim 1, is reacted with a compound of formula:

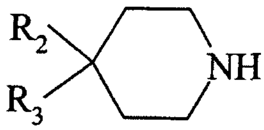

(III)

in which $R_2$ and $R_3$ are as defined for a compound of formula (I) in Claim 1, in the presence of an acid, in a solvent, and the intermediate iminium salt formed is then reduced using a reducing agent.

**6.** Process for preparing a compound of formula (I) according to any one of Claims 1 to 4, **characterized in that**:

a compound of formula:

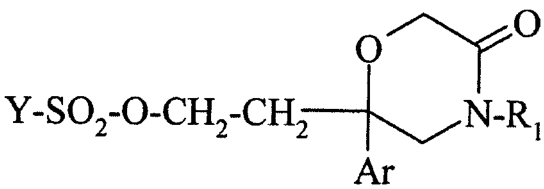

(IV)

in which Ar and $R_1$ are as defined for a compound of formula (I) in Claim 1, and Y represents a methyl, phenyl, tolyl or trifluoromethyl group, is reacted with a compound of formula:

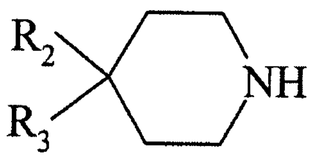

(III)

in which $R_2$ and $R_3$ are as defined for a compound of formula (I) in Claim 1.

**7.** Compound of formula:

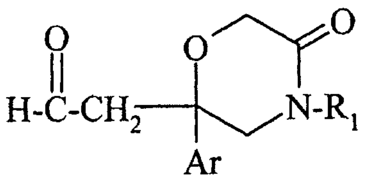

(II)

in which:

- Ar represents a phenyl that is disubstituted with a halogen atom;
- $R_1$ represents a phenyl that is unsubstituted or substituted once or twice with a halogen atom.

**8.** Compound of formula:

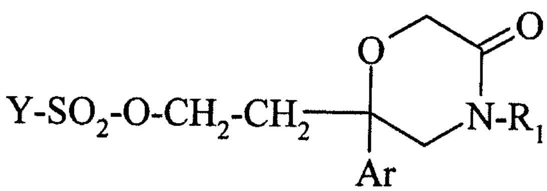

(IV)

in which:

- Y represents a methyl, phenyl, tolyl or trifluoromethyl group;
- Ar represents a phenyl that is disubstituted with a halogen atom;
- $R_1$ represents a phenyl that is unsubstituted or substituted once or twice with a halogen atom.

**9.** Compound of formula:

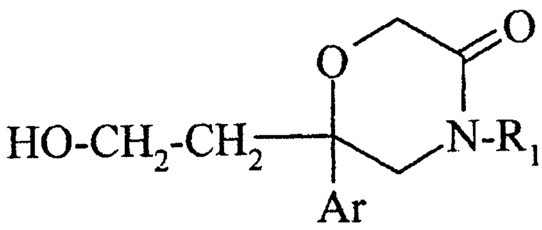

(V)

in which:

- Ar represents a phenyl that is mono- or disubstituted with a halogen atom;
- $R_1$ represents a phenyl that is unsubstituted or substituted once or twice with one or two substituents independently chosen from a halogen atom, a $(C_1-C_4)$alkyl and a $(C_1-C_4)$alkoxy.

**10.** Compound of formula:

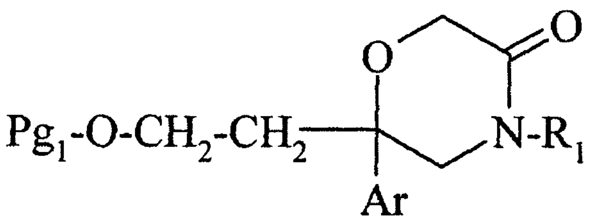

(VI)

in which:

- $Pg_1$ represents a 2-tetrahydropyranyl, benzoyl or $(C_1-C_4)$alkylcarbonyl radical;
- Ar represents a phenyl that is disubstituted with a halogen atom;
- $R_1$ represents a phenyl that is unsubstituted or substituted once or twice with a halogen atom.

**11.** Compound of formula:

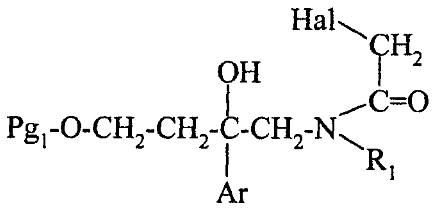

(VII)

in which:

- Hal represents a chlorine or bromine atom;
- $Pg_1$ represents a 2-tetrahydropyranyl, benzoyl or $(C_1-C_4)$alkylcarbonyl radical;
- Ar represents a phenyl that is disubstituted with a halogen atom;
- $R_1$ represents a phenyl that is unsubstituted or substituted once or twice with a halogen atom.

**12.** Compound of formula:

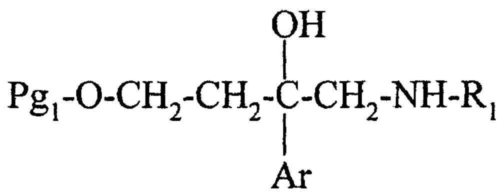

(VIII)

in which:

- $Pg_1$ represents a 2-tetrahydropyranyl, benzoyl or $(C_1-C_4)$alkylcarbonyl radical;
- Ar represents a phenyl that is disubstituted with a halogen atom;
- $R_1$ represents a phenyl that is unsubstituted or substituted once or twice with a halogen atom.

**13.** Compound of formula:

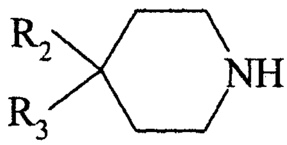

(III)

in which:

- $R_2$ represents:

. a pyridyl;
. a phenyl that is unsubstituted or substituted once or twice with one or two substituents independently chosen from a halogen atom, a ($C_1$-$C_4$)alkyl, a ($C_1$-$C_9$)alkoxy, a trifluoromethyl group and a trifluoromethoxy group;

- $R_3$ represents a group:

(11)-$(CH_2)_q$-$NR_7COOR_9$;

- q is 0;
- $R_7$ and $R_9$ together represent a group $-(CH_2)_n-$;
- n is 2 or 3;

in the form of base or of acid-addition salt.

**14.** Medicinal product, **characterized in that** it contains a compound of formula (I) according to any one of Claims 1 to 4, or a pharmaceutically acceptable acid-addition salt of this compound, or alternatively a hydrate or a solvate of the compound of formula (I).

**15.** Pharmaceutical composition, **characterized in that** it comprises a compound of formula (I) according to any one of Claims 1 to 4, or a pharmaceutically acceptable salt, a hydrate or a solvate of this compound, and also at least one pharmaceutically acceptable excipient.

**16.** Use of a compound of formula (I) according to any one of Claims 1 to 4 for the preparation of a medicinal product for treating and preventing irritable bowel syndrome (IBS); fibromyalgia; neuropathic pain; chronic fatigue syndrome; migraine; atypic facial pain; Crohn's disease; ulcerative colitis; constipation; diarrhoea; gastro-oesophageal reflux; gastritis; pancreatitis; major depression; anxiety such as generalized anxiety, social, phobic and panic anxiety; compulsive obsessive disorders; tics; mania; bipolar disorders; schizophrenia; schizoaffective disorders; personality disorders; psychotic disorders; disorders associated with attention deficit or hyperactivity; disorders caused by the use of addictive substances; prostate hypertrophy.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9623787 A
- EP 0776893 A
- WO 0034274 A
- WO 02094821 A
- WO 0058292 A
- EP 0428434 A
- EP 0512901 A
- EP 0515240 A
- WO 03104225 A
- EP 474561 A
- WO 9710211 A


### Littérature non-brevet citée dans la description


- **PENNEFATHER J.N. et al.** *Life Sci.,* 2004, vol. 74, 1445-1463
- **JOOS G.F.** *Handb. Exp. Pharm.,* 2004, vol. 164, 491-510
- **ADVENIER C. et al.** *Eur. Respir. J.,* 1997, vol. 10, 1892-1906
- **HOLZER P.** *Handb Exp. Pharm.,* 2004, vol. 164, 511-558
- **KISS S. et al.** *Neurosci. Lett.,* 2001, vol. 313, 57-60
- **WAMER F.J. et al.** *Eur. J. Pharmacol.,* 2002, vol. 438, 171-177
- **HAGAN R.M. et al.** *Regul. Pept.,* 1993, vol. 46, 9-19
- **STEINBERG R. et al.** *Eur. J. Neurosci.,* 1998, vol. 10, 2337-2345
- **BENSAID M et al.** *Neurosci. Lett.,* 2001, vol. 303, 25-28
- **SAFFROY M. et al.** *J. Neurochem.,* 2001, vol. 79, 985-996
- **SAFFROY M. et al.** *Neuroscience,* 2003, vol. 116, 761-773
- **YASHPAL K. et al.** *Brain Res.,* 1990, vol. 506, 59-266
- **EMONDS-ALT.** *Handb Exp. Pharm.,* 2004, 219-244
- **LECCI A. et al.** *Br. J. Pharmacol.,* 2004, vol. 141, 1249-1263
- **GREEN et al.** Protective Group in Organic Synthesis. John Wiley & Sons, Inc, 1991
- **J. MARCH.** Advanced in Organic Chemistry. Wiley Interscience, 1985, 310-316
- *Tetrahedron,* 1997, vol. 53 (12), 4137-4144
- *Tetrahedron Letters,* 1996, vol. 37 (19), 3295-3298
- *Tetrahedron,* 1998, vol. 54, 4313-4318
- *Synthetic Communications,* 1996, vol. 26 (24), 4569-4575
- **PONCELET et al.** *Neurosci. Lett.,* 1993, vol. 149, 40-42